(19) Europäisches Patentamt
European Patent Office
Office européen des brevets

(11) EP 1 815 846 A2

(12) **EUROPEAN PATENT APPLICATION**

(43) Date of publication:
**08.08.2007 Bulletin 2007/32**

(21) Application number: **06023465.5**

(22) Date of filing: **09.10.2003**

(51) Int Cl.:
***A61K 9/127*** *(2006.01)* ***A61K 31/192*** *(2006.01)*
***A61K 31/196*** *(2006.01)* ***A61K 31/405*** *(2006.01)*
***A61K 31/5415*** *(2006.01)*

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **11.10.2002 US 417874 P**
**04.02.2003 US 357617**
**04.02.2003 US 357618**

(62) Document number(s) of the earlier application(s) in accordance with Art. 76 EPC:
**03757944.8 / 1 551 370**

(71) Applicant: **IDEA AG**
**80807 Munich (DE)**

(72) Inventors:
- **Cevc, Gregor**
  **82131 Gauting (DE)**
- **Vierl, Ulrich**
  **82194 Gröbenzell (DE)**

(74) Representative: **Maiwald Patentanwalts GmbH**
**Elisenhof**
**Elisenstraße 3**
**80335 München (DE)**

Remarks:
This application was filed on 10-11-2006 as a divisional application to the application mentioned under INID code 62.

(54) **Aggregate with increased deformability, comprising at least three amphipats, for improved transport through semi-permeable barriers and for the non-invasive drug application in vivo, especially through the skin**

(57) The application describes combinations of at least three amphipatic substances forming aggregate suspensions in a polar liquid. Judicious choice of system components, which differ at least 2-times to 10-times in solubility, ensures said aggregates to have extended, unusually adaptable surfaces. This is probably due to simultaneous action on said aggregates of at least two more soluble substances amongst said three system components, at least one of which is an active ingredient and preferably a drug; the third component, alternatively, can take the role of a drug. The application further deals with the use of said combinations in pharmaceutical preparations capable of transporting drugs into the body of warm blood creatures. This is made possible by the drug loading capability of said aggregates with the highly flexible and deformable coating, which renders the resulting drug carriers highly adaptable. The application finally reveals suitable methods and favourable conditions for carrier manufacturing and application. The application also describes novel formulations of nonsteroidal anti-inflammatory drugs (NSAIDs) based on complex aggregates with at least three amphipatic components suspended in a suitable, e.g. pharmaceutically acceptable, polar liquid medium.

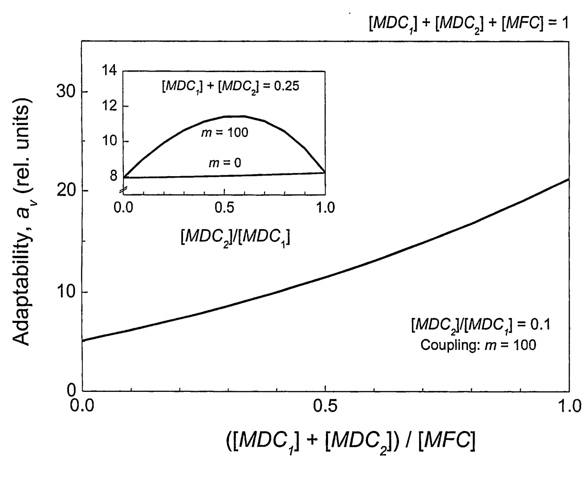


Fig. 5

EP 1 815 846 A2

## Description

### Field of Invention

The invention relates to aggregates with extended surface (extended-surface aggregates, ESAs) with increased deformability and improved barrier penetration capability, said ESAs being suspendable in a suitable liquid medium and comprising at least three amphipats (amphipatic components) and being capable to improve the transport of actives through semi-permeable barriers, such as the skin, especially for the non-invasive drug application in vivo by means of barrier penetration by such aggregates. The three amphipats include at least one membrane forming compound (MFC), which can form the membrane of said ESAs, and at least two membrane destabilising compounds ($MDC_1$ and $MDC_2$) differentiated by their capability of forming smaller aggregates (with no extended surfaces) by either themselves or else in combination with each other *and/or* characterized by their relatively high solubility in said suitable liquid medium. The ESAs are loaded with at least one biologically active compound, which can be one of the at least three amphipats.

The invention relates also to preparations comprising extended surface aggregates (ESAs), that can penetrate barriers even when the typical ESAs radius (when an ESA is considered to be spherical) is at least 40% (and preferably at least 50% or even more) greater than the average radius of a pore in the barrier before and after the ESAs have penetrated the barrier.

This invention deals also with novel formulations of nonsteroidal anti-inflammatory drugs (NSAIDs) based on complex, extended surface aggregates comprising at least three amphipatic components. One of these components is capable of forming stable, large bilayer membranes on it's own. The other at least two amphipatic components, including an NSAID, tend to destabilise such membranes. Said aggregates are normally suspended in a suitable, e.g. pharmaceutically acceptable, polar liquid medium, which also affects NSAID ionisation. The selection of the second amphipatic membrane destabilising component, which is typically a (co)surfactant, can boost the deformability of the resulting mixed extended surface aggregates. This effect may be supported by judicious choice of the other system components. The invention enables an improvement of barrier penetration and drug delivery by such aggregates. The invention also teaches how to select the most appropriate NSAID concentration, the right total amphipat concentration and, in case, amphipat ionisation in the resulting mixed aggregate suspension. The invention further relates to the preparation and application of the resulting suspension in pharmaceutical formulations, with a focus on epicutaneous application on, or less frequently in, warm blooded creatures.

### Background Information

Administration of active ingredients frequently is limited by natural barriers, such as the skin, which prevent adequate absorption of the active molecules due to the low barrier permeability for such ingredients.

Availability and use of preparations that can overcome this barrier impermeability problem and allow non-invasive active ingredient administration would be advantageous in many cases. In humans and animals, for example, a percutaneous administration of such preparations would protect the active ingredients against decomposition in the gastrointestinal tract and possibly would result in a modified, therapeutically attractive distribution of the agent in the body; such non-invasive administration could also affect the pharmacokinetics of the active ingredient and permit less frequent and/or simpler disease treatment (G. Cevc. Exp. Opin. Invest. Drugs (1997) 6: 1887-1937.). In the case of plants, improved penetration through or into the cuticle could lower the concentration of active ingredient that is required for the desired effect and, in addition, could significantly decrease contamination of the environment (Price, C.E. (1981) in: The Plant Cuticle (D.F. Cutler, K.L. Alvin, C.E. Price, Publisher), Academic, New York, pp. 237252).

Many methods for increasing the skin permeability have been discussed (see, for example, G. Cevc, 1997, *op. cit.).* Most prominent are jet injection (for a classical review see Siddiqui & Chien Crit. Rev. Ther. Drug. Carrier Syst. (1987) 3: 195-208), the use of electrical (Bumette & Ongpipattanakul J. Pharm. Sci. (1987) 76: 765-773) or accoustic (Vyas et al., J Microencapsul (1995) 12: 149-54) skin perturbation or else the use of chemical additives, such as certain solvents or surfactants. Such chemicals generally act as the skin permeation enhancers by increasing the partitioning and/or diffusivity of the active ingredient in the skin lipids.

Most often used permeation enhancers are non-ionic short or long-chain alcohols and uncharged surfactants etc., anionic materials (particularly fatty acids), cationic long-chain amines, sulfoxides, as well as various amino derivatives, and amphoteric glycinates and betaines. None of these, however, solves the problem of active ingredient transport through the skin or mucous barrier to general satisfaction.

An overview of the measures, which have been used for the purpose of increasing active ingredient penetration through plant cuticles, is summarised in the work of Price (1981, *op. cit.).*

Epidermal use of one or several amphipatic substances in the form of a suspension or an *O/W* or *W/O* emulsion, has also brought about too little improvement. An extensive review written by G. Cevc (1997, *op. cit..)* explains why liposomes, at best, can modify drug retention time or stability on the skin and or improve transcutaneous drug transport

by partly occluding the skin surface. Japanese patent application JP 61/271204 A2 (86/27 1204) provides an example for a stabilizing effect of liposomes on the skin, relying on hydroquinone glucosidal as stabilizing material.

The use of lipid vesicles loaded with an active ingredient combined with a gel-forming agent in the form of "transdermal patches" was proposed in WO 87/1938 A1. However, the ability of the active ingredient to permeate the skin was not appreciably increased. Massive use of permeation -promoting polyethylene glycol and of fatty acids, together with lipid vesicles, was required by Gesztes and Mezei (1988, Anesth. Analg. 67,1079 -1081) to attain only a moderate local analgesia with lidocaine-containing formulations applied for several hours under occlusion on the skin.

United States Patent 6.193,996 describes a pressure sensitive skin adhesive that uses skin permeation enhancers. European Patent applications EPA 102 324 and EPA 0 088 046 and US patent US 4,619.794, all by H. Hauser, describe methods for preparing unilamellar vesicles, using a single membrane destabilising component. The vesicles may be used as carriers for different drugs. However, such vesicles are not used on the skin or for transport through semi-permeable barriers. European Patent application EPA 0 152 379 by Muntwyler and Hauser similarly describes the preparation of unilamellar vesicles. However, these vesicles often need to be separated from the residual multilamellar liposomes, facilitated by the presence of charged drugs, for final use of the former for treating the human or animal body. The authors also point to the potential need to neutralize the drug during vesicle preparation to obtain the desired unilamellar liposomes. Further, such vesicles are not used for transport of drugs through a semi-permeable barrier.

European patent EP 0 475 160, corresponding US patent 6,165,500 and Canadian patent 2,067,754, all with the title "Preparation for the application of agents in mini-droplets", describe special preparations related to the suspensions described in this application. These documents report the use of different agents associated with minuscule droplets or, in particular, with the vesicles consisting of one or a few membrane-like amphiphile assemblies for overcoming semi-permeable barriers including the skin. These references describe preparations having a single membrane destabilising component. WO 98/17255 and AU 724218, likewise, describe vesicles for the transport of a variety of drugs through the skin.

In two relatively early reports on dermal liposomal tetracaine (Gesztes A, Mezei M. "Topical anesthesia of the skin by liposome-encapsulated tetracaine." Anesth. Analg. (1988),67:10791081) and lidocaine (Foldvari M, Gesztes A, Mezei M. "Dermal drug delivery by liposome encapsulation: clinical and electron microscopic studies." J Microencapsul (1990), 7:479-489), Mezei's group reported anaesthetic performance of such locally used drugs and corresponding autoradiography data. Drug was found in the epidermis and in dermis of humans and guinea pigs when the skin was treated under an impermeable (occlusive) coating with the liposome - encapsulated anaesthetics. The formulations always contained multilamellar soybean phosphatidylcholine vesicles. However, the reports demonstrate no liposome-mediated drug transport through the skin. (Foldvari M. "In vitro cutaneous and percutaneous delivery and in vive efficacy of tetracaine from liposomal and conventional vehicles." Pharm Res (1994) 11: 1593-1598) and with an additional oily ingredient (Foldvari M. "Effect of vehicle on topical liposomal drug delivery: petrolatum bases." J Microencapsul (1996), 13:589-600). This conclusion is supported by the fact that the reported maximum transported drug dose (5.3%) was more than 20-times higher than the reported transported lipid dose (0.2%) (Foldvari, 1994). Further, Foldvari's formulations evidently were not optimised for adaptability but rather for best drug retention/release.

P. Gonzalez, M. E. Planas, L. Rodriguez, S. Sanchez, and G. Cevc in an article on "Noninvasive, percutaneous induction of topical analgesia by a new type of drug carriers and prolongation of the local pain-insensitivity by analgesic liposomes" (Anesth. Analg. (1992), 95: 615-621 )report the results of investigations with surfactant-containing formulations, typically loaded with lidocaine (2%, as a free base) in a mixed lipid 4-8% suspension (w/v). Lipid aggregates were prepared from a 4/1 mol/mol phosphatidylcholine/sodium cholate mixture, starting with an ethanolic lipid solution (7-3 w-% EtOH in the final product) for easier manufacturing. However, all the tested suspensions were reported by Planas et al. to be unstable. Further, Planas et al. failed to disclose how a stable drug formulation could be prepared, which would be suitable for transdermal drug delivery.

Peters and Moll (1995) ("Pharmacodynamics of a liposomal preparation for local anaesthesia". Arzneimittelforschung (1995), 45:1253-6, describe permeation of a topically applied drug through the skin. The permeation is enhanced by ethanol, is based on diffusion, and is achieved under occlusion.

Carafa and colleagues describe the use of surfactant-based, phospholipid-free vesicles (Carafa et al., 2002 ("Lidocaine-loaded non-ionic surfactant vesicles: characterisation and in vitro permeation studies." Int J Pharm (2002), 231 :21-32). However, such vesicles do not simultaneously include both a MFC and a MDC, and are unsatisfactory.

The current state of the art in particular in NSAID delivery through the skin is transdermal drug diffusion, which is proportional to the drug concentration on the skin and inversely proportional to the skin barrier resistance, which is tantamount to saying that diffusion is proportional to the skin permeability.

Solubility of typical NSAIDs is in the range 1 ~g/ml to between 0.5 mg/ml and 10 mg/ml for the pH range between 1 and 7.5. This corresponds to a few μM and up to a few tens of mM, high values being always measured in least acidic solutions (pH » pKa) where NSAIDs are partly or completely ionised, the solubility at pH « pKa always being very low. To maximise diffusive NSAID transport through the skin one should therefore always use the highest tolerable *pH,* which can exceed the value of 9.

Taken the limitations of maximum NSAID solubility, attempts have been made to improve NSAID permeation (diffusion) through the skin by using permeability or permeation enhancers. Permeability enhancers increase NSAID flux through the barrier for a given drug concentration, but do not much affect the depth of drug distribution. Further, use of conventional lipid formulations on the skin does not affect this limitation.

For example, Henmi et al. 1994 (Chem Pharm Bull 42:651-655) used three different NSAIDs (ketoprofen, flurbiprofen and ibuprofen) in an oily gel, formed by hydrogenated soybean phospholipids (which forms very stiff membranes) and applied the preparation on the skin. The conclusion was that such lipids have no permeation enhancing effect for the skin but rather solubilise the test drug.

Burnham et al. 1998 (Clin J Sport Med 8:78-81) used a block co-polymer of polyethylene and an unspecified polypropylene glycol (pluronic), which generally is a poor membrane destabilising amphipat, to apply an NSAID on the skin. An unspecified lecithin based liposomal organo-gel (PLO) was furthermore used three times daily for one week, followed by a weekly "washout" period without using the gel. The authors noted that only a thin tissue layer under the skin was treated, thus implying that any apparently positive result could be due to free drug diffusion from PLO through the skin. Organo-gel consequently has served as merely a superficial reservoir.

Vyas et al. (J Microencapsul12:149-54, 1995) incorporated diclofenac into multilamellar, 1-5 μm large liposomes at *pH* = 7.4 that were applied on the skin under different conditions. The resulting systemic drug availability was then studied. The resulting mixed lipid vesicles were incorporated in an ointment base and were applied on the skin of rats. However, skin poration by ultrasound was required to achieve any substantial transdermal delivery of the drug, and most of the tested NSAID was typically found at the site of application.

Schramlova et al. (Folia Biol (Praha) 43:195-199,1997) associated ibuprofen with liposomes prepared from soybean phospholipid supplemented with 10 rel-% cholesterol , the knowledge in the art being that the latter is a membrane stiffening agent. The formulation with a *pH* =7.4 was injected intramuscularly or applied under occlusion on the skin. NSAID from lipid vesicles occasionally decreased the rat leg edema slightly, but not significantly, better than the drug from a conventional cream but less than an NSAID injection. This paper therefore teaches the use of a membrane stabilising component (cholesterol) rather than of a membrane destabilising component.

Saunders et al. (J Pharm Pharm Sci 2:99-107,1999), studying the skin permeation enhancement, also used liposomal structures of unspecified composition and morphology, which were claimed to be present in the MZL lotion and in a comparator gel (both prepared by Meyer Zall Laboratories (MZL)), and loaded with sodium diclofenac. The presence of oil in the oil/water base in the MZL formulation, which diminishes lipid aggregate deformability, and occludes the skin, if nothing else precluded efficient drug delivery by vesicle through the skin.

Calpena et al. (Arzneimittelforschung 49:1012-1017,1999) studied diclofenac permeation through human skin from 6 semisolid formulations containing 1 % drug in a complex mixture of gel-forming materials combined with lecithin (2.5% of unspecified quality) and cholesterol (0.5%). However, the results of the studies suggest that use of lipid vesicles is not beneficial (Calpena et al., 1999).

Skin permeability data for ibuprofen lysinate was studied, showing practically equal permeability rates for the drug in solution or in mixed micelles (containing soy-bean phosphatidylcholine) and nearly 3-times lower rate for the corresponding liposomal dispersion (Stoye et al., 1998 (Eur J Pharm Biopharm 46:191-200). Liposomes therefore were concluded to be useless in terms of supporting transdermal drug transport in the described system.

## Summary of the Invention

Applicants have discovered that incorporation of a surfactant into a bilayer membrane that is built from another less soluble amphipat, such as a phospholipid, can increase the flexibility of the resulting complex membrane. This promotes the capability of complex aggregates in the form of droplets covered by the bi-component membranes to cross pores in a semi-permeable barrier that otherwise would prevent comparably large aggregates from crossing. Further, the use of aggregates with highly deformable membrane coating can mediate agent transport into and/or across mammalian skin. This can be achieved by selecting a surfactant, which is a membrane destabilising component (= *MDC*), and a less soluble amphipat, which is the membrane forming component (= *MFC) ,* so as to maximize the mixed membrane flexibility and the mixed aggregate stability. Further the surfactant can be selected to increase bilayer membrane adaptability. Patent applications by applicant, especially WO 92/03122 and WO 98/172550 describe basic requirements for the use of lipid/surfactant mixtures for transbarrier transport.

It is an objective of the invention to provide preparations that can transport active ingredients through a barrier in the form of vesicles or other extended surface aggregates (ESAs) comprising said actives, said preparations having improved permeation capability through semi-permeable barriers.

It is a further aspect of the invention to provide a preparation based on a combination of at least one first (membrane forming component *MFC)*, at least one second (membrane destabilising component *MDC)*, and at least one third (membrane destabilising component *MDC)* amphipatic component suspended in a suitable liquid medium in the form of corresponding mixed amphipat extended surface aggregates (ESAs) with one or a few bilayer-like, mixed

amphipat coating(s), wherein said ESAs formed by a combination of all three said components have surfaces in contact with said liquid medium, that are at least 50% more extended, on the average, than the typical surfaces of aggregates comprising the said at least one second and at least one third amphipatic component alone, at the same concentrations and, in case, after adjustment for physico-chemical effects of the absence of said first amphipatic compound *(MFC)*.

A further aspect of the invention is to provide suspensions of extended surface aggregates in a liquid medium comprising: at least one first membrane forming component *(MFC)*; at least one second membrane destabilising component *(MDC)*; at least one third membrane destabilising component *(MDC)*, the third component typically being a drug, such that said complex extended surface aggregates (ESAs) can penetrate intact mammalian skin and thus increase drug concentration in the skin and/or increase the reach of drug distribution below the skin, in comparison with the result of the same drug application in a solution on the skin. In a special version of said suspensions, said extended surface aggregates are membrane-enclosed, liquid-filled vesicles, said first component is a membrane-forming lipid, and said second and third components are membrane-destabilising components.

Another aspect of the invention provides a combination of at least one first (membrane forming, component *MFC)*, at least one second (membrane destabilising component *MDC)*, and at least one third (membrane destabilising component *MDC)* amphipatic component suspended in a suitable liquid medium in the form of mixed amphipat extended surface aggregates (ESAs) with one or a few bilayer-like, mixed amphipat coating(s), wherein the

- said at least one first substance has a tendency to self aggregate and is at least 10 times less soluble in said liquid medium than said at least one second and said one third substance, allowing the first to form extended surfaces,
- said at least one second substance is at least 10-times more soluble than said at least one first substance in said liquid medium and, on its own, tends to form or supports the formation of surfaces, that are at least 2-times less extended than the surfaces containing the at least one first substance alone,
- said at least one third substance being also at least 10-times more soluble in said liquid medium than the first substance and optionally forms self-aggregates with aggregation numbers at least 10-times smaller than that of self-aggregates of said first substance; and
- said extended surfaces comprising said at least one first, at least one second and at last one third substance, in equilibrium, have at least 50% more extended surfaces than the surfaces formed by the at least one second or one third substance alone, at the same concentration and, in case, after adjustment for physico-chemical effects of the absence of said first amphipatic compound *(MFC)*.

Yet another aspect of the invention is a preparation based on a combination of at least one first (membrane forming component *MFC)*, at least one second (membrane destabilising component *MDC)*, and at least one third (membrane destabilising component *MDC)* amphipatic component suspended in a suitable liquid medium in the form of corresponding mixed aggregates with an extended surface (ESAs) with one or a few, preferably bilayerlike, mixed amphipat coating(s), wherein said *MFC* alone forms extended-surface aggregates with aggregation number of at least 5000, and preferably more than 10.000, and both *MDCs* alone and the combination of both *MDCs* form smaller aggregates with no really extended surface and aggregation number below 5000, and preferably below 1000 in contact with said suitable liquid medium.

All compositions according to the present invention comprising three amphipatic compounds which together form extended surface aggregates either have a defined solubilization point, or do comprise more than 0.1 mol% of the solubilizing amount of those components which at higher concentrations would solubilize the extended surface aggregates.

All embodiments of the invention are useful in preparations for the application, administration or transport of at least one active ingredient which can be amongst said three substances, especially for medicinal or biological purposes, into and through barriers and constrictions, such as the skin of warm blood creatures or the like.

Preferably the adaptability of extended surface comprising all three said amphipatic components to ambient stress exceeds by at least 20% or by at least twice the standard deviation of a typical measurement (whichever is smaller) the adaptability of the extended surface comprising the at least one first and the at least one second amphipatic component used at the corresponding concentrations or the adaptability of the extended surface comprising the at least one first and the at least one third amphipatic component at corresponding concentrations, whichever is smaller.

The adaptability can be expressed as the inverse value of the $p^*$ value. This specific $p^*$ value is typically higher than 50%, often is around 60% and preferably is 57% of Pmax-value.

We have further found, unexpectedly, that various combinations of at least two amphipatic components one of which is an NSAID, which can substantially destabilise a lipidbased, otherwise stable extended surface aggregate, typically in the form of a bilayer membrane, can synergistically increase the resulting at least three component aggregate

adaptability. In parallel, the aggregate (membrane) shape deformability is synergistically augmented. Consequently, the flux of such aggregate suspension through narrow pores is increased and/or the characteristic pressure that drives certain flux through the corresponding porous barrier is lowered.

The capability of said at least three-component aggregates to move through a semi-permeable barrier is thus facilitated. This finding is surprising given that the droplets covered by a bi-component bilayer membrane already have an appreciable barrier crossing capability compared to droplets enclosed by a simple lipid bilayer.

The increase of adaptability of said extended surface aggregates with at least three amphipatic components and/or the lowering of the pressure that is needed to make such aggregates move through a biological barrier has important, and unexpected, practical consequences. Specifically, when said aggregates are applied on the skin, as an example for a biological semi-permeable barrier, the transport of the aggregate associated NSAIDs through such barrier is increased and reaches further. The latter observation is explicable in terms of differential clearance in the superficial skin layers, where cutaneous blood drainage resides, of the drug, which can enter directly into blood capillaries, and of drug-loaded aggregates, which are too big to enter such capillaries. This means that NSAID carriers move further than the drug from solution, allowing deeper tissues to be treated with NSAIDs under the drug application site on the skin. Convincing evidence for this is given in one of Practical Examples. Such finding is not expected taken that simple NSAID phospholipid combinations already ensure better and deeper drug transport through the skin than conventional preparations based on NSAID solutions.

Further objectives and advantages of the instant invention will become apparent from the following description of preferred embodiments, which include a best mode preparation.

In the present description, the general terms employed hereinbefore and hereinafter have the following meanings.

The term "**aggregate**" denotes a group of more than just a few amphipats of similar or different kind. A small aggregate, as used in the context of this invention, has an aggregation number $n_a > 3$, that is, contains at least 3 molecules, but does not exceed $n_a < 5000$ or more preferably $n_a < 1000$, that is, contains no more than 5000 or 1000 molecules. The "extended surface aggregate (ESA)"," an aggregate with extended surface", a "vesicle" or an "extended surface" as used in the context of this invention, all have aggregation numbers ≥ 5000, that is, contain a minimum of 5000 molecules, and most often are characterized by an even higher aggregation number, that is, contain an even higher number of molecules. Preferred ESAs have aggregation numbers of $n_a > 10000$ and even more preferably $n_a > 50000$. For a preparation containing aggregates, the reference will always be made to the average aggregation number or to the average number of molecules per aggregate, except if indicated otherwise. The term "aggregation number" equals the number of molecules which together form an aggregate. Corresponding methods of $n_a$ determination are well known in the art.

When a lipid aggregate is water filled and surrounded with at least one membrane it is called a lipid vesicle. The membrane as defined in this description is a mixture of at least three amphipats $(MFC + MDC_1 + MDC_2)$ preferably in the form of a bilayer; a membrane destabilising component hereby is potentially a *MFC-MDC* combination (i.e. a mixed amphipat associate).

The aggregates of the invention are coated with one half, one, or several bilayers. These may also be called mixed amphipat coating(s), and correspond to a lipid monolayer, bilayer or oligolayers, respectively.

For a solid aggregate with the surface comprising only one layer of molecules (a monolayer)" the aggregate surface $S_{aggregate}$ given by the product of aggregate number and the exposed single molecule surface $S_{molecule}$:

$$S_{aggregate} = n_a\, S_{molecule}$$

$S_{molecule}$ can either be measured directly, e. g. in a Langmuir trough or with a diffractometric or reflectometric method, or else can be calculated with any suitable computer model (e.g. HyperChem).

An aggregate with a bilayer coating has a surface area only half as large:

$$S_{aggregate}(bilayer, n_a) = 0.5\ S_{aggregate}(monolayer, n_a).$$

"**Aggregate radius**" $r_{\underline{a}}$ for a spherical aggregate is proportional to the square root of the aggregate surface:

$$r_{aggregate} = (S_{aggregate}/4\pi)^{0.5}$$

other aggregate geometries requiring appropriate formula adaptation.

A "**barrier**" in the context of this invention is (as in, for example, EP 0 475 160 and WO 98/17255) a body with through-extending narrow pores, such narrow pores having a radius which is at least 25% smaller than the radius of the ESAs (considered as spherical) before said ESAs permeate through such pores.

The term "**narrow"** used in connection with a pore implies that the pore radius is significantly, typically at least 25%, preferably at least 30% smaller than the radius of the entity tested with regard to its ability to cross the pore. The necessary difference typically should be greater for the narrower pores. Using 25% limit is therefore quite suitable for >150 nm diameter whereas > 100% difference requirement is more appropriate for the smaller systems, e.g. with < 50 nm diameter. For diameters around 20 nm, aggregate diameter difference of at least 200% is often required.

The term "**semipermeable**" used in connection with a barrier implies that a solution can cross transbarrier openings whereas a suspension of non-adaptable aggregates (large enough for the above definition of "narrow" pores to apply, typically 150-200% larger than the diameter of such openings) cannot. Conventional lipid vesicles (liposomes) made from any common phosphatidylcholine in the gel lamellar phase or else from any biological phosphatidylcholine/ cholesterol1/1 mol/mol mixture or else comparably large oil droplets, all having the specified relative diameter, are three examples for such non-adaptable aggregates.

The term "**stable**" means that the tested aggregates do not change their diameter spontaneously or under the transport related mechanical stress (e.g. during passage through a semipermeable barrier) unacceptably, which most often means only to a pharmaceutically acceptable degree. A 20-40% change is normally considered acceptable; the halving or doubling of aggregate diameter is borderline and a greater change in diameter is typically unacceptable. Alternatively and very conveniently, the change in aggregate diameter resulting from pore crossing under pressure is used to assess system stability; the same criteria are then applied as for "narrow" pores, mutatis mutandis. To obtain the correct value for aggregate diameter change, a correction for flux/vortex effects may be necessary. These procedures are described in greater detail in the publication of the applicant in Cevc G., SchAtzlein A., Richardsen H. (2002) Ultradeformable Lipid Vesicles Can Penetrate the Skin and other SemiPermeable Barriers Intact. Evidence from Double Label CLSM Experiments and Direct Size Measurements. Biochim. Biophys. Acta 1564:21-30.

The term "**barrier transport resistance**" describes the resistance of a given barrier to the transport of a given fluid with or without suspended aggregates. Mathematically speaking, this resistance is given by the ratio of transport driving pressure and of transport rate (=flow): resistance = *delta p* / $j_a$. In more qualitative terms, used in some of the examples in this document, barrier resistance is identified with the total fluid volume that can be filtered through a given barrier by certain pressure within given time. Alternatively the pressure needed to achieve certain flux can be used to describe functionally barrier resistance.

Barrier transport resistance generally decreases linearly with the number and total area of pores in the given transport obstacle. For relatively small pores the resistance value can also depend on average pore diameter, mainly due to friction/viscosity effects. In addition to this, barrier transport resistance is sensitive to transported fluid / suspension characteristics and thus strongly depends on the suspended particle adaptability and sometimes concentration. In the first approximation, this later sensitivity is due to elastic and viscous loss during transport.

The term aggregate "**adaptability**" which governs the "tolerable surface curvature" is defined as the ability of a given aggregate to change easily, and essentially reversibly, its properties, such as shape, elongation ratio, and surface to volume ratio. Essential for this invention is the adjustment of aggregate shape and properties to the anisotropic stress caused by pore crossing. Sufficient adaptability implies that an aggregate is able to sustain different unidirectional forces or stress, such as pressure, without significant fragmentation, which defines a "stable" aggregate. If an aggregate passes through a barrier fulfilling this condition the terms "adaptability" and (shape) "deformability" plus "permeability" are essentially equivalent.

Non-destructing passage of ultradeformable, mixed lipid aggregates through narrow pores in a semi-permeable barrier is thus diagnostic of high aggregate adaptability. If pore radius is two times smaller than the average aggregate radius the aggregate must change its shape and surface-to-volume ratio at least 100% to pass without fragmentation through the barrier. An easy and reversible change in aggregate shape inevitably implies high aggregate deformability and requires large surface-to-volume ratio adaptation. A change in surface-to-volume ratio per se implies: a) high volume compressibility, e.g. in the case of compact droplets containing material other than, and immiscible with, the suspending fluid; b) high aggregate membrane permeability, e.g. in the case of vesicles that are free to exchange fluid between inner and outer vesicle volume.

Measuring capability of given aggregate suspension to cross a semi-permeable barrier with narrow pores thus offers simple means for functionally testing aggregate adaptability, as is described in Practical Examples. This capability for suspensions of sufficiently stable aggregates is inversely proportional to the effective barrier transport resistance

and, in the first approximation, to vesicle adaptability $a_v = a_a$ (subscripts *v* and *a* denoting vesicle and aggregate, respectively). If no other adaptability value is available, the inverse value of barrier transport resistance or $1/p^*$ value, which are defined further in the text, can be used to characterise adaptability of aggregates in a suspension.

The adaptability of a vesicle-like aggregate depends on reversible vesicle membrane permeability and deformability. Lipid bilayer permeability can be assessed by the well established methods, such as the osmotic swelling method that is described in many scientific papers and in Phospholipids Handbook, edited by G. Cevc for Marcel Dekker Publishers (New York, 1993). Less directly and quantitatively, but still telling, vesicle bilayer permeability can be checked by comparing the average aggregate diameter before and after pore crossing: vesicle bursting and fragmentation is indicative of aggregate membrane impermeability. In case of lipid vesicles, the latter is identical to lipid bilayer impermeability. Open membrane deformability is governed by lipid bilayer flexibility. This quantity is proportional to bilayer bending elasticity and is hence determined by the elastic membrane bending modulus = the elastic curvature modulus of a bilayer = B. The latter parameter can be measured with several methods known in the art, including pipette aspiration measurements, vesicle shape or fluctuation analysis, bilayer deformation under stress in an atomic force microscope, etc.. Bilayer curvature elastic energy density of a vesicle with radius $r_{ves}$ is given by $B/2r_{ves}^2$, which shows that most elastic/ flexible bilayers, with smallest B-values, are most deformable. For phosphatidylcholine bilayers in the fluid lamellar phase B-value is typically of the order $10^{-19}$ J. This value is at least one order of magnitude higher than the corresponding value determined for a suitable *MDC-MFC* or *MDC- MDC-MFC* mixture, which is B ~ 5 $10^{-17}$ J. This explains why the described three-component amphipat mixtures form very flexible bilayers and highly deformable vesicles.

It is important to realize that any system property that tends to lower aggregate shape adaptability also lowers the likelihood for aggregate motion through the pores with a radius smaller than the average aggregate radius. Incorporation of large incompressible bodies (e.g. oil droplets) into or between the shape-deformable aggregates therefore lowers, if not blocks, trans-barrier transport. Incompressibility of aggregate core has similarly negative effect. Aggregates in the form of (lipid) vesicles suspended in and filled with nearly incompressible water must therefore expel some water from vesicle interior during aggregate deformation to attain high/maximum adaptability. Introduction of membrane stiffening agents (including cholesterol and other sterols, little polar long chain lipids, etc., as quasi-MFC) into bilayers also lowers the adaptability of the resulting mixed aggregates. Vesicle-like aggregates with many bilayer coatings (= membranes) are also relatively non-adaptable (i.e. have lower $a_a$ value, as defined further in the text) and must be pushed with a higher force (i.e. have a higher $p^*$ value, as defined further in the text) through narrow pores than the aggregates with just a few or only one such coating(s). The reasons for this are obvious: in the simplest approximation, aggregate adaptability is inversely proportional to the number of bilayers enshrining liquid care of an aggregate. Further system changes that negatively impact on aggregate adaptability can be analyzed in similar fashion.

If a vesicle can pass through a narrow pore without irreversibly adjusting its diameter to the pore diameter within 50% or even 100% uncertainty range, the vesicle bilayer membrane under terms of this document is declared to be permeable as well as flexible. To assess lipid aggregate adaptability it is therefore useful to employ another aspect of the invention, by using the following method:

1) measure the flux $j_a$ of aggregate suspension through a semi-permeable barrier (e.g. gravimetrically) for different transport-driving trans-barrier pressures *delta p;*

2) calculate the pressure dependence of barrier penetrability *P* for the given suspension by dividing each measured flux value with the corresponding driving pressure value:

$$P\ (delta\ p) = j_a\ (delta\ p) / delta\ p;$$

3) monitor the ratio of final and starting vesicle diameter $2r_{ves}$ *(delta p)*/$2r_{ves,0}$ (e.g. with the dynamic light scattering), wherein $2r_{ves}$ *(delta p)/* is the vesicle diameter after semi-permeable barrier passage driven by *delta p* and $2r_{ves,0}$ is the starting vesicle diameter, and if necessary making corrections for the flow-rate effects;

4) align both data sets *P (delta p) vs.* $r_{ves}$ *(delta p)*/$_{rves,0}$, to determine the coexistence range for high aggregate adaptability and stability; it is also useful, but not absolutely essential, to parameterise experimental penetrability data within the framework of Maxwell-approximation in terms of the necessary pressure value $p^*$ and of maximum penetrability value $P_{max}$, which are defined graphically in the following illustrative schemes.

Figures 1 to 4 illustrate schematically the physical and molecular principles underlying the abovementioned approach and the mathematical model used to analyse the corresponding experimental data.

It is plausible to sum-up all the contributions to a moving aggregate energy (deformation energy/ies, thermal energy, the shearing work, etc.) into a single, total energy. The equilibrium population density of aggregate's energetic levels then may be taken to correspond to Maxwell's distribution. All aggregates with a total energy greater than the activation energy, $E > E_A$, are finally concluded to penetrate the barrier. The pore-crossing probability for such aggregates is then given by:

$$P(e) = 1 - \mathrm{erf}\left(\sqrt{\frac{1}{e}}\right) + \sqrt{\frac{4}{\pi e}} \cdot \exp\left[-\frac{1}{e}\right],$$

*e* being dimensionless aggregate energy in units of the activation energy $E_A$.

It is therefore plausible to write barrier penetrability to a given suspension as a function of transport driving pressure (= driving pressure difference) $p$ (= *delta p)* as:

$$P(P_{max}, p^*, p) = P_{max} \cdot \left\{1 - \mathrm{erf}\left(\sqrt{\frac{p^*}{p}}\right) + \sqrt{\frac{4p^*}{\pi p}} \cdot \exp\left[-\frac{p^*}{p}\right]\right\} \qquad (*)$$

$P_{max}$ is the maximum possible penetrability of a given barrier. (For the aggregates with zero transport resistance this penetrability is identical to the penetrability of the suspending medium flux.) $p^*$ is an adjustable parameter that describes the pressure sensitivity, and thus the transport resistance, of the tested system. (For barriers with a fixed pore radius this sensitivity is a function of aggregate properties solely. For non-interacting particles the sensitivity is dominated by aggregate adaptability, allowing to make the assumption: $a_a$ proportional to $1/p^*$.)

In a presently preferred embodiment of the invention, the experimental approach to quantitative aggregate adaptability determination is to identify vesicle adaptability value with the inverse pressure difference needed to attain certain predefined, practically relevant fraction of maximum achievable flux-pressure ratio with the vesicle suspension; using 50-60% maximum penetrability criterion ($P_{max}$) gives reasonable results. Specifically, all $p^*$ values given in this document correspond to 57% of $P_{max}$-value. Adaptability value, up to an uninteresting constant, is then given by the inverse value of the $p^*$ value that corresponds to 57% of the $P_{max}$-value.

By making a few more reasonable suppositions one can use the experimentally determined $p^*$-value to calculate the activation energy $E_A$ for transbarrier transport of adaptable vesicular aggregates. The dominant energetic contribution to the work of bilayer deformation - bilayer elastic energy; bilayer permeabilisation energy, as the case may be - can then be deduced from $E_A$-value. Finally, bilayer elastic energy can be translated into bilayer curvature elastic energy density, which depends on the elastic curvature modulus of bilayer, *B,* as is explained earlier in the text. Bilayer permeabilisation energy independently can be related to the work needed to break a bilayer membrane, and thus to bilayer lysis tension, assuming that elastic energy is much smaller than membrane permeabilisation energy. For simple lipid vesicles this has been done by the group of B. Frisken (cf. Biophys. J. 74: 2996-3002 (1998) and Langmuir 16: 928--933 (2000)), amongst others. Such detailed analysis is not necessary for optimising aggregate suspensions for trans-barrier transport, however, and therefore is not used in the present application.

The "**liquid suspending medium**" or "liquid medium" or "suitable liquid medium" is defined in EP 0 475 160 and in WO 98/17255.

An **"amphipat"** (or an amphipatic component) is any substance capable of forming an ESA or of modifying the adaptability of an ESA, when brought into contact with the liquid suspending medium.

For the broadest definition, the amphipats are divided into two subgroups, the "**membrane forming compounds" (*MFC*s)** or "surface building" or "extended surface-forming or "surface-supporting substance", which are capably of forming extended surface aggregates (ESAs), and "**the membrane destabilising compounds" (*MDC*s)**. The latter typically render the ESAs formed by the *MFCs* more adaptable.

In some aspects the three amphipatic compounds, one *MFC* and two *MDC*s forming the ESAs are then defined that the *MFC* alone forms ESAs, the one *MDC* alone forms small aggregates, the other *MDC* alone optionally forms small aggregates and the combination of both *MDC*s forms small aggregates, in contact with said liquid suspending medium. The ESAs and the small aggregates being defined in terms of aggregation numbers as stated above.

In some aspects the three amphipatic compounds, one *MFC* and two *MDC*s forming the ESAs are then characterised by their solubility in the liquid suspending medium. The *MFC*s are then defined to be less soluble than the

*MDC*s at least by a factor of 2. In more preferred embodiments the *MFC*s are then defined to be less soluble than the *MDC*s at least by a factor of 10 and in preferred embodiments the solubilities of the two *MDC*s differ at least by a factor of 2. Alternatively or simultaneously the *MFCs* are defined to be less soluble than the *MDC*s at least by a factor of 10, one *MDC* forms aggregates with surfaces that are at least 2 times less extended than the surfaces of aggregates formed by the *MFC* and the other *MDC forms aggregates with aggregation numbers at least 10 times smaller than* the aggregation numbers of aggregates formed by the *MFC*. Yet another possibility is to define *MDC* as molecules, which are typically characterised by hydrophilicity-lipophilicity ratio (HLB) between 10 and 20, even better between 12 and 18 and most preferred between 13 and 17.

In some aspects the *MFC* and *MDC*s are defined to form in the combination of one *MFC* and two different *MDC*s extended surface aggregates with surfaces that are at least 50% more extended, extended meaning larger, on the average than surfaces of aggregates comprising only the two different *MDC*s alone, at the same concentrations and, in case, after adjustment for physico-chemical effects of the absence of said *MFC.*

For some aspects a selection or all definitions at once apply.

Within the meaning of the present invention the MFC is preferably a lipid and more preferably a phospholipids as defined below.

The amphipats within the meaning of the present invention comprise the membrane forming substances and the "edge-active (surface active)" substances also known from EP 0 475 160 and WO 98/17255, but within the limitations defined in the attached claims.

The term "**drug**" means a biologically or therapeutically active ingredient, e.g. a medicament. Unless indicated otherwise, the generic names proposed by the world Health Organisation (WHC)) (Recommended International Non-proprietary Names), such as can be found e.g. in the Merck Index, are used for the drugs, which are specified in greater detailed further in the text.

The term "**low**" used in connection with molecular weight of a polypeptide means molar mass below 1500 and the term "intermediate" in similar context implies molar mass between 1500 and 5000.

The term **"lower"** used in connection with organic radicals, for example lower alkyl, lower alkylene, lower alkoxy, lower alkanoyl, etc., means that such organic radicals, unless expressly defined otherwise, contain up to and including 7, preferably up to and including 4, carbon atoms.

The term "**long"** used in connection with a fatty residue attached to a lipid, a surfactant or a drug implies the presence of 10 to 24 carbon atoms in alkyl, alkenyl, alkoxy, alkenyloxy or acyloxy chains, which individually or together, as the case may be, bear the class name of "fatty chains". Implicitly included in this term, but not further specified in detail, are "fatty chains" with at least one branched or a cyclic, but unpolar or little polar, segment.

The use of square brackets in the text relates to molar concentrations of the substance put between the brackets, except if indicated otherwise.

The terms "**surface active"** and "**edge active**" relates to the ability of a certain third compound to change the surface tension and/or interface tension in systems comprising at least two compounds forming a surface or interface.

In this specification the terms "**compound**", "**substance**" and "**component**" generally indicate a single chemical species, which needs, however, not to be totally uniform.

The term "**apparent dissociation constant**" refers to the measured dissociation (i.e. ionisation) constant of a drug. This constant for many drugs, including NSAIDs, is different in the bulk and in the homo- or heteroaggregates. For ketoprofen, the pKa in the bulk is approx. 4.4 whereas the pKa value measured above the drug association concentration is approx. 5, and decreases approximately linearly with the inverse ionic strength of the bulk solution. pKa of ketoprofen bound to lipid bilayers increases with total lipid concentration as well, and is approx. 6 and 6.45 in suspensions with 5 w-% and 16 w-% total lipid in a 50 mM monovalent buffer, respectively. For diclofenac, the pKa in the bulk is around 4, whereas for this drug in lipid bilayers pKa ~ 6.1 was determined. The bulk pKa reported in the literature for meloxicam, piroxicam, naproxen, indomethacin and ibuprofen is 4.2 (and 1.9), 5.3, 4.2-4.7, 4.5, and 4.3 (or in some reports 5.3), respectively.

The term aggregate "**deformability**" is closely related to the term "adaptability" Any major change in aggregate shape that does not result in a significant aggregate fragmentation is indicative of sufficient aggregate deformability, and also implies a large change in the deformed aggregate surface-to-volume ratio Deformability can therefore be measured in the same kind of experiments as is proposed for determining aggregate adaptability, or else can be assessed by optical measurements that reveal reversible shape changes.

The term "**NSAID**" (nonsteroidal anti-inflammatory drug) typically indicates a chemical entity which acts as lipoxygenase, cyclooxygenase-1 or cyclooxygenase-2 antagonist.

Examples include salts of substituted phenylacetic acids or 2-phenylpropionic acids, such as alclofenac, ibufenac, ibuprofen, clindanac, fenclorac, ketoprofen, fenoprofen, indoprofen, fenclofenac, diclofenac, flurbiprofen, pirprofen, naproxen, benoxaprofen, carprofen or cicloprofen; analgesically active heteroarylacetic acids or 2-heteroarylpropionic acids having a 2-indol-3-yl or pyrrol-2-yl radical, for example indomethacin, oxmetacin, intrazol, acemetazin, cinmetacin, zomepirac, tolmetin, colpirac or tiaprofenic acid; analgesically active indenylacetic acids, for example sulindac; analgesically active heteroaryloxyacetic acids, for example benzadac; NSAIDS from oxicame family include piroxicam, droxicam, meloxicam, tenoxicam; further interesting drugs from NSAID class are, meclofenamate, etc.

A list of commonly used NSAIDs is given in the following table:

| NSAID | Some common trade names |
|---|---|
| Acetaminofene | Tylenol |
| Cimicifuga | Artrol |
| Choline salicylate-Mg salicylate | Trilisate |
| Diclofenac | as Na salt: Apo-Diclo, Apo-Diclo SR, Arthrotec, Diclofenac Ect, Novo-Difenac, Novo-Difenac SR, Nu-Diclo, Taro-Diclofenac, Voltaren, Voltaren SR; as K salt: Voltaren Rapide |
| Diflunisal | Apo-Diflunisal, Dolobid, Novo-Diflunisal, Nu-Diflunisal |
| Etodolac | Ultradol |
| Fenoprofen calcium | Nalfon |
| Floctafenine | Idarac |
| Flurbiprofen | Ansaid, Apo-Flurbiprofen FC, Froben, Froben SR, Novo-Flurprofen, Nu-Flurbiprofen |
| Ibuprofen | Actiprofen, Advil, Advil Cold & Sinus, Amersol, Apo-Ibuprofen, Excedrin IB, Medipren, Motrin, Motrin IB, Novo-Profen, Nuprin, Nu-Ibufrofen |
| Indomethacin | Apo-Indomethacin, Indocid, Indocid SR, Indolec, Novo-Methacin, Nu-Indo, Pro-Indo, Rhodacine |
| Ketoprofen | Apo-Keto, Apo-Keto-E, Novo-Keto, Novo-Keto-Ec, Nu-Ketoprofen, Nu-Ketoprofen-E, Orudis, Orudis E, Orudis SR, Oruvail, PMS-Ketoprofen, PMS-Ketoprofen-E, Rhodis, Rhodis-EC |
| Ketorolac tromethamine | Acular, Toradol |
| Magnesium salicylate | Back-Ese-M, Doan's Backache Pills, Herbogesic |
| Mefenamic acid | Ponstan |
| Nabumetone | Relafen |
| Naproxen | Apo-Naproxen, Naprosyn, aprosyn-E, Naxen, Novo-Naprox, Nu-Naprox, PMS-Naproxen; or in the sodium form: Anaprox, Anaprox DS, Apo-Napro-Na, Naproxin-Na, Novo-Naprox Sodium, Synflex, Synflex DS |
| Oxyphenbutazone | Oxybutazone |
| Phenylbutazone | Alka Phenyl, Alka Phenylbutazone, Apo-Phenylbutazone, Butazolidin, Novo-Butazone, Phenylone Plus |
| Piroxicam | Apo-Piroxicam, Feldene, Kenral-Piroxicam, Novo-Pirocam, Nu-Pirox, PMS-Piroxicam, Pro-Piroxicam, Rho-Piroxicam |
| Salsalate | Disalcid |
| Sodium salicylate | Apo-Sulin, Dodd's, Dodd's Extra-Strength, Sulindac, Clinoril, Novo-Sundac, Nu-Sulindac, Sulindac |
| Tenoxicam | Mobiflex |
| Tiaprofenic acid | Albert Tiafen, Apo-Tiaprofenic, Surgam, Surgam SR |
| Tolmetin sodium | Novo-Tolmetin, Tolectin |

The term "**phospholipid**" has, for example, the formula

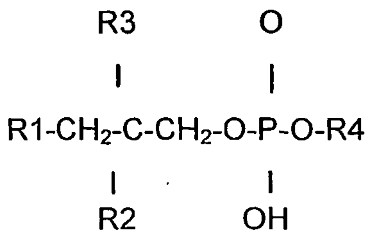

(1)

in which one of the radicals R1 and R2 represents hydrogen, hydroxy or C1-C4-alkyl, and the other radical represents a long fatty chain, especially an alkyl, alkenyl, alkoxy, akenyloxy or acyloxy, each having from 10 to 24 carbon atoms, or both radicals R1 and R2 represent a long fatty chain, especially an alkyl, alkenyl, alkoxy, alkenyloxy or acyloxy each having from 10 to 24 carbon atoms, R3 represents hydrogen or C1-C4-alkyl, and R4 represents hydrogen, optionally substituted C1-C7-alkyl or a carbohydrate radical having from 5 to 12 carbon atoms or, if both radicals R1 and R2 represent hydrogen or hydroxy, R4 represents a steroid radical, or is a salt thereof. The radicals R1, R2, R3, and R4 are typically selected so as to ensure that lipid bilayer membrane is in the fluid lamellar phase during practical application and is a good match to the drug of choice.

In a phospholipid of the formula 1, R1, R2 or R3 having the meaning C1-C4-alkyl is preferably methyl, but may also be ethyl, *n*-propyl, or *n*-butyl.

The terms alkyl, alkenyl, alkoxy, akenyloxy or acyloxy have their usual meaning. The long fatty chains attached to a phospholipid can also be substituted in any of usual ways.

Alkyl R1 or R2 is preferably straight-chained with an even number of 10 to 24 carbon atoms, for example *n*-decyl, *n*-dodecyl (lauryl), *n*-tetradecyl (myristyl), *n*-hexadecyl (cetyl), *n*-octadecyl (stearyl), *n*-eicosyl (arachinyl), *n*-docosyl (behenyl) or *n*-tetracosyl (lignoceryl). In this and all the related following definitions, the intermediate odd-numbered derivatives are useful, but are less preferred.

Alkenyl R1 and/or R2 is preferably straight-chained with an even number of 12 to 24 carbon atoms and a double bond, for example 9-*cis*-dodecenyl (lauroleyl), 9-*cis*-tetradecenyl (myristoleyl), 9-*cis*-hexadecenyl (palmitoleinyl), 9-*cis*-octadecenyl (petroselinyl), 6-*trans*-octadecenyl (petroselaidinylj, 9-*cis*-octadecenyl (oleyl), 9-*trans*-octadecenyl (elaidinyl), 9-*cis*-eicosenyl (gadoleinyl), 9-*cis*-docosenyl (cetoleinyl) or 9-*cis*-tetracosoyl (nervonyl). In this and all the related following definitions, the other corresponding trans-derivatives are potentially useful as well but are less preferred.

Alkoxy R1 and/or R2 is preferably straight-chained with an even number of 10 to 24 carbon atoms, for example *n*-decyloxy, *n*-dodecyloxy (lauryloxy), *n*-tetradecyloxy (myristyloxy), *n*-hexadecyloxy (cetyloxy), *n*-octadecyloxy (stearyloxy), *n*-eicosyloxy (arachinyloxy), *n*-docosoyloxy (behenyloxy) or *n*-tetracosoyloxy (lignoceryloxy).

Alkenyloxy R1 and/or R2 is preferably straight-chained with an even number of 12 to 24 carbon atoms, for example 9-*cis*-dodecenyloxy (lauroleyloxy), 9-*cis*-tetradecenyloxy (myristoleyloxy), 9-*cis*-hexadecenyloxy (palmitoleinyloxy), 6-*cis*-octadecenyloxy, (petroselinyloxy), 6-*trans*-octadecenyloxy (petroselaidinyloxy), 9-*cis*-octadecenyloxy (oleyloxy), 9-*trans*-octadecenyloxy (elaidinyloxy), and 9-*cis*-eicosenyl (gadoleinyloxy), 9-*cis*-docosenyl (cetoleinyloxy) or 9-*cis*-tetracosoyl (nervonyloxy).

Acyloxy R1 and/or R2 is preferably straight-chained with an even number of 10 to 24 carbon atoms, for example alkanoyloxy or alkenoyloxy, preferably *n*-decanoyloxy, *n*-dodecanoyloxy (lauroyloxy), *n*-tetradecanoyloxy (myristoyloxy), *n*-hexadecanoyloxy (palmitoyloxy), *n*-octadecanoyloxy (stearoyloxy), *n*-eicosanoyloxy (arachinoyloxy), *n-n*-docosoanyloxy (behenoyloxy) and *n*-tetracosanoyloxy (lignoceroyloxy).

Alkenoyloxy R1, and/or R2 is preferably straight-chained with an even number of 10 to 20 carbon atoms, for example 9-*cis*-dodecenyloxy (lauroleoyloxy), 9-*cis*-tetradecenoyloxy (myristoleoyloxy), 9-*cis*-hexadecenoyloxy (palmitoleinoyloxy), 6-*cis*-octadecenoyloxy (petroselinoyloxy), 6-*trans*-octadecenoyloxy (petroselaidinoyloxy), 9-*cis*-octadecenoyloxy (oleoyloxy), 9-*trans*-octadecenoyloxyelaidinoyloxy), and 9-*cis*-eicosenoyloxy (gadoleinoyloxy), 9-*cis*-docosenoyloxy (cetoleinoyloxy) and 9-*cis*-tetracosenoyloxy (nervonoyloxy).

Optionally substituted C1-C7-alkyl R4 is, for example, methyl, ethyl, isopropyl, *n*-propyl, isobutyl or *n*-butyl, which can be substituted by acidic groups, for example, carboxy or sulpho, by acidic and basic groups, for example, carboxy and amino, the amino group being in the alpha-position to the carboxy group, by free or etherified hydroxy groups, it being possible for two etherified hydroxy groups to be bonded to one another by a bivalent hydrocarbon radical, for example methylene, ethylene, ethylidene, 1,2-propylene or 2,2- propylene; or by halogen, for example chlorine or bromine, by lower alkoxycarbonyl, for example methoxy-or ethoxy-carbonyl, or by lower alkanesulphonyl, for example methanesulphonyl.

Substituted C1-C7-alkyl R4 is, for example, carboxy-lower alkyl, for example carboxymethyl, 2-carboxyethyl or 3-carboxy-*n*-propyl, (omega-amino-omega-carboxy-lower alkyl, for example 2-amino-2-carboxyethyl or 3-amino-3-carboxy-*n*-propyl, hydroxy-lower alkyl, for example 2-hydroxyethyl or 2,3-dihydroxypropyl, lower alkoxy-lower alkyl, for example methoxy- or ethoxymethyl, 2-methoxyethyl or 3-methoxy-*n*-propyl, lower alkylenedioxy-lower alkyl, for example 2,3- ethylenedioxypropyl or 2,3-(2,2-propylene)-dioxypropyl, or halo-lower alkyl, for example chloro- or bromo-methyl, 2-chloro- or 2-bromo-ethyl, 2- or 3-chloro- or 2- or 3-bromo-*n*-propyl.

Substituted C1-C7-alkyl R4 is preferably ethyl substituted by tri-lower alkylammonium, for example trimethyl- or triethyl-ammonium, for example 2-trimethylammonium-ethyl or 2-ammonium-ethyl, or is, for example omega-amino-omega-carboxy-lower alkyl, for example 2-amino-2-carboxyethyl.

A carbohydrate radical R4 having from 5 to 12 carbon atoms is, for example, a natural monosaccharide radical that is derived from a pentose or hexose present in the form of aldose or ketose. Detailed definitions of most relevant carbohydrate radicals (pentoses, hexoses, disaccharides, etc.) is given in the patent EP 0 475 160 by the same applicant.

A steroid radical R4 is, for example, a sterol radical that is esterified by the phosphatidyl group by way of the hydroxy group located in the 3-position of the steroid nucleus.

A sterol radical is, for example, the lanosterol, sitosterol, coprostanol, cholestanol, glycocholic acid, ergosterol or stigmasterol radical, preferably the cholesterol radical.

If R4 represents a steroid radical, R1 and R2 are preferably hydroxy and R3 is hydrogen.

Phospholipids of the formula 1 can be in the form of free acids or in the form of salts. Salts are formed by reaction of the free acid of the formula II with a base, for example a dilute, aqueous solution of alkali metal hydroxide, for example lithium, sodium or potassium hydroxide, magnesium or calcium hydroxide, a dilute aqueous ammonia solution or an aqueous solution of an amine, for example a mono-, di- or tri-lower alkylamine, for example ethyl-, diethyl- or triethyl-amine, 2-hydroxyethyl-tri-C1-C4-alkyl-amine, for example choline, and a basic amino acid, for example lysine or arginine.

A phospholipid of the formula 1 has especially two acyloxy radicals R1 and R2, for example alkanoyloxy or alkenoyloxy, for example lauroyloxy, myristoyloxy, palmitoyloxy, stearoyloxy, arachinoyloxy, oleoyloxy, linoyloxy or linoleoyloxy, and is, for example, natural lecithin (R3 = hydrogen, R4 = 2-trimethylammonium ethyl) or cephalin (R3 = hydrogen, R4 = 2-ammonium ethyl) having different acyloxy radicals R1 and R2, for example egg lecithin or egg cephalin or lecithin or cephalin from soy beans, synthetic lecithin (= phosphatidylcholine) or cephalin (= phosphatidylethanolamine) having different or identical acyloxy radicals R1 and R2, for example 1-palmitoyl-2-oleoyl phosphatidylcholine or phosphatidylethanolamine or dipalmitoyl, distearoyl, diarachinoyl, dioleoyl, dilinoyl or dilinoleoyl phosphatidylcholine or phosphatidylethanolamine, natural phosphatidyl serine (R3 = hydrogen, R4 = 2-amino-2-carboxyethyl) having different acyloxy radicals R1 and R2, for example phosphatidyl serine from bovine brain, synthetic phosphatidylserine having different or identical acyloxy radicals R1 and R2, for example dioleoyl, dimyristoyl- or dipalmitoyl-phosphatidyl serine, or natural phosphatidic acid (R3 and R4 = hydrogen) having different acyloxy radicals R1 and R2.

A phospholipid of the formula 1 is also a phospholipid in which R1 and R2 represent two identical alkoxy radicals, for example *n*-tetradecyloxy or *n*-hexadecyloxy (synthetic ditetradecyl or dihexadecyl phosphatidylcholine or phosphatidylethanolamine), R1 represents alkenyl and R2 represents acyloxy, for example myristoyloxy or palmitoyloxy (plasmalogen, R3 = hydrogen, R4 = 2-trimethylammonium ethyl), R1 represents acyloxy and R2 represents hydroxy (natural or synthetic lysophosphatidylcholine or lysophosphatidylethanolamine, for example 1-myristoyl- or 1-palmitoyl-lysophosphatidylcholine or - phosphatidylethanolamine; natural or synthetic lysophosphatidyl serine, R3 = hydrogen, R4 = 2-amino-2-carboxyethyl, for example lysophosphatidyl serine from bovine brain or 1-myristoyl- or 1-palmitoyl-lysophosphatidyl serine, synthetic lysophosphatidyl glycerine, R3 = hydrogen, R4 = $CH_2OH$-CHOH-$CH_2$-, natural or synthetic lysophosphatidic acid, R3 = hydrogen, R4 = hydrogen, for example egg lysophosphatidic acid or 1-lauroyl-, 1-myristoyl- or 1-palmitoyl-lysophosphatidic acid).

A lipid that is analogous to abovementioned phospholipid and can replace the latter is, for example, a lysophosphatidylcholine analogue, for example 1-lauroyl-1,3-propanediol-3- phosphoryl choline, a monoglyceride, for example monoolein or monomyristin, a cerebroside, a ganglioside, or a glyceride that does not contain a free or esterified phosphoryl or phosphonyl group in the 3-position, for example a diacylglyceride or 1-alkenyl-1-hydroxy-2-acylglyceride, having the mentioned acyl or alkenyl groups in which the 3-hydroxy group is etherified by one of the mentioned carbohydrate radicals, for example a galactosyl radical, for example monogalactosyl glycerine.

The lipids and certain surfactants mentioned hereinbefore and hereinafter having a chiral carbon atom can be present both in the form of racemic mixtures and in the form of optically pure enantiomers in the pharmaceutical compositions that can be prepared and used according to the invention.

The term "**sterol radical**" means, for example, the lanosterol, sitosterol, coprostanol, cholestanol, glycocholic acid, ergosterol or stigmasterol radical, is preferably the cholesterol radical, but can also be any other sterol radical known in the art.

The term "**surfactant**" also has its usual meaning. A long list of relevant surfactants and surfactant related definitions is given in EP 0 475 160 and USP 6 165 500 which are herewith explicitly included by reference and in appropriate surfactant or pharmaceutical Handbooks, such as *Handbook of Industrial Surfactants* or US Pharmacopoeia, Pharm. Eu., etc.. The following list therefore only offers a selection, which is by no means complete or exclusive, of

several surfactant classes that are particularly common or useful in conjunction with present patent application. This includes ionised long-chain fatty acids or long chain fatty alcohols, long chain fatty ammonium salts, such as alkyl- or alkenoyl-trimethyl-, -dimethyl- and -methyl-ammonium salts, alkyl- or alkenoyl-sulphate salts, long fatty chain dimethyl-aminoxides, such as alkyl- or alkenoyl-dimethyl-aminoxides, long fatty chain, for example alkanoyl, dimethyl-aminoxides and especially dodecyl dimethyl-aminoxide, long fatty chain, for example alkyl-N-methylglucamides and alkanoyl-N-methylglucamides, such as MEGA-8, MEGA-9 and MEGA-10, N-long fatty chain-N,N-dimethylglycines, for example N-alkyl-N,N-dimethylglycines, 3-(long fatty chain-dimethylammonio)-alkanesulphonates, for example 3-(acyldimethylammonio)-alkanesulphonates, long fatty chain derivatives of sulphosuccinate salts, such as bis(2-ethylalkyl) sulphosuccinate salts, long fatty chain-sulphobetaines, for example acyl-sulphobetaines, long fatty chain betaines, such as EMPIGEN BB or ZWITTERGENT-3-16, -3-14, -3-12, -3-10, or -3-8, or polyethylen-glycol-acylphenyl ethers, especially nonaethylen-glycol-octylphenyl ether, polyethylene-long fatty chain-ethers, especially polyethylene-acyl ethers, such as nonaethylen-decyl ether, nonaethylen-dodecyl ether or octaethylene-dodecyl ether, polyethyleneglycol-isoacyl ethers, such as octaethyleneglycol-isotridecyl ether, polyethyleneglycol-sorbitane-long fatty chain esters, for example polyethyleneglycol-sorbitane-acyl esters and especially polyethylenglykol-monolaurate (e.g. Tween 20), polyethylenglykol-sorbitan-monooleate (e.g. Tween 80), polyethylenglykol-sorbitan-monolauroleylate, polyethylenglykol-sorbitan-monopetroselinate, polyethylenglykol-sorbitan-monoelaidate, polyethylenglykol-sorbitan-myristoleylate, polyethylenglykol-sorbitan-palmitoleinylate, polyethylenglykol-sorbitan-petroselinylate, polyhydroxyethylene-long fatty chain ethers, for example polyhydroxyethylene-acyl ethers, such as polyhydroxyethylene-lauryl ethers, polyhydroxyethylene-myristoyl ethers, polyhydroxyethylene-cetylstearyl, polyhydroxyethylene-palmityl ethers, polyhydroxyethylene-oleoyl ethers, polyhydroxyethylene-palmitoleoyl ethers, polyhydroxyethylene-linoleyl, polyhydroxyethylen-4, or 6, or 8, or 10, or 12-lauryl, miristoyl, palmitoyl, palmitoleyl, oleoyl or linoeyl ethers (Brij series), or in the corresponding esters, polyhydroxyethylen-laurate, -myristate, -palmitate, - stearate or -oleate, especially polyhydroxyethylen-8-stearate (Myrj 45) and polyhydroxyethylen-8-oleate, polyethoxylated castor oil 40 (Cremophor EL), sorbitane-mono long fatty chain, for example alkylate (Arlacel or Span series), especially as sorbitane-monolaurate (Arlacel 20, Span 20) or monooleate, long fatty chain, for example acyl-N-methylglucamides, alkanoyl-N-methylglucamides, especially decanoyl-N-methylglucamide, dodecanoyl-N-methylglucamide or octadecanoyl-N-methylglucamide, long fatty chain sulphates, for example alkyl-sulphates, alkyl sulphate salts, such as lauryl-sulphate (SDS), oleoyl-sulphate; long fatty chain thioglucosides, such as alkylthioglucosides and especially heptyl-, octyl-, nonyl and decyl-beta-D-thioglucopyranoside; long fatty chain derivatives of various carbohydrates, such as pentoses, hexoses and disaccharides, especially alkyl-glucosides and maltosides, such as hexyl-, heptyl-, octyl-, nonyl- and decyl-beta-D-glucopyranoside or D-maltopyranoside; further a salt, especially a sodium salt, of cholate, deoxycholate, glycocholate, glycodeoxycholate, taurodeoxycholate, taurocholate, a fatty acid salt, especially oleate, elaidate, linoleate, laurate, or myristate, most often in sodium form, lysophospholipids, n-octadecylene-glycero-phosphatidic acid, octadecylene-phosphorylglycerol, octadecylene-phosphorylserine or phosphatidylcholine, *n*-long fatty chain-glycero-phosphatidic acids, such as *n*-acyl-glycero-phosphatidic acids, especially lauryl glycero-phosphatidic acids, oleoyl-glycero-phosphatidic acid, *n*-long fatty chain-phosphorylglycerol, such as *n*-acyl-phosphorylglycerol, especially lauryl-, myristoyl-, oleoyl- or palmitoeloyl-phosphorylglycerol, *n*-long fatty chain-phosphorylserine, such as *n*-acyl-phosphorylserine, especially lauryl-, myristoyl-, oleoyl- or palmitoeloyl-phosphorylserine, *n*-tetradecyl-glycero-phosphatidic acid, *n*-tetradecyl-phosphorylglycerol, *n*-tetradecyl-phosphorylserine, the corresponding elaidoyl-, vaccenyl-lysophospholipids, the corresponding short-chain phospholipids, as well as all surface active and thus membrane destabilising-polypeptides. Surfactant chains are typically chosen to be in a fluid state or at least to be compatible with the maintenance of fluid-chain state in carrier aggregates.

The term "**surfactant like phospholipid**" means a phospholipid with solubility, and other relevant properties, similar to those of the corresponding surfactants mentioned in this application, especially in the Claims 104 and 105. A non-ionic surfactant like phospholipid therefore should have water solubility, and ideally also water diffusion / exchange rates, etc., similar to those of a relevant non-ionic surfactant.

The lipids and certain surfactants mentioned hereinbefore and hereinafter having a chiral carbon atom can be present both in the form of racemic mixtures and in the form of optically pure enantiomers in the pharmaceutical compositions that can be prepared and used according to the invention.

## Description of Figures

**Figure 1**: Shape deformation: Schematic representation of aggregate shape deformation during pore crossing.

**Figure 2:** Energy cost deformation: Energy level associated with different states of aggregate deformation that result from an enforced aggregate passing through a narrow pore in a semi-permeable barrier.

**Figure 3**: Deformation & Penetrability: Penetrability of a semi-permeable porous barrier to the suspension of vesicles smaller the average pore diameter in the barrier as a function of transbarrier pressure which drives the suspension through the barrier.

**Figure 4**: Molecular redistribution in an aggregate-enshrining lipid bilayer during aggregate deformation and pore crossing, which lowers the activation energy for transbarrier transport.

**Figure 5:** Schematic illustration of the role played by membrane destabilising component(s) on lipid bilayer adaptability. The effect of relative concentration of the second membrane destabilising component is shown in inset. Bilayer vesicle adaptability as a function of absolute concentration of membrane destabilising components (surfactants; $MDC_1$ + $MDC_2$) and of the relative concentration of such components in the mixed, three component bilayers based on a lipid *(MFC)*, as the membrane forming component. The presence of second membrane destabilising component can increase bilayer adaptability disproportionally, arguably by increasing lipid bilayer deformability and permeability. This is presumably due to $MDC_1$-$MDC_2$ coupling/interaction, which here is proportional to the parameter *m*; although not represented, *MFC*-$MDC_1$ or MFC-$MDC_2$ coupling/interaction can be similarly important. Absolute adaptability values and precise curve form depend on specific choice of model parameters and therefore can be different from the shown ones.

**Figure 6:** Effect of second membrane destabilising amphipat (SDS; $MDC_2$) in the mixed phospholipid (SPC, *MFS)* Tween 80 (first membrane destabilising amphipat, $MDC_1$) bilayers with increasing relative concentration of the latter on Barrier Transport Resistance, measured with a simple experimental method (SEM). The curves are drawn merely to guide the eye.The effect of changing molar ratio of the second (Tween80=) and the third (surfactant; SDS) amphipatic system component, relatively to the first amphipatic system component (phospholipid; SPC), on the resistance of resulting mixed lipid suspension to the filtration through a barrier with 0.2 micrometer pore-diameter (left panel) is shown. The starting and final vesicle diameter was significantly greater than the average pore diameter.

**Figure 7**: Pressure dependence barrier penetrability to three different suspensions of mixed bilayer vesicles, pushed through narrow pores, as a function of the second surfactant concentration. Exemplified is the effect of a charged biosurfactant, sodium cholate, in mixtures with another surfactant (Tween 80) containing phospholipid bilayers on the ability of the resulting lipid vesicle suspensions to penetrate through a semipermeable barrier under influence of transbarrier hydrostatic pressure. Pressure dependence barrier penetrability to three different suspensions of mixed bilayer vesicles, pushed through narrow pores, as a function of the second surfactant concentration.

**Figure 8**: illustrates penetrability of the suspensions prepared as described in examples 143 (•) and 144 (○). The curves were calculated within the framework of Maxwell's energy distribution model, by using formula (*).

**Figure 9**: Penetration curves for different SPC/KT mixtures: Δ=2.5/1 SPC/KT, ♦ 3/1 SPC/KT, V 4/1 SPC/KT, □ SPC/Tween 1/1 Transfersomes® as a Reference suspension. The curves were calculated within the framework of the data fitting model described herein before, by using eq. (*).

**Figure 10**: Penetration curves for SPC/KT 3/1 mole/mole formulation without (o) and with (•)10 rel-mol% of Tween 80. Reference Tween-Transfersomes® *. The curves were fitted to the data using eq. (*).

**Figure 11:** Area under the curve (AUC), which reflects the cumulative delivery of the drug, calculated from the pharmacokinetic results measured with different ketoprofen (KT) formulations tested in pigs (n = 4).

**Detailed description of the Invention**

The invention describes suspensions of complex ESAs with at least three amphipatic components, one of which is membrane forming and at least two of which are membrane destabilising, which can be suspended in a suitable, e.g. pharmaceutically acceptable, polar liquid medium and loaded with at least one biologically active compound, which can correspond to one of the amphipats. An essential characteristic of such, relatively large, aggregates is the ability to penetrate pores in semi-permeable barriers even when the pore radius is significantly, i.e. at least 25% and often is more than 40% or even better more than 50% and most preferably is more than 70% smaller than the average aggregate radius before barrier crossing. Another important characteristic of aggregates introduced in this document is the relatively low concentration of one of the two membrane destabilising components, which is below the concentration needed to achieve high aggregate shape deformability when this component is used for the purpose on its own. High aggregate deformability is a prerequisite for reaching practically useful-i.e. sufficiently high-suspension flux through a barrier, such

that approaches in order of magnitude the flux of suspending medium. The other necessary condition is sufficient aggregate stability, which ensures that the average aggregate radius after barrier crossing is still at least 40%, more often is at least 50% and most typically is at least 100% larger than the pore radius. High deformability and sufficient stability of aggregates that can cross semipermeable barriers are sub-summarised in the term aggregate adaptability, which is parameterised as $a_a$. Highly adaptable complex aggregates excel in their ability to transport active ingredients through semi-permeable barriers, such as mammalian skin.

The present invention specially relates to the selection of one membrane destabilizing amphipatic component of the system such that can boost the deformability of mixed aggregates supported by judicious choice of the other system components to the effect of improving barrier penetration by such aggregates. The invention also teaches how to select the right total amphipat concentration of and, in case, amphipat ionisation in mixed aggregate suspensions. The invention further relates to the preparation and application of resulting suspensions in pharmaceutical formulations, with a focus on epicutaneous application on, or less frequently in, the warm blood creatures.

We discovered unexpectedly that incorporation of an additional, suitable amphipatic membrane destabilising component ($MDC_2$) in aforementioned bi-component ($MFC+MDC_1$) aggregates can increase the resulting three-component ($MFC + MDC_1 + MDC_2$) aggregate adaptability $a_a(MFC + MDC_1 + MDC_2) > a_a(MFC + MDC_1)$ and thus augments the shape deformability of resulting aggregates. This lowers the pressure $p^*$ needed to drive substantial suspension flux through a barrier: $p^*(MFC + MDC_1 + MDC_2) < p^*(MFC + MDC_1)$. The capability of said at least three-component aggregates to move through a semi-permeable barrier is therefore increased. This finding is surprising taken that the droplets covered by a bi-component bilayer membrane already have a rather high barrier crossing ability compared to the droplets enshrined by a simple lipid bilayer: $a_a(MFC + MDC_1) >> a_a(MFC)$.

Apparently, the third aggregate component, which acts as a second membrane destabilising component, can increase or support transport-permitting aggregate adaptability beyond normal expectation: $a_a(MFC + MDC_1 + MDC_2) > a_a(MFC + MDC_1)$ and $a_a(MFC + MDC_1 + MDC_2) > a_a(MFC + MDC_2)$. This is illustrated in inset to figure 5.

The three-component bilayer membrane comprising a lipid *(MFC),* a suitable first surfactant / amphipatic drug ($MDC_1$) and a suitable second surfactant / amphipatic drug ($MDC_2$) may also require a lower driving pressure to achieve transbarrier transport: $p^*(MFC + MDC_1 + MDC_2) < p^*(MFC + MDC_2)$. Additionally or alternatively, a lower total amount of bilayer destabilising second amphipat may suffice for obtaining sufficiently adaptable aggregates, such that can cross a semipermeable barrier. The role of both membrane destabilising compounds is potentially, but not necessarily quantitatively, interchangeable (cf. figure 5).

Specifically, we found that relative concentration of said third component, which acts as membrane destabilising amphipat in the at least quaternary suspension (liquid suspending medium + $MFC + MDC_1 + MDC_2$ preferably water+lipid+drug+surfactant) containing aggregates with a high adaptability, can be kept below the necessary $MDC_2$, preferably the surfactant, concentration in a ternary suspension (liquid suspending medium + $MFC + MDC_2$ preferably water+lipid+surfactant) containing aggregates of similar adaptability: $a_a(MFC + MDC_1 + MDC_2) \approx a_a(MFC + MDC_2)$ and $[MDC_2]_{\text{three-component}} < [MDC_2]_{\text{bi-component}}$ or $a_a(MFC + MDC_1 + MDC_2) \approx a_a(MFC + MDC_1)$ and $[MDC_1]_{\text{three-component}} < [MDC_1]_{\text{bi-component}}$, values in square brackets denoting molar membrane component concentrations. Practical Examples provide several illustrations for this. In our opinion, this phenomenon reflects a synergy between the action of two bilayer components, e.g. between both membrane destabilising constituents (preferably amphipatic drug(s), surfactant (s); $MDC_1$, $MDC_2$). The dependence of adaptability curve on the magnitude of coupling parameter m, documented in inset to figure 5, supports such notion. We furthermore suggest that the interacting two membrane destabilizing components together make said three-component lipid bilayers more permeable and/or more flexible than the two-component bilayer membranes in which one of these MDC is lacking. This means that:

$a_a([MFC]+[MDC_1]+[MDC_2]) > a_a([MFC]+[MDC_1])$ and

$a_a([MFC']+[MDC_1']+[MDC_2']) > a_a([MFC']+[MDC_2'])$, similar concentration symbols meaning identical membrane component concentration. The corresponding $p^*$ values typically exhibit the inverse behaviour of $a_a$ values.

Preferably, the aggregate adaptability fulfils the condition $a_a([MFC] + [ MDC_1] + [MDC_2]) > a_a([MFC] + [MDC_1],)$ and/or $a_a([MFC] + [MDC_1] + [MDC_2] > a_a([MFC] + [MDC_2])$, wherein the combined molar concentration of both membrane destabilizing compounds $[MDC_1] + [MDC_2]$ in aggregates comprising three amphipats $(MFC + MDC_1 + MDC_2)$ is equal or less than the molar concentration of $[MDC_1]$ in the aggregates that comprise only two amphipats $(MFC + MDC_1)$ and/or is less than the molar concentration of $[MDC_2]$ in the aggregates comprising only two amphipats $(MFC + MDC_2)$, at the same molar concentration of MFC, or the aggregate adaptability fulfils the condition $a_a([MFC] +[MDC_1] + [MDC_2]) \approx a_a([MFC] + [MDC_1],)$ and/or $a_a([MFC] + [MDC_1] + [MDC_2]) \approx a_a([MFC] + [MDC_2])$, wherein the combined molar concentration $[MDC_1] + [MDC_2]$ in aggregates comprising three amphipats $(MFC + MDC_1 + MDC_2)$ is less than the molar concentration of $[MDC_1]$ in the aggregates comprising only two amphipats $(MFC + MDC_1)$ and/or is less than the molar concentration of $[MDC_2]$ in the aggregates comprising only two amphipats $(MFC + MDC_2,)$, at the same molar concentration of MFC. The corresponding $p^*$ values typically exhibit the inverse behaviour of $a_a$ values.

Therefore, a second membrane destabilizing compound can be used to form aggregates comprising three amphipates *(MFC + 2 different MDCs)* and thus achieve aggregate adaptability $a_a$ which is higher than that of an

aggregate comprising only two amphipats *(MFC + MDC).* Accordingly $MDC_1$ can be used to increase the adaptability $a_a$ of an aggregate comprising *MFC* and $MDC_2$, and $MDC_2$ can be used to increase the adaptability $a_a$ of an aggregate comprising *MFC* and $MDC_1$ by forming an aggregate comprising three amphipats *(MFC + 2 different MDCs).* Likewise the second membrane destabilizing compound can be used to decrease the amount of the first membrane destabilizing compound which would be necessary to achieve a certain adaptability $a_a$ when used alone in an aggregate comprising two amphipats. Accordingly $MDC_1$ can be used to form an aggregate comprising three amphipats *(MFC +* $MDC_1$ *and* $MDC_2$*)* to lessen the amount of $MDC_2$ necessary when used alone in an aggregate comprising *MFC* and $MDC_2$ to achieve a certain adaptability $a_a$ and/or $MDC_2$ can be used to form an aggregate comprising three amphipats *(MFC +* $MDC_1$ *and* $MDC_2$*)* to lessen the amount of $MDC_1$ necessary when used alone in an aggregate comprising *MFC* and $MDC_1$ to achieve a certain adaptability. Preferably the second membrane destabilizing compound $MDC_1$ or $MDC_2$ is used to form an aggregate comprising three amphipats *(MFC +* $MDC_1$ *+* $MDC_2$*)* whereby the total molar amount of destabilizing compound necessary to achieve a certain adaptability of an aggregate comprising two amphipats, one membrane forming compound and the respective other membrane destabilizing compound *(MFC +* $MDC_1$*)* or *(MFC +* $MDC_2$*),* is reduced, so $[MDC_1]$ + $[MDC_2]$ in amphipats comprising $[MFC]$ + $[MDC_1]$ + $[MDC_2]$ is less than $[MDC_1]$ in amphipats comprising $[MFC]$ + $[MDC_1]$ and/or $[MDC_2]$ in amphipats comprising $[MFC]$ + $[MDC_2]$.

We note that the characteristics listed in previous paragraph favourably affect the transport of said pluri-component mixed lipid vesicles through the skin. Simultaneous presence of at least two bilayer destabilising amphipats in aggregate suspension based on the lipid that forms stable bilayers is therefore beneficial for application of corresponding pharmaceutical formulations on semi-permeable barriers, such as the skin.

We thus unveil a fairly general, previously unknown phenomenon with great practical and commercial potential. An example is the transport of drugs across various biological barriers mediated by the three-component aggregates (typically vesicles comprising two membrane-destabilising amphipats) in said at least quaternary mixture. The requirement for this is the capability of complex aggregates to cross pores with a radius at least 25% smaller than the average aggregate radius before passage through the pores. The pores can also be part of the pathway through the skin, which makes said at least quaternary mixtures suitable for transdermal drug delivery. Quaternary mixtures containing at least one polar, but poorly soluble lipid (which on its own forms extended aggregates) and at least two relatively highly soluble amphipats (surfactants / drugs, which tend to destabilise the aforementioned lipid bilayer), consequently can improve drug transport into the body of warm blood creatures.

Most drugs are amphipatic. Many such molecules, especially in the ionised form, are also edge active and are thus attracted to the hydrophilic-hydrophobic boundaries. Some drugs may self-aggregate or at least tend to adsorb to an air-water or lipid-water interface; this is mainly due to hydrophobic, ionic, or H-bond interactions between drugs and lipid (aggregates), which can lead to the creation of weak drug-lipid associates. The solubility and/or amphipaty of such associates typically are greater than that of the involved lipid or drug alone. This is the reason why amphipatic drugs under certain conditions can destabilise or even permeate and solubilise lipid bilayer membranes. Such drugs then act as membrane destabilising components (*MDC*) in the sense of the present invention, but this is not necessarily the case under all conditions. Typically, sufficiently high drug solubility and sufficiently high drug partition coefficient in or binding constant to a bilayer membrane are both required for the effect. The specific, suitable value for these two parameters depends on choice of other system characteristics (pH, salt and its concentration, lipid concentration, water activity, etc.). The rule of thumb is that the highest membrane-concentration of the most water-soluble drug form normally will work best, stability considerations permitting. These conditions are also fulfilled for the drugs with a solubilising capability used for preparing mini-droplets according to EP 0 475 160.

To solve the above mentioned problems, this invention describes preparations based on a combination of at least one first, at least one second, and at least one third amphipatic component suspended in a suitable liquid medium in the form of corresponding mixed amphipat aggregates with one or a few bilayer-like, mixed amphipat coating(s), in which the combination of all three said components form extended surfaces in contact with said liquid medium that are at least 50% more extended, on the average, than the typical surface of the aggregates comprising the said at least one second and at least one third amphipatic component alone and the adaptability of extended surface aggregates comprising all three said amphipatic components to ambient stress exceeds by at least 20% or by at least twice the standard deviation of a typical measurement, whichever is smaller, the adaptability of the aggregates with extended surface that comprises the at least one first and the at least one second amphipatic component used at the corresponding concentrations or the adaptability of the extended surface comprising the at least one first and the at least one third amphipatic component at corresponding concentrations, whichever is smaller, for the application, administration or transport of an active ingredient, which can be one of the three amphipatic components, especially for biological, medical, immunological, or cosmetic purposes, into and through the pores in semi-permeable barriers or other constrictions, such as through the skin of warm blood creatures or the like.

In an alternative definition of.the described problems solution, a combination of at least one first, at least one second, and at least one third amphipatic component suspended in a suitable liquid medium in the form of mixed amphipat aggregates with one or a few bilayer-like, mixed amphipat coating(s), and thus with an extended surface, is used, in

which the said at least one first amphipatic component, on the one hand, and said at least one second and one third amphipatic components, on the other hand, have at least 2-times different solubilities in said liquid medium, and said at least one first substance has a tendency to self aggregate and is at least 10-times less soluble in said liquid medium than said at least one second and said one third substance, allowing the first to form extended surfaces; furthermore, said at least one second substance is at least 10-times more soluble in said liquid medium and, on its own, tends to form or supports the formation of surfaces that are at least 2-times less extended than the surfaces containing the at least one first substance alone and said at least one third substance is also at least 10-times more soluble in said liquid medium than the first substance and may, but needs not, form self-aggregates with aggregation numbers at least 10-times smaller than that of self-aggregates of said first substance; and said extended surfaces comprising said at least one first, at least one second and at last one third substance, in equilibrium, have at least 50% greater extended surfaces than the surfaces formed by the at least one second or one third substance alone and/or both together, and preferably the aggregates with an extended surface comprising all three said amphipatic components have adaptability to ambient stress that exceeds by at least 20% or by at least twice the standard deviation of a typical measurement, whichever is smaller, provided that the adaptability of the extended surface comprising the at least one first and the at least one second amphipatic component used at the corresponding concentrations or the adaptability of the extended surface comprising the at least one first and the at least one third amphipatic component at the corresponding concentrations, whichever is smaller, all of which serves the purpose of application, administration or transport of at least one active ingredient, which can be amongst said three substances, especially for medicinal or biological purposes, into and through barriers and constrictions, such as the skin of warm blood creatures or the like.

A favourable problem solution relies on use of said extended surfaces in the form of membrane surfaces.

Suitable combinations also fulfil the requirements as stated in previous paragraphs, simultaneously ensuring that the said at least one second substance increases the flexibility of extended surfaces comprising said at least one first, at least one second, and at least one third substance in comparison with the surfaces formed merely by an at least one first substance or else with the surfaces formed by at least one first and at least one third substance.

Further suitable combinations fulfil the requirements by ensuring that the said at least one second and one third substance together increase the permeability of extended surfaces containing the said at least one first, at least one second, and at least one third substance, in comparison with the surfaces formed merely by the at least one first substance or else with the surfaces formed by at least one first and at least one third substance.

Combinations, which also fulfil said requirements contain said at least one second substance such that increases the ability to tolerate high curvature, as assessed by relative stability of said aggregates with an extended surface comprising said one first, said one second and said one third substance against enforced higher curvature during passing through a constriction with maximum diameter at least 1.4 times smaller than the average diameter of an extended surface formed by an at least one first substance alone.

When expressed in terms of relative solubilities of different components, combinations as taught by this application preferably comprise at least one first substance and the at least one second substance that differ in solubility in the suspending medium on the average at least 10-fold. Preferably and/or alternatively, the at least one second substance and the at least one third substance differ in solubility in the suspending medium on the average at least 2-fold.

It is furthermore recommendable to use combinations, as described in previous paragraphs, characterised by the fact that the concentration of said at least one second substance used in the combination with said one first and said one third substance is below 80% of the concentration that would be needed to render the aggregates comprising only said one first and said one second substance as adaptable to ambient stress as the selected combination of all at least three substances. In preferred combinations according to the description in penultimate paragraph, the concentration of said at least one second substance amounts to at least 0.1 % of the relative stated concentration. In further preferred combination, the concentration of said at least one second substance amounts to 1 - 80% of the relative stated concentration.

It is also possible to define the combination suitable for solving the problems described in this application by selecting relative concentration of said at least one third substance, used in combination with said one first and said one second substance, to be above 0.1% of maximum possible concentration of the said at least one third substance in the system, a) as defined in terms of the solubility of said third substance in the system or in said at least three-component aggregates, or else b) as determined by the negative action of said at least one third substance on the stability of said at least three-component aggregates. This means that more than 0.1 % of saturating concentration of said third substance in the at least three component aggregates is preferably used or else , that the 0.1 % limit pertains to maximum possible concentration of said third substance which results in at least three-component aggregates to fail to fulfil the necessary aggregate stability criterion defined previously in the text.

Furthermore, it is possible to define a suitable combination by requesting relative concentration of said at least one third substance used in combination with said one first and with said one second substance to be between 1% and 99%, more favourably to be between 10% and 95%, and
most preferably to be between 25% and 90% of maximum possible concentration of said at least one third substance,

a) as defined in terms of the solubility of said third substance in the system or in said at least three-component aggregates, or else b) as determined by the detrimental effect of said at least one third substance on the stability of said at least three-component aggregates, the qualitative meaning of these definitions being described in previous paragraph.

Problem solving amphipat combination preferably contains between 0.01 weight-% and 50 weight-% dry mass as total mass of all at least three amphipatic substances, which together form highly adaptable aggregates with an extended surface. In more preferred formulations, this mass is selected to be between 0.1 weight-% and 40 weight-%, even more preferably between 0.5 weight-% and 30 weight-% and most preferably between 1 weight-% and 15 weight-%.

Amphipat combinations designed according to this application form extended surfaces with a high adaptability, containing said at least three substances, preferably with an average surface curvature corresponding to an average radius between 15 nm and 5000 nm. A particularly favoured choice are the systems with extended highly adaptable surfaces, which contain said at least three substances, with an average curvature corresponding to an average radius between 30 nm and 1000 nm, more preferably between 40 nm and 300 nm and most preferably between 50 nm and 150 nm.

Electrolyte composition and concentration affects the desirable properties of said amphipat combinations. It is therefore preferred to select these characteristics of the electrolyte in which the extended surfaces with at least one first, at least one second, and at least one third substance are suspended, and which comprises mono and/or oligovalent ions, so as to attain ionic strength between I = 0.001 and I = 1. A more preferred choice yields ionic strength between I = 0.02 and I = 0.5 which even more preferably is selected to be between I = 0.1 and I = 0.3.

Proton concentration in the selected electrolyte is an important parameter in case of ionizable systems. *p*H value of the suspending electrolyte therefore preferably should be chosen: a) in the vicinity of the logarithm of the apparent ionisation constant (*p*Ka) of said at least one second substance, if the latter is mono-ionizable, or in the vicinity of such pKa value that maximises the solubility of said at least one second substance, if the latter has several ionizable groups, or else b) in the vicinity of *p*H optimum for the most rapidly decaying or the otherwise most sensitive amongst the said at least three substances, if the said at least one second substance is not ionizable. More specifically, the *p*H value of the polar medium in which the extended surfaces comprising at least one first, at least one second, and at least one third substance are suspended should be between *p*H = *p*Ka - 3 and *p*H = *p*Ka + 3, the final *p*H selection being also affected by said stability considerations. When a narrower choice is desirable, fixing electrolyte a) between *p*H = *p*Ka - 1.5 and *p*H = *p*Ka + 2, if said at least one third substance is more soluble at high *p*H, and b) between *p*H = *p*Ka - 2 and *p*H = *p*Ka + 1.5, if said at least one third substance is more soluble at low *p*H, is recommendable, the final *p*H selection again being subject to stability considerations.

A preferred solution to outlined problems is the use of said combinations characterised in that the at least one first substance, which is less soluble in the liquid medium and/or is the surface-building substance in the system, is a lipid, preferably a phospholipid (e.g. as described herein before in the definition section), in that the at least one second substance, which is more soluble in the liquid medium and/or increases the tolerable surface curvature or adaptability of said extended surface, is a membrane destabilising amphipat and typically a surfactant, and in that said at least one third substance is either a biologically active amphipatic ingredient, which has a capability of its own to increase the tolerable surface curvature or adaptability of said extended surface, or else is a different surfactant different from the said at least second substance. The second and third substance may be interchanged.

Some preferred amphipat combinations that can conveniently be used to solved the outlined problems are favourably arranged in the form of minute fluid droplets suspended or dispersed in a liquid, and surrounded by a coating of one or several layers of the at least one first substance, which is capable of self-aggregation, and of at least one second substance and of at least one third substance, which are both amphipatic, provided that a) the former substance and the latter two substances differ in solubility in a suitable liquid suspending medium at least 10-fold, or else provided that b) the average radius of homo-aggregates of the more soluble amongst the at least one second and third substance or of hetero-aggregates of the at least one first, the at least one second and the at least one third substance is smaller than the average radius of homo-aggregates of said at least one first substance, which is the least soluble amongst the three.

A preferred and practically very useful choice for the at least one first substance, as defined herein, is a polar or a non-polar, surface-forming lipid. This lipid is most often capable of forming bilayer membranes and preferably forms bilayers on its own. When looked upon from the solubility point of view, such surface-forming lipid can be dissolved in the liquid suspending medium e.g. suspension supporting polar medium preferably in a concentration range between $10^{-12}$ M and $10^{-7}$ M.

For biological applications it is commendable to select the at least one first substance forming extended surfaces as described in this document from the group of lipids, lipoids, from a biological source, corresponding synthetic lipids and biochemical or chemical modifications, i.e. derivatives, thereof.

Particularly preferred and attractive in the sense of previous paragraph is the group comprising glycerides, glycolipids, glycerophospholipids, isoprenoidlipids, sphingolipids, steroids, sterines or sterols, sulphur-containing lipids, lipids containing at least one carbohydrate residue, or other polar fatty derivatives, which are therefore all suitable

candidates for said at least one first substance that forms said extended surfaces. More specifically, the selection is made amongst phosphatidylcholines, phosphatidyl-ethanolamines, phosphatidylglycerols, phosphatidylinositols, phosphatidic acids, phosphatidylserines, sphingomyelins, sphingophospholipids, glycosphingolipids, cerebrosides, ceramidpolyhexosides, sulphatides, sphingoplasmalogenes, or gangliosides.

Said extended surface-forming substance, which solves the problems outlined in the application, is preferably selected from the group of lipoids or lipids, with one or two, often different, fatty chains, especially with acyl-, alkanoyl-, alkyl-, alkylene-, alkenoyl-, alkoxy, or chains with omega-cyclohexyl-, cyclo-propane-, iso- or anteiso-branched segments, or any other practically useful aliphatic chain. There is some preference to use lipids with *n*-decyl, *n*-dodecyl (lauryl), *n*-tetradecyl (myristyl), *n*-hexadecyl (cetyl), *n*-octadecyl (stearyl), *n*-eicosyl (arachinyl), *n*-docosyl (behenyl) or *n*-tetracosyl (lignoceryl), 9-*cis*-dodecenyl (lauroleyl), 9-*cis*-tetradecenyl (myristoleyl), 9-*cis*-hexadecenyl (palmitoleinyl), 9-*cis*-octadecenyl (petroselinyl), 6-*trans*-octadecenyl (petroselaidinylj, 9-*cis*-octadecenyl (oleyl), 9-*trans*-octadecenyl (elaidinyl), 9-*cis*-eicosenyl (gadoleinyl), 9-*cis*-docosenyl (cetoleinyl) or 9-*cis*-tetracosoyl (nervonyl), *n*-decyloxy, *n*-dodecyloxy (lauryloxy), *n*-tetradecyloxy (myristyloxy), *n*-hexadecyloxy (cetyloxy), *n*-octadecyloxy (stearyloxy), *n*-eicosyloxy (arachinyloxy), *n*-docosoyloxy (behenyloxy) or *n*-tetracosoyloxy (lignoceryloxy), 9-*cis*-dodecenyloxy (lauroleyloxy), 9-*cis*-tetradecenyloxy (myristoleyloxy), 9-*cis*-hexadecenyloxy (palmitoleinyloxy), 6-*cis*-octadecenyloxy, (petroselinyloxy), 6-*trans*-octadecenyloxy (petroselaidinyloxy), 9-*cis*-octadecenyloxy (oleyloxy), 9-*trans*-octadecenyloxy , (elaidinyloxy), and 9-*cis*-eicosenyl (gadoleinyloxy), 9-*cis*-docosenyl (cetoleinyloxy) or 9-*cis*-tetracosoyl (nervonyloxy), *n*-decanoyloxy, *n*-dodecanoyloxy (lauroyloxy), *n*-tetradecanoyloxy (myristoyloxy), *n*-hexadecanoyloxy (palmitoyloxy) *n*-octadecanoyloxy (stearoyloxy), *n*-eicosanoyloxy (arachinoyloxy), *n*-*n*-docosoanyloxy (behenoyloxy) and *n*-tetracosanoyloxy (lignoceroyloxy), 9-*cis*-dodecenyloxy (lauroleoyloxy), 9-*cis*-tetradecenoyloxy (myristoleoyloxy), 9-*cis*-hexadecenoyloxy (palmitoleinoyloxy), 6-*cis*-octadecenoyloxy (petroselinoyloxy), 6-*trans*-octadecenoyloxy (petroselaidinoyloxy), 9-*cis*-octadecenoyloxy (oleoyloxy), 9-*trans*-octadecenoyloxyelaidinoyloxy), and 9-*cis*-eicosenoyloxy (gadoleinoyloxy), 9-*cis*-docosenoyloxy (cetoleinoyloxy) and 9-*cis*-tetracosenoyloxy (nervonoyloxy) or the corresponding sphingosine derivative chains.

A preferred suitable solution to the problems outlined herein are amphipat combinations in which said at least one second substance is a surface active substance such as a surfactant / detergent. The latter is preferably selected from the group comprising nonionic, zwitterionic, anionic and cationic surfactants. It is preferred to use a surfactant with the solubility in a liquid suspending medium such as a polar liquid, in which the extended surfaces are prepared, in the range 5 $x10^{-7}$ M to $10^{-2}$ M.

A long list of surfactants that qualify for the use in said quaternary suspensions are given herein before in the definition section.

For the solution of problems addressed by the application, charge-charge or charge-polar headgroup interactions amongst the involved amphipats may be important. If so, the following consideration can be made: if the at least one second substance is charged the at least one third substance can be is uncharged and if the at least one second substance is uncharged the at least one third substance ideally should be charged; similar preference of combinations is also possible for the said at least one first and one second or for the said at least one first and one third substance, respectively. When at least one charged amphipat is used to prepare aggregates with at least three different components, the extended aggregate surface, formed by the at least one first, one second and one third substance, at least one of which is charged, is preferably chosen to contain between 1% and 75% of the charged component. An even more favourable choice is to use combinations of at least one first, one second and one third substance, at least one of which is charged, that contain between 5% and 50% of the charged component and most preferably between 10% and 30% of the charged component.

In some cases it is preferred to use combinations according to claims of this application such that contain a phosphatidylcholine, a phosphatidylethanolamine-N-mono- or N-di-methyl, phosphatidic acid or its methyl ester, phosphatidylserine and/or phosphatidylglycerol as the surface-supporting at least one first substance and a lysophospholipid, especially a lysophosphatidic acid, lysomethylphosphatidic acid, lysophosphatidylglycerol, lysophosphatidylcholine, a partially N-methylated lysophosphatidylethanolamine, or else a monovalent salt of cholate, deoxycholate, glycocholate, glycodeoxycholate, or a sufficiently polar sterol-derivative, or a suitable salt form of laurate, myristate, palmitate, oleate, palmitoleate, elaidate or some other pharmaceutically acceptable long-chain fatty acid salt and/or a Tween-, a Myrj-, or a Brij-surfactant with said aliphatic chains, or a Triton, a long-chain fatty sulphonate, -sulphobetaine, -N-glucamide or -sorbitane (Arlacel or Span) surfactant, any of which can take the role of the at least one second or of at least one third substance, as the case may be, such second/third substance on its own forming less extended surfaces than the at least one first substance on its own.

Preferred combinations that conveniently solve the outlined problems may alternatively contain a biologically active amphipat, which can destabilise lipid membranes, as the least one second or one third substance, as the case may be, unless a surfactant different from the at least one second substance or one third substance, but otherwise selected from similar surfactant classes, is selected for the purpose.

As a useful rule of the thumb, which can be applied to select a suitable at least one third or second substance, is preferably to select the solubility of such substance in the liquid suspending medium, such as a polar liquid, to be

between $10^{-6}$ M and 1 M.

For some embodiments it is preferred to seek such molecule taking the role of at least one third or second amphipat that adsorbs to the surface of lipid bilayers but is also well miscible with or reasonably soluble in the polar liquid in which said extended lipid bilayer surfaces are formed.

It is furthermore preferred, and practically useful, to use such drug or drug form that can take the role of as the at least one third or second substance, as the case may be, especially when this role is not taken by the at least one first and/or the at least one second or third substance, respectively. If so, such ionisation or salt form of the drug is chosen that serve the purpose best. To the effect, the bulk pH, electrolyte composition and concentration value, and in case of need also co-solvents including different short chain alcohols or other short chain polar amphipats are selected appropriately.

Drugs suitable for solving the problems sketched in this work can belong to the class of substituted ammonium compounds of the formula

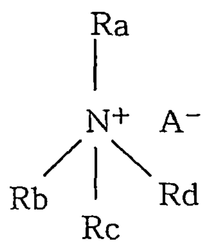

(1)

in which a) Ra represents a hydrophobic group, and Rb, Rc, and Rd, independently of one another, each represents hydrogen, Cl-C4-alkyl, 2-hydroxyethyl, allyl or cycle-C3-C6-alkyl-Cl-C3-alkyl, or two of the radicals Rb, Rc and Rd together represent C4- or C5- alkylene optionally interrupted by -HN-, -N(C1-C4-alkyl)-, -N(2-hydroxyethyl)- or by oxygen, or; b) Ra and Rb are two hydrophobic groups or together represent a hydrophobic group, and Rc and Rd, independently of one another, each represents hydrogen, C1-C4-alkyl, allyl or cyclo-C3-C6-alkyl-C1-C3-alkyl, or c) Ra, Rb and Rc together represent a hydrophobic group, and Rd represents hydrogen or C1-C4-alkyl, and $A^-$ represents the anion of a pharmaceutically acceptable acid, as a carboxylic acid salt of the formula

$$Ra\text{-}COO^- \; Y^+ \qquad (2)$$

Ra representing a hydrophobic group and $Y^+$ representing the cation of a pharmaceutically acceptable base, as an alpha-amino acid compound of the formula

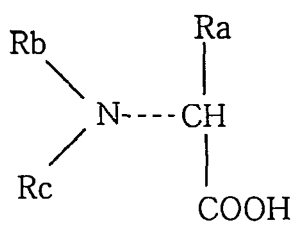

(3)

In the above formula 3, Ra represents a hydrophobic group and Rb and Rc, independently of one another, each represents hydrogen or C1-C4-alkyl,

as a phosphoric acid monoester of the formula

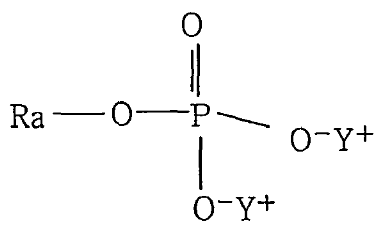

(4)

wherein Ra represents a hydrophobic group and $Y^+$ represents the cation of a pharmaceutically acceptable base, or as an acid addition salt of a compound having a hydrophobic group Ra and an imidazoline, imidasolidine or hydrasino group as hydrophilic group.

In a substituted ammonium compound of the formula 1 that can be used as a medicament, in case a) the hydrophobic group Ra is an aliphatic hydrocarbon radical that can be interrupted by an oxygen or sulphur atom, may contain the groups -CO(=O)-, -O-C(=O)-, -C(=O)-NH-, -O-C(=O)-NH- or hydroxy, and can be substituted by from 1 to 3 optionally substituted, monocyclic, aliphatic or aromatic hydrocarbon radicals, by an optionally substituted, bi- or tri-cyclic, aromatic or partially saturated hydrocarbon radical, by an optionally substituted, monocyclic, aromatic, partially saturated or saturated heterocycle or by an optionally substituted, bi- or tri-cyclic, aromatic, partially saturated or benzo-fused heterocycle.

The hydrophobic group Ra can also be an optionally substituted, monocyclic, aliphatic or aromatic hydrocarbon radical or a bicyclic, aliphatic or benzo-fused hydrocarbon radical. The hydrophilic group is, for example, a group of the formula

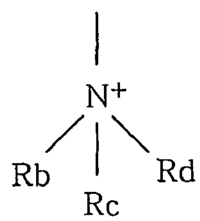

wherein Rb, Rc, and Rd, independently of one another, each represents hydrogen, C1-C4-alkyl, for example methyl, ethyl, isopropyl or *n*-propyl, or 2-hydroxyethyl, or in which two of the radicals Rb, Rc, and Rd together represent piperidino, piperazinyl, 1-methylpiperazinyl, 1-(2-hydroxyethyl)-piperazinyl or morpholino, and the other radical represents hydrogen.

In a substituted ammonium compound of the formula 1 that can be used as a medicament, in case b) Ra and Rb are two hydrophobic groups, for example two aliphatic hydrocarbon radicals, which can be substituted by one or two optionally substituted, monocyclic, aliphatic or aromatic hydrocarban radicals or by an optionally substituted, monocyclic, aromatic, partially saturated or saturated heterocycle, or Ra and Rb together represent an optionally substituted, monocyclic, aromatic, saturated, partially saturated or benzo-fused heterocycle. The hydrophilic group is a group of the formula

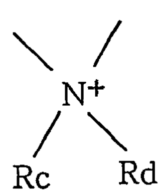

in which Rc and Rd, independently of one another each represents hydrogen or C1-C4-alkyl, preferably methyl.

In a substituted ammonium compound of the formula 1, which can be used as a medicament, in case c) Ra, Rb, and Rc form the hydrophobic group and together represent an optionally substituted, aromatic, partially saturated

or benzo-fused heterocycle. The hydrophilic group is a group of the formula

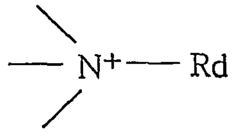

in which Rd represents hydrogen or C1-C4-alkyl, preferably methyl.

$A^-$ is the anion of a pharmaceutically acceptable acid, for example a mineral acid, for example the chloride, hydrogen sulphate or dihydrogen phosphate ion, the bromide or iodide ion, or the anion of an organic acid, for example a lower alkanecarboxylic acid, for example the acetate ion, of an unsaturated carboxylic acid, for example the fumarate or maleate ion, of a hydroxy acid, for example the lactate, tartrate or citrate ion, or of an aromatic acid, for example the salicylate ion.

In a carboxylic acid salt of the formula 2, which can be used as a medicament, the hydrophobic group Ra is an aliphatic hydrocarbon radical that can be substituted by an optionally substituted, monocyclic, aromatic hydrocarbon radical or by an optionally substituted, bi- or tri-cyclic, aromatic or partially saturated hydrocarbon radical, by an optionally substituted, monocyclic, aromatic or partially saturated heterocycle or by an optionally substituted, bi- or tri-cyclic, aromatic, partially saturated or benzo-fused heterocycle or by a steroid radical, or Ra is an optionally substituted, monocyclic, aromatic hydrocarbon radical, an optionally substituted, bi- or tri-cyclic, aromatic or partially saturated hydrocarbon radical, an optionally substituted, monocyclic, aromatic or partially saturated heterocycle or an saturated or benzo-fused heterocycle.

The cation $Y^+$ of a pharmaceutically acceptable base is, for example, an alkali metal ion, for example a lithium, sodium or potassium ion, an alkaline earth metal ion, for example a magnesium or calcium ion, or an ammonium or mono-, di- or tri-Cl-C4-alkylammonium ion, for example a trimethyl-, ethyl, diethyl- or triethyl-ammonium ion, a 2-hydroxyethyl-tri-C1-C4-alkylammonium ion, for example cholinyl, or the cation of a basic amino acid, for example lysine or arginine.

Carboxylic acid salts of the formula 2 having biological activity or carboxylic acids that can be converted into them by salt formation are, for example, salts of glucocorticoids that are esterified in the 21-position by a dicarboxylic acid, for example methylprednisolone sodium succinate, prednisolone sodium succinate; short-term narcotics of the 3,20-dioxo-5β-pregnane type that can be esterified by succinic acid, for example hydroxydione succinate sodium or 11,20-dioxo-3alpha-hydroxy-5alpha-pregnane, for example alphaxolone, or the 21-compound, for example alphadolone; salts of choleritics, for example cholic acid salts or deoxycholic acid salts; analgesics, for example salts of substituted phenylacetic acids or 2-phenylpropionic acids, for example alclofenac, ibufenac, ibuprofen, clindanac, fenclorac, ketoprofen, fenoprofen, indoprofen, fenclofenac, diclofenac, flurbiprofen, pirprofen, naproxen, benoxaprofen, carprofen or cicloprofen; analgesically active anthranilic acid derivatives, for example of the formula optionally substituted, bi- or tricyclic, aromatic,

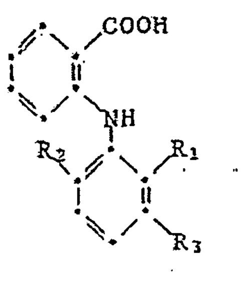

(2.1)

in which R1, R2 and R3 independently of one another, each represents hydrogen, methyl, chlorine or trifluoromethyl, for example mefenamic acid, flufenamic acid, tolfenamic acid or meclofenamic acid; analgesically active anilino-substituted nicotinic acid derivatives, for example miflumic acid, clonixin or flunixin; analgesically active heteroarylacetic acids

or 2-heteroarylpropionic acids having a 2-indol-3-yl or pyrrol-2-yl radical, for example indomethacin, oxmetacin, intrazol, acemetazin, cinmetacin, zomepirac, tolmetin, colpirac or tiaprofenic acid; analgesically active indenylacetic acids, for example sulindac; analgesically active heteroaryloxyacetic acids, for example benzadac, prostanoic acids that stimulate the smooth musculature, for example PGE2 (dinoprostone), PGF2alpha (dinoprost), 15 (S)-15-methyl-PGE2, 15 (S)-15-methyl-PGF2alpha, (carboprost), (±)15 (Xi)-15-methyl-13,14-dihydro-11-deoxy-PGE1 (deprostil), 15(S)-15-methyl-11-deoxy-PGE1 (doxaprost), 16,16-dimethyl-PGE2, 17-phenyl-18,19,20-trinor-PGF2alpha, 16-phenoxy-17,18,19,20-tetranor-PGF2alpha, for example cloprostenol or fluprostenol, or N-methylsulphonyl-15-phenoxy-17,18,19,20-tetranor-PGF2alpha (sulproston); bacteriostatics, for example salts of nalixidic acid derivatives, for example salts of nalixidic acid, cinoxacin, oxolinic acid, pironidic acid or pipenidic acid, penicillanic acid and cephalosporanic acid derivatives having antibiotic activity with 6β- or 7β-acylamino groups, which are present in fermentatively, semi-synthetically or totally synthetically obtainable 6β-acylamino-penicillanic acid or 7β-acylaminocephalosporanic acid derivatives or 7β-acylaminocephalosporanic acid derivatives modified in the 3-position, for example penicillanic acid derivatives that have become known under the names penicillin G or V, phenethicillin, propicillin, nafcillin, oxacillin, cloxacillin, dicloxacillin, flucloxacillin, cyclacillin, epicillin, mecillinam, methicillin, azlocillin, sulbenicillin, ticarcillin, mezlocillin, piperacillin, carindacillin, azidocillin or ciclazillin, or cephalosporin derivatives that have become known under the names cefaclor, cefuroxime, cefazlur, cephacetrile, cefazolin, cephalexin, cefadroxil, cephaloglycin, cefoxitin, cephaloridine, cephsulodin, cefotiam, ceftazidine, cefonicid, cefotaxime, cefmenoxime, ceftizoxime, cephalothin, cephradine, cefamandol, cephanone, cephapirin, cefroxadin, cefatrizine, cefazedone, ceftrixon or ceforanid, and other β-lactam antibiotics, for example moxalactam, clavulanic acid, nocardicine A, sulbactam, aztreonam or thienamycin; or antineoplastics having a 4-[bis-(2-chloroethyl)-amino-phenyl]-butyric acid structure, for example chlorambucil, or antineoplastics having two carboxy grows, for example methotrexate.

Compounds of the formula 3 having a biological activity are, for example, neurotransmitters in which the hydrophobic group is methyl substituted by hydroxyphenyl, for example L-tyrosine, L-dopa, alpha-methyldopa or metirosine; thyroid hormones having iodine-substituted phenyl radicals, for example levo-thyrosine, diiodotyrosine or liothyronine; or anti-neoplastics having an amino acid structure, for example melphalen.

In a compound of the formula 4 having biological activity the non-polar, hydrophobic group Ra is a glucocorticoid radical and $A^+$ is sodium, for example betamethasone disodium phosphate, dexamethasone disodium phosphate, cortisone phosphate, hydrocortisone phosphate, prednisolone disodium phosphate or paramethasone-21-disodium phosphate, Salt-type compounds having a hydrophobic group and an imidazoline, imidazolidine or hydrazino group as hydrophilic group are, for example, salts of anti-depressantly active hydrazine derivatives, for example iproniazid, nialamide, isocarboxazid, phenelzine, pheniprazine, mebanazine or fenoxypropazine; a-sympathomimetics having an imidazoline structure, for example naphazoline, tetryzolin, tramazoline, xylo-metazoline or oxyinetazoline; n-sympatholytics having an imidazoline structure, for example phentolamine or tolazoline, or centrally active antihypertensives having an imidazoline structure, for example clonidine, tolonidine or flutonidine; or vasodilatators having a hydrazino group, for example dihydralazine, hydralazine or picodralazine.

The said at least one third amphipatic substance in said combination, which acts as a drug, can be an adrenocorticostatic , a β-adrenolytic, an androgen an antiandrogen, an antiparasitic, an anabolic, an anaesthetic, an analgesic, an analeptic, an antiallergic, an antiarrhythmic, an antiarterosclerotic, an antiasthmatic, a bronchospasmolytic, an antibiotic, an antidrepressive, an antipsychotic, an antidiabetic, an antidot, an antiemetic, an antiepileptic, an antifibrinolytic, an anticonvulsive, an anticholinergic, an enzyme, a coenzyme or corresponding inhibitor, an antihistaminic, an antihypertonic, a biological inhibitor of drug activity, an antihypotonic, an anticoagulant, an antimycotic, an antimyasthenic, an agent against Morbus Parkinson or Morbus Alzheimer, an antiphlogistic, an antipyretic, an antirheumatic, an antiseptic, a respiratory analeptic or a respiratory stimulant, a broncholytic, a cardiotonic, a chemotherapeutic, a coronary dilatator, a cytostatic, a diuretic, a ganglium-blocker, a glucocorticoid, an antiflew agent, a haemostatic, a hypnotic, an immunoglobuline or its fragment, an immunologically active substance, a bioactive carbohydrate, a bioactive carbohydrate derivative, a contraceptive, an anti-migraine agent, a mineralo-corticoid, a morphine-antagonist, a muscle relaxant, a narcotic, a neurotherapeutic, a neuroleptic, a neurotransmitter or its antagonist, a small peptide, a small peptide derivative, an ophthalmic, a sympaticomimetic or a sympathicolytic, a para-sympaticomimetic or a para-sympathicolytic, a psoriasis drug, a neurodermitis drug, a mydriatic, a psychostimulant, a rhinologic, a sleep-inducing agent or its antagonist, a sedating agent, a spasmolytic, tuberculostatic, an urologic agent, a vasoconstrictor or vasodilatator, a virustatic, a wound-healing substance, or a combination of aforesaid agents.

When a drug is used as said at least one second or third component, its content is preferably chosen to be between 0.1 rel.% and 60 rel.% compared to the total mass of all three said substances forming said extended surfaces. Somewhat narrower, and more preferred, choice is to use between 0.5 rel.% and 50 rel.% and most favourably between 1 rel.% and 40 rel.% compared to the total mass of all three said substances that form said extended surfaces.

Said at least one third substance in amphipat combination, which solves the outlined problems, can be a low molecular weight immunomodulator, a bio-catalyst, a co-enzyme, a hormone, or a low molecular weight agonist or antagonist of some biologically important substance action.

Any low to intermediate weight polypeptide with membrane destabilising properties is also useful in the context of this invention, if included into said combinations in suitable form and concentration.

In one important aspect of the present invention as described above, one of the at least one second or at least on third amphipatic component is a non-steroidal anti inflammatory drug (NSAID).

In one important aspect of the present invention, the invention provides preparations, based on a suspension of extended surface aggregates in a liquid medium comprising at least one first amphipatic component; at least one second amphipatic component; at least one third amphipatic component, the first amphipatic component being a vesicle membrane forming lipid component, the second and third component being membrane destabilising components, wherein the third component is a non-steroidal anti-inflammatory drug (NSAID) such that said aggregates are capable of penetrating semi-permeable barriers with pores at least 50% smaller than the average aggregate diameter before the penetration without changing their diameter by more than 25%.

It is another aspect of the invention suspensions of extended surface aggregates in a liquid medium are provided, comprising: at least one first amphipatic component; at least one second amphipatic component; at least one third amphipatic component; the first amphipatic component being an aggregate, typically a membrane, forming lipid component; the second and third component being aggregate, typically membrane, destabilising components; wherein the third component is a NSAID, such that the extended surfaces formed by the first and second component alone or else by the first and third component alone, the second or third component, respectively, being present at a relative concentration X, have a lower propensity to overcome barriers with pores having a diameter at least 50% smaller than the average aggregate diameter, before the pore crossing, than the extended surfaces formed by the first, second and third component together, if the second and third components are present at or below the combined relative concentration of X. More specifically, this e.g. means that: a) said extended surfaces formed by the first and second component alone, the second component being present at a relative concentration X, have a lower propensity to overcome barriers with the pores at least 50% smaller than the average aggregate diameter before the pore crossing than the extended surfaces formed by the first, second and third component, if the second and third components are present at or below a combined concentration of X compared to the concentration of the first component; or else b) such extended surfaces formed by the first and third component alone, the third component being present at a relative concentration X, have a lower propensity to overcome barriers with the pores at least 50% smaller than the average aggregate diameter before the pore crossing r than extended surfaces formed by the first, second and third component, the second and third components together being present at or below a concentration of X compared to the concentration of the first component.

In yet another aspect of the invention extended surface aggregates suspended in a liquid medium are provided, comprising: at least one first amphipatic component; at least one second amphipatic component; at least one third amphipatic component; the first amphipatic component being a membrane forming lipid component; the second and third component being membrane destabilising components, such that the third component is a NSAID; and the inclusion of the second or third component to an otherwise two amphipatic-component mixture increases the suspension flux (at a given transbarrier pressure, *delta p)* through the pores at least 50% smaller than the average aggregate diameter before the penetration in comparison with the flux of the suspension containing aggregates comprising merely the first and second or the first and third components, respectively. More specifically, the inclusion of the third component increases the flux of said suspension compared with the flux of the suspension containing simpler aggregates comprising merely the first and second component or else the inclusion of the second component increases the flux of said suspension compared with the flux of the suspension containing simpler aggregates comprising merely the first and third component.

In a further aspect of this invention extended surface aggregates suspended in a liquid medium comprise: at least one first amphipatic component;, at least one second amphipatic component; at least one third amphipatic component; the first amphipatic component being a membrane forming lipid component; the second and third component being membrane destabilising components, such that the third component is a NSAID and that the addition of the second or third component to an originally two component mixture increases aggregate adaptability of the resulting extended surface aggregates with at least three components compared to the aggregates containing respective combinations of the first and the third or the first and the second components alone. More specifically, the inclusion of the third component increases the aggregate adaptability of an extended surface aggregate comprising the first and second components alone; or else, the inclusion of the second component increases the aggregate adaptability of an extended surface aggregate comprising the first and third components alone.

Yet another aspect of this invention provides extended surface aggregates suspended in a liquid medium, comprising: at least one first amphipatic component; at least one second amphipatic component; at least one third amphipatic component; the first amphipatic component being a membrane forming lipid component; the second and third component being aggregate destabilising components, such that the third component is an NSAID; and
the inclusion of the second or third component to an otherwise two amphipatic component mixture lowers the driving pressure required for aggregate penetration of pores at least 50% smaller than the average aggregate diameter before the penetration in comparison with the aggregates comprising merely the first and second or the first and third components, respectively. More specifically, the inclusion of the second component lowers the driving pressure required for aggregate

penetration of pores at least 50% smaller than the average aggregate diameter before the penetration in comparison with the aggregates comprising merely the first and third components; alternatively, the inclusion of the third component lowers the driving pressure required for aggregate penetration of pores at least 50% smaller than the average aggregate diameter before the penetration in comparison with the aggregates comprising merely the first and second components.

It is a further aspect of this invention to provide extended surface aggregates suspended in a liquid medium, comprising: at least one first amphipatic component; at least one second amphipatic component; at least one third amphipatic component; the first amphipatic component being a membrane forming lipid component; the second and third component being membrane destabilising components, such that the third component is an NSAID and the inclusion of the second or third component to an otherwise two amphipatic component mixture increases the deformability of extended surface aggregates compared with the aggregates comprising merely the first and second or the first and third component, respectively. More specifically, the inclusion of the third component increases the deformability of the extended surface aggregates compared with the aggregates comprising merely the first and second component; alternatively, the inclusion of the second component increases the deformability of the extended surface aggregate compared with the aggregates comprising merely the first and third component.

The invention teaches preparation and use of said extended surface aggregates in the form of membrane-enclosed, liquid-filled vesicles, whereby said first component is a membrane-forming lipid, and said second and third components are membrane-destabilising components.

The invention includes suspensions of extended surface aggregates in a liquid medium comprising: at least one first amphipatic component; at least one second amphipatic component; at least one third amphipatic component; the first amphipatic component being a membrane forming lipid component; the second and third component being membrane destabilising components, such that the third component is a non-steroidal anti-inflammatory drug (NSAID) and such that said extended surface aggregates can penetrate intact mammalian skin, thus increasing NSAID concentration in the skin and/or increasing the reach of NSAID distribution below the skin, in comparison with the result of the same NSAID application in a solution on the skin. In a special version of said suspensions, said extended surface aggregates are membrane-enclosed, liquid-filled vesicles, said first component is a membrane-forming lipid, and said second and third components are membrane-destabilising components.

It is also an aspect of this invention to provide said suspensions wherein the third component is an NSAID, as defined above, most preferably is ketoprofen, ibuprofen, diclofenac, indomethacin, naproxen or piroxicam. To prepare said suspensions with these or other NSAID ingredients, the first, stable membranes forming, component is selected from the group consisting of lipids, lipoids, from a biological source, corresponding synthetic lipids or lipoids, or modifications thereof. In this context it is preferable to choose amongst glycerides, glycolipids, glycerophospholipids, isoprenoidlipids, sphingolipids, steroids, sterines or sterols, sulphur-containing lipids, lipids containing at least one carbohydrate residue, or other polar fatty derivatives. Specifically, the preferred choice are the groups of phosphatidylcholines, phosphatidylethanolamines, phosphatidylglycerols, phosphatidylinositols, phosphatidic acids, phosphatidylserines, sphingomyelins, sphingophospholipids, glycosphingolipids, cerebrosides, ceramidpolyhexosides, sulphatides, sphingoplasmalogenes, or gangliosides.

To manufacture a pharmaceutical formulation, it may advisable or necessary to prepare the product in several steps, changing temperature, pH, ion strength, individual component (e.g. membrane destabiliser, formulation stabiliser or microbicide) or total lipid concentration, or suspension viscosity during the process.

Quite detailed recommendations on the preparation of said combinations is given in EP 0 475 160 and USP 6 165 500, which are herewith included by reference, using filtering material with pore diameters between 0.01 μm and 0.1 μm, more preferably with pore diameters between 0.02 μm and 0.3 μm and even more advisable filters with pore diameters between 0.05 μm and 0.15 μm to homogenise final vesicle suspension, when filtration is used for the purpose. Other methods of mechanical homogenisation or for lipid vesicle preparation known in the art are useful as well.

A list of relevant and practically useful thickening agents is given e.g. in PCT/EP98/08421, which also suggests numerous interesting microbicides and antioxidants; the corresponding sections of PCT/EP98/08421 are therefore included into the present application by reference. Practical experiments have confirmed that sulphites, such as sodium sulphite, potassium sulphite, bisulphite and metasulphite; and potentially other water soluble antioxidants, which also contain a sulphur or else a phosphorus atom (e.g. in pyrosulphate, pyrophosphate, polyphosphate), erythorbate, tartrate, glutamate, etc. or even L-tryptophan), ideally with a spectrum of activity similar to that of sulphites) offer some antioxidative protection to said formulations, final selection being subject to regulatory constraints. Any hydrophilic antioxidant should always be combined with a lipophilic antioxidant, however, such as BHT (butylated hydroxytoluene) or BHA (butylated hydroxyanisole).

It is a further aspect of this invention to teach that the first suspension component is preferably selected amongst lipids, , with one or two, not necessarily identical, fatty chains, especially with acyl-, alkanoyl, alkyl-, alkylene-, alkenoyl-, alkoxy, or chains with omega-cyclohexyl-, cyclo-propane-, iso- or anteiso-branched segments, or the corresponding chains mixtures. Useful chains include *n*-decyl, *n*-dodecyl (lauryl), *n*-tetradecyl (myristyl), *n*-hexadecyl (palmityl), *n*-octadecyl (stearyl), *n*-eicosyl (arachinyl), *n*-docosyl (behenyl) or *n*-tetracosyl (lignoceryl), 9-*cis*-dodecenyl (lauroleyl),

9-*cis*-tetradecenyl (myristoleyl), 9-*cis*-hexadecenyl (palmitoleinyl), 9-*cis*-octadecenyl (petroselinyl), 6-*trans*-octadecenyl (petroselaidinylj, 9-*cis*-octadecenyl (oleyl), 9-*trans*-octadecenyl (elaidinyl), 9-*cis*-eicosenyl (gadoleinyl), 9-*cis*-docosenyl (cetoleinyl) or 9-*cis*-tetracosoyl (nervonyl), *n*-decyloxy, *n*-dodecyloxy (lauryloxy), *n*-tetradecyloxy (myristyloxy), *n*-hexadecyloxy (cetyloxy), *n*-octadecyloxy (stearyloxy), *n*-eicosyloxy (arachinyloxy), *n*-docosoyloxy (behenyloxy) or *n*-tetracosoyloxy (lignoceryloxy), 9-*cis*-dodecenyloxy (lauroleyloxy), 9-*cis*-tetradecenyloxy (myristoleyloxy), 9-*cis*-hexadecenyloxy (palmitoleinyloxy), 6-*cis*- octadecenyloxy, (petroselinyloxy), 6-*trans*-octadecenyloxy (petroselaidinyloxy), 9-*cis*-octadecenyloxy (oleyloxy), 9-*trans*-octadecenyloxy (elaidinyloxy), and 9-*cis*-eicosenyl (gadoleinyloxy), 9-*cis*-docosenyl (cetoleinyloxy) or 9-*cis*-tetracosoyl (nervonyloxy), *n*-decanoyloxy, *n*-dodecanoyloxy (lauroyloxy), *n*-tetradecanoyloxy (myristoyloxy), *n*-hexadecanoyloxy (palmitoyloxy) *n*-octadecanoyloxy (stearoyloxy), *n*-eicosanoyloxy (arachinoyloxy), *n*-*n*-docosoanyloxy (behenoyloxy) and *n*-tetracosanoyloxy (lignoceroyloxy), 9-*cis*-dodecenyloxy (lauroleoyloxy), 9-*cis*-tetradecenoyloxy (myristoleoyloxy), 9-*cis*-hexadecenoyloxy (palmitoleinoyloxy), 6-*cis*-octadecenoyloxy (petroselinoyloxy), 6-*trans*-octadecenoyloxy (petroselaidinoyloxy), 9-*cis*-octadecenoyloxy (oleoyloxy) ,9-*trans*-octadecenoyloxyelaidinoyloxy), and 9-*cis*-eicosenoyloxy (gadoleinoyloxy), 9-*cis*-docosenoyloxy (cetoleinoyloxy) and 9-*cis*-tetracosenoyloxy (nervonoyloxy) or the corresponding sphingosine derivative chains, or corresponding two double bonds combinations, especially in the sequence 6,9-*cis,* 9,12-*cis* or, in case, 12,15-*cis* or else the related three double bonds combinations, especially in the sequence, 6,9,12-*cis*, or 9,12,15-*cis* are preferable. A preferred choice in case of phosphatidylcholines of biological, and preferably plant, origin, is to use the lipids extracted from soy (bean), coconut, olive, safflower or sunflower, linseed, evening primrose, primrose, or castor oil, and the like, other biological sources of general availability, such as eggs, also being an option.

According to the invention the second suspension component, which tends to destabilise lipid membranes, is preferably a surfactant. The selected surfactant can belong to the group of nonionic, zwitterionic, anionic and cationic surfactants. Preferentially, any such surfactant is chose to have solubility in the liquid medium ranging from about 5 $x10^{-7}$ M to about $10^{-2}$ M. An alternative definition of surfactants useful for the use in said suspensions of extended surface aggregates relates to hydrophilicity-lipophilicity ratio (HLB), which should be between 10 and 20, preferably between 12 and 18 and most preferred between 13 and 17. A good choice of non-ionic surfactants according to this invention are polyethyleneglycol-sorbitan-long fatty chain esters, from polyethyleneglycol-long fatty chain esters or -ethers and from polyhydroxyethylen-long fatty chain esters or -ethers; preferably, the number of ethyleneglycol or hydroxyethylen units per such surfactant molecule is selected to be in the range 6 to 30, more conveniently to be between 8 and 25 and most and typically to be between 12 to 20. Alternatively, non-ionic phospholipids with water solubility similar to that of said non-ionic surfactants, can be used to the same effect. Examples include lyso-phospholipids, certain phosphatidylglycerols, phospholipids with one long and one short (C1-C6) chain, etc. In order to ensure sufficient fluidity of resulting complex extended surface aggregates, the hydrophobic chain attached to such polar groups is preferentially chosen to be sufficiently short or to be unsaturated; polyethylenglycol-sorbitan-monolaurate and polyethylenglycol-sorbitan-monooleate, polyethyleneglycol-monolaurate and polyethyleneglycol-monooleate or polyethyleneglycol-monolaurate-ether and
polyethyleneglycol-monooleate-ether are good choices in this respect. More specifically, it is preferable in the context of this invention, to use a surfactant which is polyethyleneglycol-sorbitan-monooleate or monolaurate (e.g. Tween 80 or Tween 20) or else is polyethyleneglycol-oleate or laurate (i.e. POE-oleate or POE-laurate) or else is polyethyleneglycol-oleyl-ether or lauryl-ether, with 6 to 30, more preferably 8 to 15 and most preferred 12 to 20 ethyleneglycol (i.e. oxyethylene or OE) units per surfactant molecule.

It is another aspect of this invention to combine, in said suspension, a phosphatidylcholine, as the first component, and ketoprofen, diclofenac, ibuprofen, indomethacin, naproxen, or piroxicam, as the third, NSAID, component. A preferred choice is the combination of soy phosphatidylcholine, as the first, and of ketoprofen, diclofenac, ibuprofen, indomethacin, naproxen or piroxicam as the third component.

In a preferred embodiment of the invention, the second component is a non-ionic surfactant, such as a polyethyleneglycol-sorbitan-long fatty chain ester, a polyethyleneglycol-long fatty chain ester or a polyethyleneglycol-long fatty chain ether or else the corresponding surfactant with a polyhydroxyethylene polar group. A preferred choice is the use of polyethyleneglycol-sorbitan-monooleate or -laurate, of polyethyleneglycol-monooleate or -laurate, or else of polyethyleneglycol-oleyl-ether or -lauryl-ether as the second component. In the resulting suspension, the second component is preferablely chosen to carry a polyethyleneglycol (PEG or POE) polar head with 6 to 30, more preferably 8 to 15 and most preferred 12 to 20 ethyleneglycol (i.e. oxyethylene or OE) units per surfactant molecule. Alternatively, non-ionic phospholipids, with water solubility similar to that of said non-ionic surfactants, can be used for similar purpose. Moreover, the hydrophobic chains are always chosen to be in a fluid state or at least to be compatible with such state of a carrier aggregate.

In another preferred embodiment of this invention is to provide said suspensions such that contain aggregates with an average diameter before the aggregates penetrate the pores at least 40% larger than the average pore diameter in the barrier of interest.

In a preferred embodiment of the invention, extended surface aggregates are proposed to have an average

aggregate diameter that is at least 50% larger before pore penetration than the average pore diameter. Preferably, the average aggregate diameter before the aggregates penetrate the pores is at least 70%, even more preferably is at least 100% and most preferably is at least 150% larger than the average pore diameter.

Another aspect of the invention is to provide suspensions in which the first component and the second component differ in solubility in the liquid medium at least 10-fold, on the average. The preferred difference in solubility between the second and third component is, on the average, at least 2-fold.

In a further preferred embodiment of the invention said suspension comprises a total dry mass of the at least three amphipatic components between 0.01 weight-% and 50 weight-%. A more preferred choice is to keep this total dry mass between 0.1 weight-% and 40 weight-%, better to keep even it between 0.5 weight-% and 30 weight-% and best to select the total dry mass of the three amphipatic components between 1 weight-% and 15 weight-%, at the time of formulation preparation and/or application.

Yet another aspect of the invention is to provide suspensions of extended surface aggregates, formed by the three components, with an average curvature corresponding to the average aggregate diameter between 15 nm and 5000 nm, preferably between 30 nm and 1000 nm, more preferred between 40 nm and 300 nm and most preferred between 50 nm and 150 nm.

A different aspect of the invention is to advocate using suspensions of extended surface aggregates that contain a lower aliphatic alcohol with a membrane partition coefficient and polarity such that the alcohol, as the at least one further second component, takes the role of a membrane destabilising component. Alcohols that potential qualify for such use include mono-alcohols, diols, or to some extent polyols, of low carbon number (C1-C6), and ethers thereof; preferred examples are ethanol, isopropanol, 1,2-propanediol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products. The preferred choice are simple alcohols, short chain diols or a short chain triols, preferably with the OH-residues grouped together, corresponding methyl-, ethyl-, or butyl-derivatives also being a possibility. This includes especially n-propanol, iso-propanol, or 2-propanol, n-butanol, or 2-butanol, 1,2-propanediol, 1,2-butanediol; if ethanol is used, the total alcohol and lipid concentration are selected such that practically useful ethanol association with a pore penetrating aggregate is ensured. Specifically, if used individually to increase extended surface aggregate adaptability, ethanol, n-propanol, 2-propanol, butanol, and benzyl alcohol are preferably used at concentrations up to 15 w-%, 10 w-%, 8 w-%, 4 w-% and 2 w-%, respectively, in case of an initially 10 w-% total lipid suspension. The published water-membrane partition coefficients for other alcohols can be used together with these recommendations to select preferred concentration of other alcohols, or of alcohol combinations.

An important further aspect of the invention is to propose pharmaceutical preparations comprising suspensions according to the invention. A very convenient and preferred form of aggregates in such suspension is that of liquid-filled vesicles in an aqueous medium, the vesicles being enclosed by membranes formed from at least one lipid component and comprising at least two membrane destabilising components one of which is an NSAID, whereby the extended surfaces formed by the first and second component alone or else by the first and third component alone, the second or third component, respectively, being present at a relative concentration X, have a lower propensity to overcome barriers with pores at least 50% smaller than the average aggregate diameter before the pore crossing than the extended surfaces formed by the first, second and third component together, if the second and third components are present at or below the combined relative concentration of X.

It is also an important aspect of the invention, to teach pharmaceutical preparations comprising a suspension of liquid-filled vesicles in an aqueous medium, the vesicles being enclosed by membranes formed from at least one lipid component and comprising at least three membrane destabilising components, whereby the membrane destabilising components comprise at least one surfactant, at least one lower aliphatic alcohol and at least one non-steroidal anti-inflammatory drug; such that the membrane destabilising components increase the adaptability of the resulting extended surface aggregates with at least three components compared to the aggregates containing respective combinations of the first and the third or the first and the second components alone.

It is a further aspect of the invention to provide pharmaceutical preparations comprising a suspension of liquid-filled vesicles in an aqueous medium, the vesicles being enclosed by membranes formed from at least one lipid component and comprising at least three membrane destabilising components, whereby the membrane destabilising components comprise at least one surfactant, at least one lower aliphatic alcohol and at least one non-steroidal anti-inflammatory drug, such that the membrane destabilising components increase the deformability of the vesicles and the vesicles are capable of penetrating barriers with pores at least 50% smaller than the average aggregate diameter before the penetration without changing their diameter by more than 25%.

It is a different aspect of the invention to provide pharmaceutical preparations comprising a suspension of liquid-filled vesicles in an aqueous medium, the vesicle being enclosed by membranes formed from at least one lipid component and comprising at least three membrane destabilising components, whereby the membrane destabilising components comprise a surfactant, a lower aliphatic alcohol and a non-steroidal anti-inflammatory drug, whereby the membrane destabilising components increase the vesicle ability to penetrate mammalian skin and thus increase the

reach of NSAID distribution in the skin, and beyond, in comparison with the result of an NSAID application in a solution on the skin.

A preferred embodiment of the invention provides vesicle containing pharmaceutical preparations in which a phosphatidylcholine takes the role of first component and an NSAID, such as ketoprofen, diclofenac, ibuprofen indomethacin, naproxen, or piroxicam is the third component.

In another preferred embodiment of the invention pharmaceutical preparations contain a nonionic surfactant, preferably a polyethyleneglycol-sorbitan-long fatty chain ester, a polyethyleneglycol-long fatty chain ester or a polyethyleneglycol-long fatty chain ether, the polyethyleneglycol chain being potentially replaced by a polyhydroxyethylene polar group. Specifically preferred are polyethyleneglycol-sorbitan-monooleate (e.g. Tween 80) or - laurate (e.g. Tween 20), or else polyoxyethylene-monooleate (e.g. Cithrol 10MO / Chemax E-1000) or -laurate (e.g. Cithrol 10ML) or else polyoxyethylene-oleyl-ether (e.g. Brij 98) or -lauryl-ether (e.g. Brij 35). Alternatively, non-ionic phospholipids, with water solubility similar to that of said non-ionic surfactants, are used as a preferred nonionic surfactant.

In a related embodiment of the invention, said pharmaceutical preparations contain an alcohol, which preferably is selected from *n*-propanol, iso-propanol, 2-propanol, *n*-butanol or 2-butanol, 1,2-propanediol, or 1,2-butanediol, a methyl- or ethyl-derivative thereof, or ethanol. When ethanol is used, the total alcohol and lipid concentration is chosen to ensure a practically useful ethanol association with a pore penetrating aggregate.

It is also an aspect of the invention to provide such pharmaceutical preparations that are characterised by the bulk pH value above the logarithm of the apparent dissociation constant (pKa) of the NSAID in a solution and in the extended surface aggregates, the latter pKa being higher than the former. Preferably, the bulk *p*H value is selected to be between 0.2 *p*H and 2.2 *p*H units above pKa of the NSAID in an extended surface aggregate, more preferably is between 0.5 *p*H and 1.9 *p*H units above this pKa and ideally is between 0.8 *p*H and 1.6 *p*H units above such pKa. Specifically, for the particularly interesting NSAIDs, ketoprofen or ibuprofen, the selected bulk *p*H is between 6.4 and 8.3, more preferably is between 6.7 and 8 and most preferably is between 7 and 7.7; for diclofenac the preferred bulk *p*H is between 6.2 and 8.1, more preferably is between 6.5 and 7.8 and most preferably is between 6.8 and 7.5; for naproxen the corresponding preferred *p*H value is between 6.3 and 8.2, more preferably is between 6.6 and 7.9 and most preferably is between 6.9 and 7.6; for piroxicam the choice of suspension bulk *p*H should be between 7.2 and 9, more preferably between 7.3 and 8.5 and most preferably between 7.4 and 8.2.

It is another aspect of the invention to select the bulk ionic strength of said pharmaceutical preparation to be between 0.005 and 0.3, even better between 0.01 and 0.2 and best between 0.05 and 0.15.

In preferred embodiment of the invention the said pharmaceutical formulation has viscosity between 50 mPa s and 30.000 mPa s. Preferably, the formulation viscosity is chosen to be between 100 mPa s and 10.000 mPa s, even better between 200 mPa s and 5000 mPa s, and most preferred between 400 mPa s and 2000 mPa s. To achieve such viscosity, at least one thickening agent may be added to said pharmaceutical formulation, precise choice and concentration of such agent depending on the ambient temperature, *p*H, ion strength, presence of other viscosity modifiers (such as glycerol), etc..

Thickening agents that are useful in the context of present invention are typically pharmaceutically acceptable hydrophilic polymers, including partially etherified cellulose derivatives, such as carboxymethyl-, hydroxyethyl-, hydroxypropyl-, hydroxypropylmethyl- or methyl-cellulose; completely synthetic hydrophilic polymers, including polyacrylates, polymethacrylates, poly(hydroxyethyl)-, poly(hydroxypropyl)-, poly(hydroxypropylmethyl)methacrylate, polyacrylonitriles, methallyl-sulphonates, polyethylenes, polyoxiethylenes, polyethylene glycols, polyethylene glycol-lactides, polyethylene glycol-diacrylates, polyvinylpyrrolidones, polyvinyl alcohols, poly(propylmethacrylamide), poly(propylene fumarate-co-ethylene glycol), poloxamers, polyaspartamides, (hydrazine cross-linked) hyaluronic acids, silicone; natural gums, such as alginates, carrageenan, guar-gum, gelatine, tragacanth, (amidated) pectin, xanthan, chitosan collagen, agarose; mixtures and further derivatives or copolymers thereof and/or other biologically acceptable polymers.

Most of such thickening agents in said pharmaceutical preparation are employed in weight concentration between 0.1 w-% and 10 w-%.

For the use of pharmaceutical formulations of the invention, the following hydrophilic polymer are preferred, amongst others: partially etherified cellulose derivatives, such as carboxymethyl -, hydroxyethyl-, hydroxypropylcellulose or amongst completely synthetic hydrophilic polymer s from the class of polyacrylates, such as polymethacrylates, poly (hydroxyethyl)-, poly(hydroxypropyl)-, poly(hydroxypropylmethyl)methacrylate, especially Carbopols.

Most preferably, such formulation thickeners are chosen from the group of polysaccharides and derivatives thereof that are commonly used on the skin, including e.g. hyaluronic acid or hydroxypropylmethylcellulose; particularly preferablely choices from the group of polyacrylates include the group of Carbopols, such as Carbopol grades 974, 980, 981, 1 382, 2 984, 5 984, in each case individually or in combination. In case of Carbopols (e.g. Carbopol 974), used to thicken the suspension-based multicomponent formulations for improving NSAID delivery through permeability barriers and the skin, the polymer concentration preferably is selected to be between 0.3 w-% and 5 w-%, better between 0.5 w-% and 3w-% and best between 0.75 w-% and 1.75 w-%. Manufacturer's recommendations for obtaining certain viscosity can be combined with these guiding concentrations to use other polymers or polymer combinations in a formulation for similar purpose.

It is another preferred embodiment of the invention to use of at least one antioxidant in said pharmaceutical formulations, which is typically selected amongst synthetic phenolic compounds and their derivatives, the quinone-group containing substances, aromatic amines, ethylenediamine derivatives, various phenolic acids, tocopherols and their derivatives, including the corresponding amide and thiocarboxamide analogues; ascorbic acid and its salts; primaquine, quinacrine, chloroquine, hydroxychloroquine, azathioprine, phenobarbital, acetaminephen); aminosalicylic acids and derivatives; methotrexate, probucol, sulphur or phosphate atom containing anti-oxidants, thiourea; chellating agents, miscellaneous endogenous defence systems, and enzymatic antioxidants, etc.. Preferred are combinations of at least two antioxidants, one being lipophilic, such as butylated hydroxyanisol (BHA), butylated hydroxytoluene (BHT), di-tert-butylphenol, or tertiary butylhydroquinone (TBHQ), and the other being hydrophilic, such as a chellating agent, especially EDTA, GDTA, or desferral, and/or is a sulphite, such as s sodium or potassium metabisulphite, a pyrosulphate, pyrophosphate or polyphosphate. The butylated hydroxyanisol (BHA) or hydroxytoluene (BHT) are typically used at concentrations between 0.001 w-% and 2 w-%, more preferably between 0.0025 w-% and 0.2 w-%, and most preferably is between 0.005 w-% and 0.02 w-%; EDTA or GDTA concentration is typically chosen between 0.001 w-% and 5 w-%, preferably between 0.005 w-% and 0.5 w-%,more preferably between 0.01 w-% and 0.2 w-% and most preferably between 0.05 and 0.975 w-%; a sulphite, such as sodium or potassium metabisulphite is used preferably used in concentration range between 0.001 w-% and 5 w-%, more preferably between 0.005 w-% and 0.5 w-%, and most preferably between 0.01 w-% and 0.15 w-%.

In preferred embodiments of the invention pharmaceutical preparations contain at least one microbicide in concentration range between 0.1 w-% and 5 w-%, as is required for proper action and as is acceptable by a regulatory body.

Likewise, it is preferred according to of the invention that molar concentration ratio of the phospholipid component, which forms stable lipid membranes, and of the third, surfactant-like component, which destabilises such membranes, in said pharmaceutical preparations should be between 40/1 and 4/1. More preferably such a molar ratio is between 30/1 and 7.5/1, the ratios between 20/1 and 10/1 being most preferred.

It is a further aspect of the invention to suggest composing a kit, comprising, in a tube or otherwise packaged form, at least one dose of the pharmaceutical preparation containing an NSAID associated with the aggregates suitable for overcoming biological barriers such as the skin.

It is another aspect of the invention to propose a method for treating peripheral pain and/or inflammation by applying said pharmaceutical preparation on the skin of a warm blooded mammal.

A further aspect of the invention is to select different formulation doses per area to control the depth of NSAID delivery, if desirable using a non-occlusive patch for the purpose.

In a special embodiment of the invention at least one dose of an NSAID in said pharmaceutical formulation is applied, and the application is repeated several, e.g. up to five times per day, if necessary, the preferred choice being two applications per day.

In presently preferred pharmaceutical preparations the first, i.e. phospholipid, component and the third, i. e. NSAID, components are present in the molar range between 10/1 and 1/1. A more preferred range molar range of these two components is between 5/1 and 2/1, or even between 4/1 and 2.5/1 and the most preferred composition have phospholipid/NSAID molar ratio near 3/1.

Last but not least, it is envisaged by the invention to use transdermal carriers, typically in the form of barrier penetrating extended surface aggregates, to deliver NSAIDs below the skin and into the underlying muscle tissue or the adjacent joints.

A list of potential ingredients that can be used for preparing pharmaceutical formulations according to the present invention is given in Cosmetic Ingredient Review (CIR Compendium), which is regularly published in Washington, DC, and in appropriate Food and Drug Administration or other national regulatory agency publications, including the list of GRAS (Generally Recognised As Safe) compounds.

It is furthermore an explicit aim of the document, to teach the use of amphipat combinations, as described herein, as drug carriers, drug depots, or for other kind of medicinal or biological application. For the purpose the required extended surfaces are advantageously provided in the form of membranes formed by the at least one first substance, the at least one second and the at least one third substance, which together surround miniature droplets. The substance with a biological activity, such as a drug, is then mainly associated with said droplets at the surface or else is mainly incorporated into the droplet to be carried by the droplet to the place where the biologically active substance is supposed to act.

Relatively detailed recommendations for preparing compositions, as advocated in this application, are given in EP 0 475 160 and US 6 165 500, which are herewith included by reference. When filtration is use to prepare aggregate suspensions, filter material with pore diameters between 0.01 μm and 0.1 μm, more preferably with pore diameters between 0.02 μm and 0.3 μm and even more advisable with pore diameters between 0.05 μm and 0.15 μm are used for homogenisation.

The present patent application moreover teaches suitable methods for preparing combinations such that solve

the outlined problems by providing suitable formulations of biologically, cosmetically and/or pharmaceutically active agents, comprising the steps of: a) selecting the at least one first and the at least one second substance which together form extended surfaces, when in contact with said liquid suspending medium, such that said extended surfaces formed by the at least one first and the at least one second substance are more adaptable than the at least one first substance alone and the surfaces formed by the at least one second substance alone are not extended; alternatively; b) selecting the at least one first and the at least one third substance which together form extended surfaces, when in contact with said medium, such that said extended surfaces formed by the at least one first and the at least one third substance are more adaptable than the at least one first substance alone and the surfaces formed by the at least one third substance alone are not extended, if this substance self-aggregates; and c) generating said combination of at least one first, at least one second, and at least one third substance, such that the surface of resulting at least three component combination is even more adaptable than the surface prepared from at least one first and one second substance alone or of the surfaces formed by the at least one first and one third substance alone, bringing the combination of at least two or all three said substances into suspension by means of controlled mechanical fragmentation, preferably in the presence of or before being mixed with the at least one third substance, such that said third substance is incorporated at least partly in said extended surface formed by controlled mechanical fragmentation to obtain final preparation.

It is particularly preferred to use filtration, pressure change or mechanical homogenisation, or else shaking, stirring, or mixing as said means of controlled mechanical fragmentation. The desirable intermediary or final characteristics of the liquid medium used to prepare aggregate suspension are defined in previous paragraphs of this section.

The present patent application furthermore teaches methods based on use of said at least quaternary mixtures containing at least one active agent selected from the group comprising anti-diabetic agents, growth factors, immunomodulators, enzymes, recognition molecules, adrenocorticostatics, adrenolitics, androgens, antiandrogens, antiparasitics, anabolics, anaesthetics, analgesics, analeptics, antiallergics, antiarrhythmics, antiarterosclerotics, antiasthmatics, bronchospasmolytics, antibiotics, antidrepressiva, antipsychotics, antidots, antiemetics, antiepileptics, antifibrinolytics, anticonvulsiva, anticholinergics, enzyme, coenzymes or corresponding inhibitors, antihistaminics, antihypertonics, biological inhibitors of drug activity, antihypotonics, anticoagulants, antimycotics, antimyasthenics, agents against Morbus Parkinson or Morbus Alzheimer, antiphlogistics, antipyretics, antirheumatics, antiseptics, respiratory analeptics or respiratory stimulants, broncholytics, cardiotonics, chemotherapeutics, coronary dilatators, cytostatics, diuretics, ganglium-blockers, glucocorticoids, antiflew agents, haemostatics, hypnotics, immunologically active substances, contraceptives, anti-migraine agents, mineralo-corticoids, morphine-antagonists, muscle relaxants, narcotics, neurotherapeutics, neuroleptics, neurotransmitters or their antagonists, peptides, peptide derivatives, ophthalmics, sympaticomimetics or sympathicolytics, para-sympaticomimetics or para-sympathicolytics, anti-psoriasis drugs, neurodermitis drugs, mydriatics, psychostimulants, rhinologics, sleep-inducing agents or their antagonists, sedating agents, spasmolytics, tuberculostatics, urologics, vasoconstrictors or vasodilatators, virustatics, wound-healing substances, or a combination of aforesaid agents.

Aforesaid method can rely on either using the recommended at least three amphiphilic substances as such, or else dissolved in a physiologically compatible polar fluid, comprising water or water-miscible fluids, or in solvation-mediating agent, together with a polar solution. Use of co-solvents is also possible.

A preferred, particularly practical method for preparing said aggregate formulations contains at least one surfactant or surfactant-like amphipat, which destabilises bilayer membrane, and at least one more membrane destabilising, biologically active ingredient or an additional surfactant in said polar solution.

In the case of need, the method can include the formation of said surfaces induced by addition of one or more formulation or aggregate components into a fluid phase, e.g. by using evaporation from a reverse phase, injection or dialysis, or even by additional mechanical stress.

Furthermore, it may be-preferred to use preparation method in which the formation of said surfaces is induced by filtration, the filtering material having pores between 0.01 μm and 0.8 μm wide. The choice of most convenient or favourable pore diameter depends on the desired final aggregate dimensions and also on the anticipated or achieved suspension flux through a filter. Higher flux rates produce stronger shear and relatively smaller final vesicle diameter, suspension viscosity also being important.

When filtration is used to manufacture aggregate suspensions, it may be convenient to use several filters sequentially or in parallel. In the former case, pore diameters in different filters can vary in diameter.

An preferred advantageous method for preparing suspensions according to the present invention is such that ensures said agents and carriers to associate, at least partly, after the formation of said extended surfaces.

For better convenience, said extended surfaces, with which agent molecules are made to associate, may be prepared just before the application of the formulation. If desired, and possible, this can be done from a suitable concentrate or a lyophylisate.

It is practically convenient to use a single container comprising the selected pharmaceutical composition based on the combination of substances as described in previous text. It is also convenient to make said container a part of a package.

The present patent application moreover teaches a method for generating a therapeutic effect on a warm blood creature by applying a suitably selected pharmaceutical composition onto or into a leaving creature's body, whereby the selection of a suitable combination of substances is made according to the claims of this document.

Special application of the method described herein is to choose such administration volume that ensures control over the applied medicament dose and the outcome of therapeutic application.

It may be preferred, and practically valuable, to load a suspension of drug-free aggregates with the drug, via association, during the day prior to an administration, preferably within 360 min, more preferably within 60 min and most preferably within 30 min time window before the administration of resulting formulation in or on the body.

The method of treatment done according to the present invention typically involves administration of at least one dose of the pharmaceutical composition with therapeutic activity on or in a warm blood animal.

Last but not least, the present invention teaches a method for finding suitable compositions, as described herein. This method comprising the steps of: a) determining the flux of aggregates in a suspension associated with a drug through pores in a well-defined barrier, or various barriers, as a function of the driving force or the driving pressure, which acts across the barrier; b) describing the data within the framework of a suitable model such that fits the characteristic flux vs. pressure or penetrability vs. pressure curve; c) to deduce the characteristic system parameters, such as $p^*$ and $P_{max}$, in particular; d) employing said parameters to optimise or characterise the formulation for application. Eq. (*) is recommended as, and is claimed herein to be, particularly suitable for describing and analysing such data.

**Practical Examples**

The following examples illustrate the invention without setting or delineating its limits. All temperatures are in degree Celsius. Carrier diameters are in nanometers, pressures in Pascal (Pa) and other units correspond to standard SI system. Ratios and percentages are given in moles, unless otherwise stated.

All measurements were done at room temperature, except when specified otherwise. For aggregate adaptability / barrier transport resistance measurements the test temperature was constant to within plus/minus 2 degrees. For aggregate size measurements the temperature accuracy was plus/minus 0.1 degree. The pH value of the bulk suspension was determined with a commercial (gel) electrode. All substances were used as received and were of p.a. quality, unless stated otherwise. Molar masses were taken to be identical to the published reference data.

Suspension viscosity was measured with a rotation viscosimeter, typically at room temperature and using 20 RPM, which corresponded to 150 1/s.

Determination of Barrier Transport Resistance and aggregate Adaptability. Barrier resistance to the transport of test vesicle suspension in earlier patent applications by the same applicant was called "permeation" resistance. In this document, more precise term "penetration" resistance is used to stress the fact that vesicles do not diffuse (=permeate) through but rather penetrate barriers.

In first approximation one relies on simple experimental method (SEM) and takes barrier transport resistance (in arbitrary units) to be proportional to the pressure (in arbitrary units) needed to drive a suspension of relatively large vesicles through a 0.2 micrometer filter with good efficacy. (In our experience, a porous filter acts as a permeability barrier when the average pore diameter is at least 40% to 50%, for the vesicles bigger and smaller than 150 nm, respectively, and more preferably is at least 100% smaller than the average vesicle diameter in the tested suspension.) Barrier transport resistance is then given in relative units of 1 to 10 elsewhere (in EP 0 475 160 and USP 6 165 500) and in this document whenever reference is made to a 0.2 micrometer filter. Barrier penetrability, which in older publications is called permeability, is identified with inverse barrier resistance value. Aggregate adaptability is a direct function of the former value, as is explained e.g. in Critical Reviews in Therapeutic Drug Carrier Systems 13:257-388 (1996) or in Adv. Drug Delivery Rev. 18:349-378 (1996).

Use of relative penetrability and barrier resistance values is also convenient. These values are given by the ratio of the penetrability/permeability or of the corresponding barrier resistance values measured with a given suspension and its supporting medium (e.g. water), e.g.: (relative) Penetrability η $P_{rel} = P_{suspension}/P_{medium}$. Similar use of the trans-barrier flux data, measured with constant driving pressure, provides more direct but still relative measure of barrier penetrability/permeability for different formulations. Theoretical explanation for such comparisons and calculations is given in Critical Reviews in Therapeutic Drug Carrier Systems 13:257-388 (1996).

To get an absolute Barrier Transport Resistance or Penetrability data, and to interpret these values in molecular terms, an improved analytical method is needed, which is described in brief in Definitions sections (see especially e.q. (*)). To get absolute penetrability - and thus aggregate adaptability - data, transbarrier flux is first measured serially. (This can be done as is described in this document or in Biochim. Biophys. Acta 1368: 201-215 (1998).) Barrier resistance / penetrability value for the test suspension is then calculated from the flux *vs*. pressure data using e.q. (*), following the description given in previous sections. From calculated resistance / penetrability value, a convenient parameter that describes the adaptability of mixed aggregates is deduced, e.g. by assuming: $a_a = 1/p^*$.

Aggregate adaptability is thus identified with the inverse pressure difference needed to attain a predefined,

practically relevant fraction of maximum achievable flux-pressure ratio; using 50-60% maximum penetrability criterion gives reasonable results. Specifically, all $p^*$ values given in this document correspond to 57% of $P_{max}$-value. If the maximum penetrability for a given suspension-barrier combination cannot be measured, the penetrability of a barrier to the medium in which the tested aggregates are suspended is used as surrogate: $P_{max} = f\ x$ Suspending medium flux / Driving pressure. Proportionality factor is then typically taken to be up to 3-times (and more often up to 2-times) smaller than 57%, to allow for trivial friction effects.

Exemplary results are given in figures 4 and 5 ?. The latter figure also graphically illustrates the meaning of parameters "$p^*$" (in pressure units, and proportional to the barrier transport resistance) and "Maximum penetrability" (= $P_{max}$; in flux per pressure units, and indicative of barrier porosity).)

Aggregate size (diameter) determination. The average aggregate (most often vesicle) diameter was measured with the dynamic light scattering (for a few samples with a Malvern Zeta-Sizer instrument and for the majority of samples with the instrument with an ALV 5000 correlator. Cumulant analysis method and an implementation of software package "Contin" were used for analysing the correlation curves obtained with Zeta-Sizer. To analyse the ALV measurements the software delivered by the manufacturer (cumulant analysis or "Contin") was employed.

**Examples 1-120:**

| Composition: | |
|---|---|
| 37.74 - 84.5 mg | Phosphatidylcholine from soy-bean (SPC, ~85% purity, *MFC*) introduced as an ethanolic solution SPC/EtOH = 1/1 VN and containing approx. 10% charged phospholipid (presumably anionic phosphatidylglycerol) |
| 187-34.9 mg | Polysorbate (Tween 80, pharmaceutical grade; $MDC_1$) |
| 5.6 - 20 rel. mol-% | Sodium dodecylsulphate (SDS, p.a.; $MDC_2$) replacing phospholipid to the given amount |
| ad 1 ml | Isotonic phosphate buffer (*p*H = 7.2) |

Objective: to test the synergism between membrane destabilising, and thus aggregate adaptability increasing, activity of two different surfactants, used in a combination with a lipid, as the basic membrane forming system component.

Suspension preparation. To prepare a series with changing lipid/surfactant ratio in the range 1/1 to 9/1, the necessary amounts of phospholipid and surfactant are pipetted into buffer to yield 10% lipid suspensions. These are first stirred at room temperature for 5 days and then pre-filtered through a 0.8 micrometer polycarbonate filter to narrow down the starting aggregate diameter. The average vesicle diameter ($2r_{ves}$) is determined and confirmed to exceed at least 2-fold the nominal diameter of pores in the test filter ($2r_{pore}$), which is approximately 0.2 micrometer. This is done with the dynamic light scattering e.g. by using a Malvern Zeta-Sizer instrument.

Transport (pore penetration) capability. Transport resistance is equated with the volume of test suspension that does not pass through a 0.2 micrometer filter in a sterile holder. (A ready-to-use, commercially available "blue" filter unit of Sartorius (Göttingen, Germany) is used for the test.) This reveals that transport resistance decreases with increasing Tween content when the relative SPC content is lower than 6/1 (SPCrrw); see also examples 40-49 in EP 0 475 160. The trend is enhanced by the presence of sodium dodecylsulphate in the mixed lipid aggregates. The latter shifts the minimum amount of Tween needed to cross the semi-permeable barrier to increasingly lower relative concentration values.

For example, when 12 mol-% of SPC in the mixed amphipat aggregates is replaced by SDS, the suspension can be pushed through a barrier with 0.2 micrometer pores practically without transport resistance even when the relative SPC/Tween concentration is as low as 15/1. Increasing SDS content further does not improve the situation, as measured in this test series. In contrast, reducing SDS content to and below 10 mol-% relative to SPC shows a clear deterioration of penetration ability of the resulting quaternary suspension. Rather low transport resistance is now measured for SPC/Tween 7/1 (in case of 10 mol-% SDS concentration) and for SPC/Tween 4/1, when SDS concentration is between approx. 2 mol-% and 5 mol-%, as can be seen from figure 6. In contrast, maximum barrier resistance value of 10 is found for the suspensions without SDS and/or with little Tween and SDS, the properties of which approach those of plain, single component liposomes, which also have characteristic resistance value of 10.

Post-test determination of vesicle diameter confirms that vesicles are still at least 1.3-times greater than the nominal pore diameter.

**Examples 121-129:**

| Composition: | |
|---|---|
| 14.2 mg | Polysorbate (Tween 80) |
| 85.8 mg | Phosphatidylcholine from soy-bean (SPC), as with examples 1-120 |
| 0-17.5 rel. mol-% | Sodium dodecylsulphate (SDS), relative to SPC and replacing phospholipid to the given amount |
| ad 1 ml | Isotonic phosphate buffer (*p*H = 7.2) |

Objective: as with examples 1-120, to test the synergism of different surfactant action on extended surface aggregate properties.

Suspension preparation. The method used to prepare vesicle suspension was the same as in examples 1-120. The only notable difference between both test series was the somewhat greater average diameter and polydispersity of the vesicles used in examples 121-129.

Transportability (pore penetration capability and adaptability) of aggregate suspension. To characterise the resistance of semi-permeable barrier to suspension flux (= transbarrier flux), the same method as in examples 1-120 was used. The resistance was measured as a function of relative SDS concentration in bilayer, to determine minimum amount of this latter surfactant that is needed to maximise suspension flux across the barrier and minimises the barrier transport resistance value. Experimental data suggest that the threshold limit is around 6 mol-%, with some uncertainty in the 2-6 mol% region. This is consistent with the results of first test series (examples 1-120) except in that the measured resistance values are now somewhat higher. This is explicable by different starting vesicle diameter and polydispersity. The results are given in following table.

**Table 1:** The effect of SDS, as the second surfactant (*$MDC_2$*) in addition to Tween 80 (*$MDC_1$*; 10 mol-% rel. to SPC), on the resistance of mixed lipid membranes containing phosphatidylcholine (SPC; *MFC*), as the basic building block, to the passage through a semi-permeable barrier with pores, which were at least ~50% smaller than the average aggregate diameter.

| SDS/SPC [mol/mol] | Barrier transport resistance *[rel. units,* as defined in SEM*]* |
|---|---|
| 0/100, reference Tween Tfs | 10 |
| 2/98 | 4 |
| 4/96 | 10 |
| 6/94 | 1.88 |
| 8/92 | 1.75 |
| 10/90 | 1.50 |
| 25/175 | 1.12 |
| 15/85 | 0.75 |
| 35/165 | 0.44 |

**Examples 130-131:**

| Composition: | |
|---|---|
| [52.1 mg | Phosphatidylcholine from soy-bean (SPC), actual amount = 52.2 mg - Na Chol amount in mg |
| 45.2 mg | Polysorbate (Tween 80) |
| 5, 10, 15 mol-% | Sodium cholate = Na Chol (relative to SPC in the suspension) |
| ad 1 ml | Isotonic phosphate buffer (*p*H = 7.2) |

Objective: as with examples 1-120, but using a different charged surfactant (cholate instead of SDS).

Suspension preparation. The starting suspension was prepared as in previous examples. However, to make vesicles in the test formulation more uniform before actual measurements, the starting suspension was pre-filtered through 80 nm pore filters. This yielded vesicles with approx. 120 nm diameter, as determined with the dynamic light

scattering using ALV 5000 correlator and a personal computer.

Vesicle transport ability (pore penetration capability / adaptability). The actual transport test was done with relatively narrow pore (30 nm) filters, using different pressures applied on the filter to characterise the penetrability of such semi-permeable filter to the test suspension. This revealed fairly comparable penetration ability for the vesicles with 10 mol-% and 15 mol-% cholate, exceeding the pore penetration ability, and thus the adaptability, of the vesicles with merely 5 mol% of cholate as the third membrane destabilising component (cf. figure 3). These results indicate that incorporation of the second surfactant into mixed lipid bilayers does not increase membrane adaptability proportionally, as one would expect on the basis of model results shown in figure 7.

**Examples 132-138:**

| Composition: | |
|---|---|
| See Table 2 | Phosphatidylcholine from soy-bean (SPC) |
| See Table 2 | Ketoprofen, sodium (KT); |
| See Table 2 | Tween 80, see Table 2 |
| Ad 1 ml | Phosphate buffer (pH = 7.2) |

Objective: to test the synergistic effect of a membrane destabilising drug (KT) combined with a surfactant (Tween 80) in a lipid (SPC) suspension in terms of mixed aggregate adaptability and relative capability to cross semi-permeable barriers.

Test suspension preparation. The stated phospholipid and drug amounts were brought into suspension using mechanical homogenisation. That resulting average aggregate diameter was around 100 nm.

Vesicle transport ability (pore penetration capability / adaptability). The efflux of the test suspension from a vessel pressurised with nitrogen gas was measured as a function of the time to determine the pressure dependency of material transport through the 20 nm pore filter in front of an opening in the measuring vessel. From the measured flux data, the effective "barrier penetrability", which defines the adaptability of the tested mixed amphipat vesicles, was calculated as is described in the main text body. The measured curves were also analysed in terms of the pressure $p^*$, needed to achieve 57% of maximum possible suspension flux/pressure ratio. The result of the test series indicate that both ketoprofen and Tween can act as a membrane destabilising component. Consequently, either of these two system ingredients improves the ability of test suspension to penetrate barriers compared with simple phosphatidylcholine, reference liposomes in a suspension without KT or Tween 80. When a combination of said membrane destabilising components is used, extended surface aggregate adaptability is increased to the value measured with proper non-ionic Tween-based Transfersome® suspension, with surfactant concentration much higher than that used in the quaternary mixture. Data given in Table 2 justify the conclusion. They are also compared with those pertainint to simple buffer fluid (Ref. fluid) in which the mixed SPC/KT/Tween vesicles were suspended.

**Table 2:** Experimental and fit results for the pore penetration experiments done with various quaternary suspensions of a phospholipid (SPC; *MFC*), a drug (KT; $MDC_1$), and Tween 80 ($MDC_2$) co-suspended in a buffer; TL = total lipid

| Tween 80 [mol% of SPC] | Ketoprofen [mol% of TLI] | $p^*$ [MPa] | $P_{max}$ [$10^{-11}$m $Pa^{-1} \cdot sec^{-1}$] | Adaptability $a_a$, [$MPa^{-1}$] |
|---|---|---|---|---|
| 0 (Liposomes) | 0 | > 3 | Not measurable | (< 0.3) |
| 0 | 25 | $2.41 \pm 0.15$ | Not measurable | 0.415 |
| 0 | 33 | $1.66 \pm 0.07$ | $345 \pm 37$ | 0.602 |
| 10 | 33 | $0.25 \pm 0.03$ | $230 \pm 17$ | 4.000 |
| 50 | 0 | $0.20 \pm 0.01$ | $227 \pm 3$ | 5.000 |
| 0 (=Ref. Fluid) | 0 | Not applicable | $613 \pm 15$ | Not applicable |

**Examples 139-142:**

| Composition: | |
|---|---|
| 75.0 mg | Phosphatidylcholine from soy-bean (SPC), used as a saturated ethanolic solution SPC amount = 75 mg - Brij 98 content given in Table 3 |
| 25.0 mg | Ketoprofen, sodium (KT) |
| See Table 3 | Brij 98 |
| Ad 1 ml | Phosphate buffer (*p*H = 7.2) |

Objective: to test adaptability / pore penetrability supporting activity of a different surfactant (Brij) combined with a membrane destabilising drug (KT) in lipid (SPC) extended surface aggregates.

Suspension preparation was essentially the same as in examples 132-135.

Vesicle transport ability (pore penetration capability / adaptability). In order to test whether or not the increased adaptability of SPC/KT ternary suspensions is a unique feature of Tween, as the fourth component, the effect of another surfactant was investigated. In order to avoid undesired electrostatic interactions between the anionic KT and such additive, the uncharged Brij 98 (oleoyl-chain, 20 oxyethylene units per molecule) was chosen. The penetrability of resulting SPC/KT/Brij 3/1/0-0.323 w/w/w mixtures was finally calculated using eq. (*).

The results for similar series measured with Brij 98 are given in Table 3.

**Table 3:** Fit results, based on e.q. (*), for the transbarrier flux of suspensions containing a lipid (SPC; *MFC*), a drug (KT; $MDC_1$) and Brij 98 ($MDC_2$) in different relative concentrations, SPC and Brij together representing the total lipid (TL)

| Brij 98 [mol% of SPC] | KT [mol% of TL] | $p^*$ [MPa] | $P_{max}$ [$10^{-11}$m $Pa^{-1}\cdot sec^{-1}$] | Adaptability $a_a$, [$MPa^{-1}$] |
|---|---|---|---|---|
| 0 | 33 | 1.66 ± 0.07 | 345 ± 37 | 0.602 |
| 2.5 | 33 | 0.56 ± 0.07 | 266 ± 28 | 1.786 |
| 5.0 | 33 | 0.29 ± 0.07 | 191 ± 30 | 3.448 |
| 7.5 | 33 | 0.32 ± 0.06 | 171 ± 21 | 3.125 |
| §The quoted error only accounts for analytical and not for experimental data uncertainty, which for example 16 exceeds 80% | | | | |

**Examples 143-146:**

| Composition: | |
|---|---|
| 100 mg | Total lipid (TL, including SPC and Tween 80) |
| See Table 4 | Phosphatidylcholine from soy-bean (SPC) |
| See Table 4 | Tween 80 |
| See Table 4 | Diclofenac |
| See Table 4 | Ethanol (EtOH) |
| 5.25 | Benzyl alcohol |
| Ad 1 g | 154 mM Phosphate buffer, pH = 7.2 |

Objective: to test the effects of a surfactant (Tween 80) and a drug (diclofenac), as two membrane destabilising amphipats, and of a short-chain alcohol (ethanol) as an additional - and potentially the second membrane destabilising amphipat.

Vesicle preparation was done essentially as described in example 8 of WO 98/17255, but a more modern version of barrier penetration assay was used to assess vesicle aggregate adaptability. For historic comparison, vesicles with a similar overall composition but lacking ethanol were tested (cf. examples. The results are given in Figure 8 and in Table 4.

Due to the limited measuring range of pore penetration assay, it was only possible to obtain a rough estimate for the adaptability of extended surface aggregates tested in this test series. The estimated *p**-value of the preparations

containing ethanol were lowered to ~1.6 MPa from ~4.8 MPa measured in the absence of this alcohol. (It must be kept in mind, however, that experimental variability in these tests was at least 50%, as the standard deviations given in Table 4 only stem from the fit routine.) The direction of the change is reasonable, but the calculated absolute difference in $p^*$ is not significant.

**Table 4:** Results of driving pressure and aggregate adaptability analysis for the test.

| Ex | Tween 80 [mol% of SPC] | EtOH [w-%] | Diclofenac [w-% of TL] | $p^*$ [MPa] | $P_{max}$ [$10^{-11}$m $Pa^{-1}\cdot sec^{-1}$] | Adaptability $a_a$, [$MPa^{-1}$] |
|---|---|---|---|---|---|---|
| 143 | 0 | 0 | 10 | (4.8 ± 1.6) | Not measurable | 0.208 |
| 144 | 0 | 9 | 10 | 2.4 ± 0.04 | 402 ± 17 | 0.417 |
| 145 | 0 | 9 | 20 | | | |
| 146 | 10 | 9 | 10 | | | |
| §The quoted error only accounts for analytical and not for experimental data uncertainty, which for example 16 exceeds 80%. | | | | | | |

Data given in Figure 8 and in Table 4 imply that ethanol makes the tested lipid aggregates more adaptable. The effect is much smaller, however, than in case of using a surfactant, such as Tween 80 (see Table 2).

Simple use of a membrane destabilising drug (diclofenac) and of a short-chain alcohol, as membrane softening agents disclosed in the prior art, thus only produces extended surface aggregates with an adaptability significantly inferior to that of the formulations disclosed in the present invention.

Specifically, the formulation described in Example 8 of WO 98/17255 is capable of crossing semipermeable barriers with narrow pores, but leaves space for further improvement. Ethanol containing, diclofenac loaded vesicles, indeed, are more adaptable than the ethanol-free vesicles. However, even the former vesicles have a much higher $p^*$ value, and therefore are far less adaptable, than the ternary mixtures of phosphatidylcholine, a non-ionic surfactant (Tween 80) and ketoprofen described in Table 2; the beneficial effect of a surfactant-like membrane destabilising component, such as Tween 80, is directly reflected in the lower $p^*$ value and/or in a higher flux of the modified formulation through a barrier. This conclusion is practically inaffected if the latter formulation contains ethanol.

It therefore stands to reason that at least two membrane destabilising components should be present in an aggregate with extended surface in adequate quantities to maximise the adaptability of extended surface aggregates. Mere use of a lipid, ethanol and a drug, as is disclosed in the prior art, is insufficient for reaching the goal.

**Examples 147-150**

| Composition: | |
|---|---|
| 80.0-71.4 mg | Phosphatidylcholine from soy-bean (SPC) |
| 20-28.6 mg | Ketoprofen, sodium (KT), replacing SPC in the suspension to achieve constant amphipat amount |
| ad 1 ml | Phosphate buffer, $p$H = 7.2, if necessary readjusted with NaOH |

Objective: to demonstrate that ketoprofen, an NSAID, acts as membrane destabilising component and can render mixed amphipat aggregates with extended surface adaptable enough to penetrate narrow pores.

Test suspension preparation. The stated phospholipid and drug amounts were brought into suspension using mechanical homogenisation. That resulting average aggregate diameter was around 100 nm. For reference, a comparable suspension containing SPC and sodium cholate in 3.75/1 mol/mol ratio was used.

Vesicle transport ability (pore penetration capability / adaptability). The efflux of the test suspension from a vessel pressurised with nitrogen gas was measured as a function of the time to determine the pressure dependency of material transport through the 20 nm pore filter in front of an opening in the measuring vessel. From the measured flux data, the effective "barrier penetrability", which defines the adaptability of the tested mixed amphipat vesicles, was calculated as is described in the main text body. The measured curves were also analysed in terms of the pressure $p^*$, needed to achieve 57% of maximum possible suspension flux/pressure ratio. The calculated $p^*$-value decreased from 2.41 ± 0.15 MPa (mean value ± standard error) through 1.66 ± 0.07 MPa to 1.36 ± 0.10 MPa with increasing drug concentration. This is indicative of membrane destabilising activity of the drug, which arguably promotes bilayer flexibility

and permeability. More detailed information is given in Table 5, which reveals essentially identical $p^*$ values for the SPC/KT 3/1 mol/mol mixture and for the reference anionic Transfersome® suspension. To deduce vesicle adaptability from $p^*$-value, contribution from suspension viscosity effects must be included or must be known to be negligible. This is not an issue, however, as long as one can make comparisons with suitable reference formulation(s), as is done in the following table by inclusion of last line.

In this test series, an in all other practical examples reported herein, the final aggregate diameter after narrow pore crossing was at least 300%, and typically was more than 400% greater than the pore diameter, the final to starting aggregate diameter ratio being typically > 0.70, implying fragmentation of less than 30%, and more often merely 10-20%.

**Table 5:** Fit results, based on eq. (*) for the barrier penetrability (flux/pressure ratio) experiments done with the suspensions characterised by different lipid/drug, SPC/KT ratios

| SPC/KT [mole/mole] | $p^*$ [MPa] | $P_{max}$ [$10^{-11}$m $Pa^{-1}\cdot sec^{-1}$] | Adaptability $a_a$, [$MPa^{-1}$] |
|---|---|---|---|
| 10/0 | ~3 | Not measurable | ~0.3 |
| 4/1 | $2.41 \pm 0.15$§ | Not measurable | 0.415 |
| 3/1 | $1.66 \pm 0.07$§ | - | 0.602 |
| 2.5/1 | $1.36 \pm 0.10$§ | $345 \pm 37$ | 0.735 |
| Reference anionic Tfs§§ | $1.76 \pm 0.13$§ | $318 \pm 39$ | 0.568 |

§The quoted error only accounts for analytical and not for experimental data uncertainty, the latter often amounting to 20-30%. §§These Tfs vesicles were prepared from an SPC/Na cholate 3.75/1 mol/mol mixture.

Graphic representation of the results of these experiments is given in Figure 9.

**Examples 151-153:**

| Composition: | |
|---|---|
| 75.0, 75.0, 37.7 mg | Phosphatidylcholine from soy-bean (SPC) |
| 25.0, 25.0, 0.0 mg | Ketoprofen, sodium (KT) |
| 0.0, 25.4, 62.3 mg | Tween 80 |
| 0.0, 0.0, 37.7 mg | Ethanol |
| ad 1 ml | Phosphate buffer ($p$H = 7.2) |

Objective: to test the synergistic effect of the second and first membrane destabilising amphipat (Tween 80, ketoprofen, respectively) in terms of an extended surface aggregate adaptability.

Suspension preparation was essentially the same as with examples 147-150.

Vesicle transport ability (pore penetration capability / adaptability). Transbarrier flux of the test suspension containing 5 mol-% Tween is much higher than for the formulation that contains merely phospholipid (as the basic amphipat) and ketoprofen (as the surface active, membrane destabilising, surfactant-like amphipat) components. This is clearly seen from Figure 10, which illustrates pressure dependence of said suspension flux divided by driving pressure.

**Examples 154-158:**

| Composition of aggregates: | |
|---|---|
| 75.0 mg | Phosphatidylcholine from soy-bean (SPC), the actual value is: 75 mg - Tween 80 amount in mg |
| 25.0 mg | Ketoprofen, sodium (KT) |
| see the following table | Tween 80 |
| ad 1 ml | Phosphate buffer ($p$H = 7.2) |

(continued)

| Composition of aggregates: | |
|---|---|
| Reference buffer: | Phosphate buffer (*p*H = 7.2) |

Objective: to study the effect of relative concentration of a surfactant, as the second membrane destabilising amphipat, on adaptability of extended surface mixed amphipat aggregates.
Suspension preparation: as with examples 147-150.
Vesicle transport ability (pore penetration capability / adaptability) data, as measured in this test series, confirm and expand the findings obtained with examples 147-150. Tween acting as the second membrane destabilising component improves the ability of test suspension to penetrate barriers even when this surfactant is present in the quaternary mixture merely in small amount, as long as relative concentration of Tween is at least approximately 2.5 mol-%, and even better 5 mol-%. Data given in Table 6 justify the conclusion. They are compared with the reference non-ionic Tween-based Transfersome® formulation (Reference Tfs) and with the buffer fluid (Reference fluid) in which mixed amphipat vesicles were suspended.
The suspension viscosity for example 157 was around 730 mPa s at 20 RPM

**Table 6**: Fit results for the pore penetration experiments done with various quaternary suspensions of a phospholipid (SPC; stable membranes forming component), a drug (KT; 1st membrane destabilising component), and Tween 80 (2nd membrane destabilising component) co-suspended in a buffer at different relative concentrations of Tween 80.

| Nr | Tween 80 content [mol% of SPC] | $p^*$ [MPa] | $P_{max}$ [$10^{-11}$m $Pa^{-1}\cdot sec^{-1}$] | Adaptability $a_a$, [$MPa^{-1}$] |
|---|---|---|---|---|
| | 0 | 1.66 ± 0.07 | 345 ± 37 | 0.602 |
| 154 | 1.25 | 0.51 ± 0.05 | 293 ± 23 | 1.961 |
| 155 | 2.5 | 0.50 ± 0.04 | 339 ± 26 | 2.000 |
| 156 | 5 | 0.23 ± 0.03 | 215 ± 19 | 4.348 |
| 157 | 7.5 | 0.22 ± 0.02 | 213 ± 14 | 4.545 |
| 158 | Reference Tfs (Tween) | 0.20 ± 0.01 | 227 ± 3 | 5.000 |
| | Reference fluid (buffer) | Not applicable | 613 ± 15 | Not applicable |

§The quoted error only accounts for analytical and not for experimental data uncertainty, the latter often amounting to 20-30%.
Reference Tfs vesicles were prepared from an equimolar (50/50 mol/mol) SPC/Tween 80 mixture.

**Examples 159-160:**

| Composition: | |
|---|---|
| 43.65 mg | Phosphatidylcholine from soybean (+95% = PC) |
| 72.00 mg | Tween 80 |
| 34.35 mg | Ketoprofen |
| 6.08 mg | Sodium hydroxide |
| 5.25 mg | Benzyl alcohol |
| 36.51 mg | Ethanol 96% |
| ad 1 g | 154 mM phosphate buffer, *p*H = 7.4 |

Objective: The confirm novelty of the formulations described in this application in general.
For the purpose, the closest examples given in previous relevant patents (applications) were reproduced combining all the potentially relevant explicit teachings from such previous art documents. Experimental procedures were selected correspondingly, except for the usage of more modern analytical methods.

Suspension preparation: On the one hand, the concentration of the surfactant Tween 80, which acts as a membrane destabilising component, was used at the level suggested by examples 40-49 of USP 6 165 500 (or of the equivalent EP 0475 160 A1). An equimolar mixture of phosphatidylcholine and Tween 80 was chosen, as the adaptability of such mixture, expressed in terms of its inverse value which is proportional to the tested barrier resistance, for such mixture approaches zero. On the other hand, the partially ionised ketoprofen, which binds to lipid bilayers and makes such membranes more flexible, was used as the second membrane destabilising component. Such a drug usage is taught explicitly in WO 98/17255 for two other NSAIDs: diclofenac and ibuprofen, e.g. in Practical Examples 8-17 and 18-25.

The tested membrane composition thus corresponded to the optimum SPC/KT ratio suggested in Table 6 for the SPC/KT/Tween 80 mixtures with an increased molar SPC/Tween 80 ratio. The weight percent of ketoprofen, relative to the total lipid concentration, was thus around 3/1 and hence similar to that taught in said examples in WO 98/17255 for diclofenac, which cover molar ratios 4/1 to 1/4. Also in accord with WO 98/17255, an isotonic phosphate buffer was used to suspend the resulting mixed lipid vesicles.

Hydration of the components mixed in given proportions produced a clear, yellowish fluid. This is indicative of micellar suspension and implies that the tested mixed lipid aggregates are colloidally not stable. Determination of the average diameter of aggregates in such suspension with the dynamic light scattering confirmed the conclusion (mean particle diameter approx. 22 nm, which is incompatible with existence of vesicles).

Diluting the preparation with the corresponding buffer from 15% total lipid to 10% total lipid, making essentially the same observation further corroborated the result. Based on the existing information about phosphatidylcholine solubilisation by Tween 80, even a reduction of relative surfactant concentration by a factor of 2, thus creating a SPC/Tween 80 2/1 mol/mol mixture loaded with approx. 30 mol-% ketoprofen, still would yield unstable aggregates.

Addition of Tween 80 much beyond the rather low relative molar concentration proposed in example 158 thus destabilises the three component lipid aggregates to the point of solubilisation, or at close to this point. Compositions originating from the combination of relevant experimental teachings in patents WO 98/17255 and EP 0475 160 A1 therefore do not fulfil the required stability criterion for the extended surface aggregates and consequently do not represent prior art to present application.

**Comparative Examples 161-162:**

| Composition: | |
|---|---|
| 66.71 mg | Soybean-phosphatidylcholine |
| 11.00 mg | Tween 80 |
| 22.21 mg | Ketoprofen |
| 0.00 / 66.71 mg | Ethanol (EtOH; for examples 16 and 17, respectively) |
| 11.56 mg | NaOH (30%) |
| 0.50 mg | Na metabisulphite |
| 1.00 mg | Disodium edetate (EDTA) |
| 0.20 mg | Butylhydroxytoluene (BHT) |
| 1.46 mg | Methylparabene |
| 1.00 mg | Linalool |
| 5.25 mg | Benzyl alcohol |
| ad 1 g | 7.8 mM Phosphate buffer, *p*H = 7.2 |

Objectives: First, to check membrane destabilising and aggregate adaptability increasing effect of ethanol was in the range of concentrations described in previous inventions (see USP 6 165 500 or the equivalent EP 0475 160 A1). The results, given in Table 7, confirm that the adaptability of the aggregates proposed in prior art is far inferior to that of the newly proposed formulations.

Second, to test the effect of system stabilisers (Na metabisulphite; EDTA; BHT, benzyl alcohol) on essential characteristics of extended surface aggregates. The results confirmed the that the key system parameters, which determine the suspension ability to cross semipermeable barriers, i.e. pressure $p^*$ and aggregate adaptability, are not inacceptably affected by such additives.

Suspension preparation. Vesicular intermediate preparation with 17.14% total lipid containing no ethanol and ketoprofen in identical concentration as in Example 157 was mixed with the SPC mass equivalent of ethanol. This was done to match as closely as possible the examples 8-17 given in WO 98/17255. To meet the needs of pharmaceutical

formulations as well, several suspension stabilising agents (EDTA, BHA, methylparabene, and benzyl alcohol) were included in the formulation. Characterisation was done as with examples 147-150.

**Table 7:** Results of driving pressure and aggregate adaptability analysis for examples 15 and 16.

| Formulation | $p^*$ [MPa] | $P_{max}$ [$10^{-8}$ kg / ($m^2 \cdot s \cdot Pa$)] | Adaptability $a_a$, [$MPa^{-1}$] |
|---|---|---|---|
| Example 161 (no EtOH) | 0.233 ± 0.013§ | 216.5 ± 7.4 | 4.292 |
| Example 162 (with EtOH) | 0.133 ± 0.006§ | 254.3 ± 9.7 | 7.519 |
| §The quoted error only accounts for analytical and not for experimental data uncertainty. | | | |

Specifically, the pressure required to drive vesicles through narrow pores, *p**, was found to decrease in the presence of EtOH from 0.233 MPa to 0.133 mPa; this is a decrease of approx. 40% and thus near the limit of insignificance (see Table 6 for comparison). The reason is the limited assay resolution, which for *p** in the studied situation is 20-30%.

Speaking in absolute terms, and making comparison with the magnitude of positive effect on aggregate adaptability caused by Tween 80 (cf. Tables 6 and 7), ethanol in the range disclosed in USP 6 165 500, EP 0475 160 A1 and WO 98/17255 only increases the adaptability of tested aggregates moderately. Similar conclusion is reached by comparing examples 50-61 in USP 6 165 500 (or in the equivalent EP 0475 160 A1).

Comparison of the results from experiments 161 and 162 and 157, moreover, confirms that the tested system preservatives (Na metabisulphite; EDTA; BHT, benzyl alcohol) neither affect negatively the desirable extended surface aggregate adaptability nor do they change much the pressure required for driving adequate suspension transport through a nano-porous barrier.

**Examples 163-165:**

| Composition: | |
|---|---|
| 75 mg | Phosphatidylcholine from soy-bean (SPC), |
| 25 mg | Ketoprofen, sodium (KT) |
| See the following table | Tween 80 (mol-% referring to SPC) |
| ad 1 ml | Water or 50 mM buffer (*p*H = 7.2) |

Objective: to test the influence of ionic strength of the bulk inorganic electrolyte on the adaptability of mixed amphipat aggregates suspended in such an electrolyte.

Suspension preparation and characterisation. The test suspension was prepared essentially as with examples 147-150, except in that the buffer was sometimes exchanged for water with practically the same *p*H-value. This had important consequences. When the ionic strength (I) of the bulk electrolyte solution with a pH near 7 changes, ketoprofen distribution and degree of ionisation in Transfersome® suspension also changes. This modifies - most probably decreases - extended surface aggregate adaptability, which must be considered when designing products on the basis of given formulation composition. Experimental evidence for this is given in Table 8.

**Table 8:** The fit results based on formula (*) for the transbarrier flux/driving pressure ratio (barrier penetrability), of various quinternary suspensions with KT as the drug in different buffer systems.

| Formulation | $p^*$ [MPa] | $P_{max}$ [$10^{-11}$m $Pa^{-1} \cdot sec^{-1}$] | Adaptability $a_a$, [$MPa^{-1}$] |
|---|---|---|---|
| 10 mol% Tween no buffer | 0.49 ± 0.02 | 212 ± 8 | 2.041 |
| 10 mol-% Tween, 50 mM buffer, I=117mM | 0.25 ± 0.03 | 230 ± 17 | 4.000 |
| 7.5 mol-% Tween, 6.3% v/v EtOH no buffer | 0.31 ± 0.06 | 194 ± 23 | 3.226 |

(continued)

| Formulation | $p^*$ [MPa] | $P_{max}$ [$10^{-11}$m $Pa^{-1}\cdot sec^{-1}$] | Adaptability $a_a$, [$MPa^{-1}$] |
|---|---|---|---|
| 7.5 mol-% Tween, 6.3% v/v EtOH 50 mM buffer, I = 117 mM | 0.13 ± 0.01 | 248 ± 11 | 7.692 |
| Reference Tween Tfs in the buffer | 0.20 ± 0.01 | 227 ± 3 | 5.000 |

**Examples 166-167:**

| Composition: | |
|---|---|
| 75.0 mg | Phosphatidylcholine from soy-bean (SPC), |
| 25 mg | Ketoprofen, sodium (KT) |
| 12.4 mg | Tween 80 |
| ad 1 ml | Buffer *p*H = 7.2 and pH = 7.7 |

Suspension preparation and characterisation: see previous test series.
Objective: to test the effect of ketoprofen ionisation, which above the pKa(KT) ~ 6.4 increases with pH, on adaptability of the drug loaded mixed lipid vesicles.
Results: Adaptability of simple formulations containing three amphipatic components was confirmed to depend on the ionisation state of its only titratable component, ketoprofen. Detailed results are given in the following Table 9.

**Table 9:** Fit results, based on eq. (*), for the pressure normalised transbarrier flux of KT-Tfs suspensions at different *p*H

| *p*H | $p^*$ [MPa] | $P_{max}$ [$10^{-11}$m $Pa^{-1}\cdot sec^{-1}$] | Adaptability $a_a$, [$MPa^{-1}$] |
|---|---|---|---|
| 7.2 | 1.66 ± 0.07 | 345 ± 37 | 0.602 |
| 7.7 | 0.62 ± 0.07 | 237 ± 28 | 1.613 |
| Reference Tfs | 0.20 ± 0.01 | 227 ± 2.9 | 5.000 |

**Examples 168-169:**

| Composition: | |
|---|---|
| 100 mg/ml | Phosphatidylcholine from soy-bean (SPC) as large unilamellar vesicle suspension |
| 254 mg/ml | Ketoprofen, sodium (KT) in solution |
| | Buffer *p*H = 7.2 and *p*H = 7.7 |
| | *Mixed during experiments to yield increasing relative ratio of KT in SPC aggregates suspension.* |

Objective: to test the ability of ketoprofen to solubilise lipid bilayer membranes.
Results: The ability of ketoprofen to solubilise soybean phosphatidylcholine (SPC) membranes was determined by measuring the turbidity of a suspension (10 w-%) of large unilamellar vesicles during successive addition of 1 M solution of ketoprofen. In the first test series this was done in 50 mM phosphate buffer at *p*H = 7.4, where more than 50% of the drug is ionised and more than 50% of the drug is vesicle-bound, but chiefly in the non-charged form, which does not destabilise lipid membranes significantly. SPC vesicles under tested these conditions were not measurably solubilised, despite the presence of some ionised ketoprofen, but were partly destabilised, as demonstrated in previous examples.
The second experiment was performed at *p*H = 11.6, under which condition all ketoprofen molecules are deprotonated and hence have a maximum solubilisation, i.e. membrane destabilisation, capability. Solubilisation of SPC

membranes was now observed when the molar ratio for the drug in vesicle bilayers was above ketoprofen/SPC ~10.8/1 mole/mole. SPC-ketoprofen association thus produces weakly bound complexes with membrane solubilising capability.

**Examples 170-174:**

| Composition: | |
|---|---|
| 75.0 mg | Phosphatidylcholine from soy-bean (SPC, used as a saturated ethanolic solution) the actual number is: 75 mg - Brij content |
| 25.0 mg | Ketoprofen, sodium (KT) |
| See the following table | Brij 98 |
| ad 1 ml | Phosphate buffer (*p*H = 7.2) |

Objective: to demonstrate the usefulness of another surfactant, Brij, different from Tween 80, to increase the flux through narrow pores of ketoprofen/SPC extended surface aggregates in a suspension.

Suspension preparation was essentially the same as in examples 147-150.

Flux determination. The flux of suspension of extended surface aggregates containing SPC, KT and, in case, Brij 98 was measured using the same device as is used for aggregate adaptability determination. The only difference was that only a single driving pressure was used for suspension characterisation. For comparison, the ratio of KT-loaded and of empty Brij Transfersomes® was calculated (= Rel. Flux).

The results of the test series measured with Brij 98, a polyoxyethylene-oleyl-ether with 20 OE units in polar head are given in Table 10.

**Table 10**: Flux of mixed amphipat suspensions through 20 nm pores in a semi-permeable barrier driven by trans-barrier pressure of 0.1 MPa.

| Brij 98 content [mol% of SPC] | *Flux* [mg $cm^{-2}$ $sec^{-1}$] | *Rel. Flux* |
|---|---|---|
| 0 | <1 | |
| 2.5 | 10 | >10 |
| 5.0 | 30 | >30 |
| 7.5 | 29 | >29 |

**Examples 175-178:**

| Composition KT Form(ulation) B (Expt 175): | |
|---|---|
| Weight-% | |
| 2.857 | Ketoprofen |
| 7.143 | Phosphatidylcholine |
| 3.000 | Glycerol |
| 2.087 | Sodium Hydroxide, 50% (FCC) |
| 0.120 | Phosphate buffer salts |
| 0.100 | Linalool |
| 0.100 | Disodium edetate EDTA |
| 1.250 | Carbomer 974 |
| 0.100 | Carbomer 1342 |
| 1.000 | Propylen Glycol |
| 0.200 | Ethylparaben |
| 0.525 | Benzyl Alcohol |
| 0.020 | Butylated hydroxytoluene |

(continued)

| Composition KT Form(ulation) B (Expt 175): | |
|---|---|
| Weight-% | |
| 81.499 | Water |

| Composition KT Form.(ulation) A (Expt 176): | |
|---|---|
| Weight-% | |
| 2.290 | Ketoprofen |
| 6.870 | Soy Phosphatidylcholine (SPC) |
| 0.850 | Polysorbate (Tween 80) |
| 3.651 | Ethanol 96% |
| 0.930 | NaOH (sodium hydroxide) |
| 0.235 | Phosphate buffer salts |
| 0.050 | Sodium metabisulphite |
| 0.020 | Butylhydroxytoluene (BHT) |
| 0.100 | Disodium edetate (EDTA) |
| 0.250 | Methyl parahydroxybenzoate |
| 0.525 | Benzyl alcohol |
| 0.100 | Linalool |
| 1.250 | Carbomer (Carbopol 980) |
| 3.00 | Glycerol |
| 79.879 | Water |

Commercial topical formulation Gabrilen (Expt. 177): according to desk physicians' reference, the preparation contains 25 mg KT/g gel, supplemented with 96% ethanol, 3-propanol, 10% ammonia solution and Carbomer in purified water.

Commercial oral formulation Ketoprofen Ratiopharm (KT Ratiopharm) (Expt. 178): according to desk physicians' reference each film tablet contains 50 mg KT in addition to microcrystalline cellulose, gelatine, SiO2, corn starch, talcum, crosscarmelose sodium, Mg stearate, hypromelose, macrogol, glycerol, dyes E 171 and E 172.

Preparation of formulations A and B, which both contained extended surface vesicles, was done essentially as described for examples 147-150. Commercial comparators were purchased in a local pharmacy and used as obtained.

Methodology: The test pigs were numbered and central vein catheters were implanted into the animals. The application area on a hind limb of each animal was shaved with an electric clipper and cleaned with warm water and soap. Then, an application area of 10 cm x 10 cm (= 100 $cm^2$) was marked.

At time zero of the sampling period, 2 ml of the blood were sampled from each test animal into a citrate-coated vial to generate plasma. The pigs were anaesthetised for approximately 60 min and the appropriate dose of the test medication was applied onto the application site of a pig or else was given to the animal orally. Further plasma samples (0.5 ml each) were taken 0.5, 1, 2, 3, 5, 8 and 12 hours post application. They were kept frozen until analysis.

Ketoprofen concentration was determined with HPLC using standard methods, in case of muscle tissue samples after the specimen homogenisation. Area under the curve (AUC) was calculated by integrating all time-point data.

Results of experiments are given in Tables 11 and Figure 11. Whereas the individual pharmacokinetic data sets are rather scattery, yielding standard deviations comparable to the mean because of small group size, the overall data analysis does demonstrate the superiority of at least three amphipat component preparations, in comparison with two amphipat component formulations, to deliver an NSAID (ketoprofen) deep under the application site on the skin. The greater is the investigated tissue depth the greater is the observed advantage (superficial muscle = 0-1.5 cm; deep muscle > 1.5 cm).

**Table 11a:** Area under the curve ($AUC_{0-8h}$ [ng x $mg^{-1}$ x h]), measured with different KT formulations in pigs

| | Gabrilen® (n=4) | Formulaion B (n=7) | Formulation A (n=7) | KT Ratiopharm® (oral, n=3) |
|---|---|---|---|---|
| Superficial muscle tissue | 102 | 209 | 306 | 7 |

(continued)

| | Gabrilen® (n=4) | Formulaion B (n=7) | Formulation A (n=7) | KT Ratiopharm® (oral, n=3) |
|---|---|---|---|---|
| Deep muscle tissue | 53 | 147 | 301 | 9 |

**Table 11b:** Ketoprofen (KT) concentration in superficial muscle tissue (ng/mg)

| Time (hours) | Gabrilen® (n=4) | KT-Tfs Form. B (n=7) | KT-Tfs Form. A (n=7) | KT-Ratiopharm (oral) (n=3) |
|---|---|---|---|---|
| 0 | 0.0 ± 0.0 | 0.0 ± 0.0 | 0.0 ± 0.1 | 0.0 ± 0.0 |
| 1 | 5.0 ± 3.3 | 50.4 ± 48.6 | 55.5 ± 66.3 | 1.0 ± 1.2 |
| 2 | 12.8 ± 22.6 | 75.2 ± 83.8 | 36.3 ± 32.1 | 1.6 ± 1.2 |
| 3 | 10.9 ± 11.5 | 3.0 ± 3.2 | 25.7 ± 28.5 | 1.4 ± 0.3 |
| 5 | 19.3 ± 18.7 | 12.9±11.1 | 45.2 ± 72.9 | 0.7 ± 0.2 |
| 8 | 3.8 ± 3.8 | 19.6 ± 17.9 | 22.0 ± 17.9 | 0.2 ± 0.1 |

**Table 11c:** Ketoprofen (KT) concentration in deep muscle tissue (ng/mg)

| Time (hours) | Gabrilen® (n=4) | KT-Tfs Form. B (n=7) | KT-Tfs Form. A (n=7) | KT-Ratiopharm (oral) (n=3) |
|---|---|---|---|---|
| 0 | 0.0 ± 0.0 | 0.0 ± 0.0 | 0.0 ± 0.1 | 0.0 ± 0.0 |
| 1 | 2.6 ± 2.3 | 53.4 ± 66.5 | 24.8±19.0 | 1.5±1.6 |
| 2 | 5.4 ± 9.3 | 63.0±51.9 | 18.8±21.5 | 1.8±1.0 |
| 3 | 9.0 ± 9.3 | 1.4 ± 0.8 | 49.8 ± 71.8 | 1.6 ± 0.5 |
| 5 | 7.9 ± 5.8 | 5.6 ± 2.2 | 49.9 ± 65.0 | 1.0 ± 0.2 |
| 8 | 2.9 ± 2.9 | 14.1 ±10.9 | 30.2 ± 28.7 | 0.3 ± 0.2 |

**Examples 179-180:**

Composition for ketoprofen in carrier suspension (KT-Tfs sol):

| Weight-% | |
|---|---|
| 3.435 | Ketoprofen (KT) |
| 10.305 | Soy Phosphatidylcholine (SPC) |
| 1.275 | Polysorbate (Tween 80) |
| 5.477 | Ethanol 96% |
| 0.533 | NaOH (sodium hydroxide) |
| 0.235 | Phosphate buffer salts |
| 0.050 | Sodium metabisulphite |
| 0.020 | Butylhydroxytoluene (BHT) |
| 0.100 | Disodium edetate (EDTA) |
| 0.250 | Methyl parahydroxybenzoate |
| 0.525 | Benzyl alcohol |
| 0.100 | Linalool |

(continued)

| Weight-% | |
|---|---|
| 3.00 | Glycerol |
| 74.695 | Water |

<u>Composition for ketoprofen in carrier gel (KT-Tfs gel):</u>

As in experiment 179, except in that the first four components are diluted 1.5-fold and Carbomer (Carbopol 980), buffered to *p*H = 7.2, is included to final concentration of 1.25 w-%.

<u>Objective</u>: to test the effect of formulation viscosity, and the presence of a thickening agent as viscosity modifier, on the ability of NSAID loaded extended surface aggregates to deliver the drug (ketoprofen) deep under the application site on the skin.

<u>Methodology</u> was the same as in experiments 175-178, except in that no oral comparator was included. A total of 4 pigs were used in each group.

Area under the curve (AUC) was calculated by integrating all PK (pharmacokinetic) data measured in different tissues (plasma, not shown) and the muscles under drug application site on the skin. The results obtained for superficial (0-1.5 cm) and deep (> 1.5 cm) muscle are given in Tables 12, and suggest no detrimental effect of the thickening agents used in KT-Tfs gel to achieve the desired suspension viscosity of approx. 730 mPa s. If anything, the thickening agent present in the tested gel is beneficial.

**Table 12a**: Area under the curve ($AUC_{0-8h}$ [ng $mg^{-1}$ h]), measured with two carrier-based ketoprofen (KT) formulations in pigs

| | KT-Tfs gel 17 mg (n=4) | KT-Tfs sol. 17 mg (n=4) | KT-Tfs gel 50 mg (n=4) | KT-Tfs sol. 50 mg (n=4) |
|---|---|---|---|---|
| Superficial muscle tissue | 147 | 44 | 278 | 186 |
| Deep muscle tissue | 97 | 63 | 266 | 202 |

**Table 12b**: KT concentration in superficial muscle tissue (ng/mg)

| Time (hours) | KT-Tfs gel 17 mg (n=4) | KT-Tfs sol. 17 mg (n=4) | KT-Tfs gel 50 mg (n=4) | KT-Tfs sol. 50 mg (n=4) |
|---|---|---|---|---|
| 0 | 0.0 ± 0.0 | 0.0 ± 0.0 | 0.0 ± 0.1 | 0.0 ± 0.0 |
| 1 | 83.3 ± 82.9 | 2.3 ± 1.5 | 55.5 ± 66.3 | 23.0 ± 29.3 |
| 2 | 24.1 ±27.5 | 0.8 ± 0.3 | 36.3 ± 32.1 | 21.2 ± 33.6 |
| 3 | 8.1 ± 8.0 | 2.8 ± 0.1 | 25.7 ± 28.5 | 9.0 ± 2.1 |
| 5 | 14.2 ± 14.2 | 10.6 ± 12.5 | 45.2 ± 72.9 | 34.8 ± 49.8 |
| 8 | 3.1 ± 2.6 | 3.5 ± 2.4 | 22.0 ± 17.9 | 29.8 ± 50.1 |

**Table 12c**: KT concentration in deep muscle tissue (ng/mg)

| Time (hours) | KT-Tfs gel 17 mg (n=4) | KT-Tfs sol. 17 mg (n=4) | KT-Tfs gel 50 mg (n=4) | KT-Tfs sol. 50 mg (n=4) |
|---|---|---|---|---|
| 0 | 0.0 ± 0.0 | 0.0 ± 0.0 | 0.0 ± 0.1 | 0.0 ± 0.0 |
| 1 | 36.0 ± 49.1 | 14.7 ± 1.5 | 24.8 ± 19.0 | 24.5 ± 44.7 |
| 2 | 19.4 ± 23.5 | 0.8 ± 0.3 | 18.8 ± 21.5 | 4.5 ± 4.0 |

(continued)

| Time (hours) | KT-Tfs gel 17 mg (n=4) | KT-Tfs sol. 17 mg (n=4) | KT-Tfs gel 50 mg (n=4) | KT-Tfs sol. 50 mg (n=4) |
|---|---|---|---|---|
| 3 | 2.4 ± 2.6 | 9.2 ± 3.1 | 49.8 ± 71.8 | 25.4 ± 43.0 |
| 5 | 13.5 ± 8.8 | 9.3 ± 12.5 | 49.9 ± 65.0 | 46.6 ± 85.6 |
| 8 | 2.4 ± 1.4 | 6.4 ± 2.4 | 30.2 ± 28.7 | 15.6 ± 23.4 |

**Claims**

**1.** Preparation based on a combination of at least one first (membrane forming component MFC), at least one second (membrane destabilising component MDC), and at least one third (membrane destabilising component MDC) amphipatic component suspended in a suitable liquid medium in the form of corresponding mixed amphipat aggregates with extended surface (ESAs) with one or a few mixed amphipat coating(s), which are preferably bilayer like, wherein said ESAs formed by a combination of all three said components have surfaces in contact with said liquid medium that are at least 50% more extended, on the average, than the typical surfaces of aggregates comprising the said at least one second and at least one third amphipatic component alone, at the same concentrations and, in case, after adjustment for the physico-chemical effects of resulting from the absence of said first amphipatic compound (MFC)
for application, administration or transport of an active ingredient, which can be one of the three amphipatic components, especially for biological, medical, immunological, or cosmetic purposes, into and through the pores in semi-permeable barriers or other constrictions, such as through the skin of warm blood creatures or the like.

**2.** A combination of at least one first (membrane forming component MFC), at least one second (membrane destabilising component MDC), and at least one third (membrane destabilising component MDC) amphipatic component suspended in a suitable liquid medium in the form of mixed amphipat aggregates with extended surface (ESAs) with one or a few mixed amphipat coating(s), which are preferably bilayer like, wherein the

- said at least one first substance has a tendency to self aggregate and is at least 10-times less soluble in said liquid medium than said at least one second and said one third substance, allowing the first to form extended surfaces,
- said at least one second substance is at least 10-times more soluble than said at least one first substance in said liquid medium and, on its own, tends to form or supports the formation of surfaces that are at least 2-times less extended than the surfaces containing the at least one first substance alone,
- said at least one third substance being also at least 10-times more soluble in said liquid medium than the first substance and optionally forms self-aggregates with aggregation number at least 10-times smaller than that of self-aggregates of said first substance; and
- said extended surfaces comprising said at least one first, at least one second and at last one third substance, in equilibrium, have at least 50% greater surface than the surfaces formed by the at least one second or one third substance alone, at the same concentration and, in case, after adjustment for the physico-chemical effects of the absence of said first amphipatic compound (MFC)
for a preparation for application, administration or transport of at least one active ingredient, which can be one of the three amphipatic components, especially for medicinal or biological purposes, into and through barriers and constrictions, such as the skin of warm blood creatures or the like.

**3.** Extended-surface aggregates (ESAs) comprising at least one first (membrane forming component, MFC), at least one second (membrane destabilising component, MDC), and at least one third (membrane destabilising component, MDC), all of which are amphipatic, suspended in a suitable liquid medium, which permits said ESAs to permeate barriers with the pores with at least 40% smaller radius than the average ESAs radius, as measured after the ESAs have permeated the barrier pores and assuming spherical ESAs geometry.

**4.** Preparation based on a combination of at least one first (membrane forming component MFC), at least one second (membrane destabilising component MDC), and at least one third (membrane destabilising component MDC) amphipatic component suspended in a suitable liquid medium in the form of corresponding mixed amphipat aggregates

with an extended surface (ESAs) with one or a few, preferably bilayer-like, mixed amphipat coating(s), wherein said MFC alone forms extended-surface aggregates with aggregation number of at least 5000, and preferably more than 10.000, and both MDCs_alone and the combination of both MDCs form smaller aggregates with no substantially extended surface and aggregation number below 5000, and preferably below 1000 in contact with said suitable liquid medium.

**5.** A combination according to claims 1 to 4,
wherein the said extended surfaces are in the form of membrane surfaces.

**6.** A combination according to any of preceding claims,
wherein the said at least one second substance increases the flexibility of extended surfaces comprising said at least one first, at least one second, and at least one third substance in comparison with the surfaces formed merely by an at least one first substance or else with the surfaces formed by at least one first and at least one third substance.

**7.** A combination according any of preceding claims,
wherein the said at least one second and one third substance together increase the permeability of extended surfaces containing the said at least one first, at least one second, and at least one third substance, in comparison with the surfaces formed merely by the at least one first substance or else with the surfaces formed by at least one first and at least one third substance.

**8.** A combination according to any of preceding claims,
wherein the said at least one second substance or the said at least one third substance increases the ability to tolerate high curvature, as assessed by relative stability of said extended surface comprising said one first, said one second and said one third substance against enforced higher curvature during passing through a constriction with maximum diameter at least 1.4 times smaller than the average diameter of an extended surface formed by an at least one first substance alone.

**9.** A combination according to any of preceding claims,
wherein the at least one first substance and the at least one second substance or the at least one third substance differ in solubility on the average at least 10-fold.

**10.** A combination according to any of preceding claims,
wherein the at least one second substance and the at least one third substance differ in solubility on the average at least 2-fold.

**11.** A combination according to any of preceding claims,
wherein the at least one second substance or the at least one third substance have the hydrophilicity-lipophilicity ratio between 10 and 20.

**12.** A combination according to any of preceding claims,
wherein the concentration of said at least one second substance used in the combination with said one first and said one third substance is below 80% of the concentration that would be needed to render the aggregates comprising only said one first and said one second substance as adaptable to ambient stress as the selected combination of all at least three substances, whereby the said one second and said one third substance can exchange roles.

**13.** A combination according to any of preceding claims,
wherein the concentration of said at least one second substance or of said at least one third substance, as the case may be, amounts to at least 0.1 % of the relative concentration as defined in claim 8.

**14.** A combination according to any of preceding claims,
wherein the concentration of said at least one second or of said at least one third substance amounts to 1 - 80% of the relative concentration as defined in claim 8.

**15.** The combination according to any of preceding claim,
wherein relative concentration of said at least one third substance used in combination with said one first and said one second substance is above 0.1 % of maximum possible concentration of the said at least one third substance in the system,

a) as defined in terms of the solubility of said third substance in the system or in said at least three-component aggregates, or else
b) as determined by the negative action of said at least one third substance on the stability of said at least three-component aggregates,
whereby the said one third and one second substance can also exchange roles.

**16.** The combination according to any of preceding claims,
wherein relative concentration of said at least one third substance used in combination with said one first and with said one second substance is between 1% and 99% of maximum possible concentration of said at least one third substance,

a) as defined in terms of the solubility of said third substance in the system or in said at least three-component aggregates,
b) or else as determined by the detrimental effect of said at least one third substance on the stability of said at least three-component aggregates,
whereby the said one third and one second substance can also exchange roles.

**17.** The combination according to any of preceding claims,
wherein relative concentration of said at least one third substance used in combination with the said one first and the said one second substance is between 10% and 95% of the maximum possible concentration of said at least one third substance, as defined in terms of the third substance solubility in the system or in said aggregates, or else as determined by the detrimental effect of said at least one third substance on the stability of said at least three-component aggregates, whereby the said one third and one second substance can also exchange roles.

**18.** The combination according to any of preceding claims,
wherein relative concentration of said at least one third substance used in combination with the said one first and the said one second substance is between 25% and 90% of the maximum possible concentration of said at least one third substance, as defined in terms of the third substance solubility in the system or in said aggregates, or else as determined by the detrimental effect of said at least one third substance on the stability of said at least three-component aggregates, whereby the said one third and one second substance can also exchange roles.

**19.** The combination according to any of preceding claims,
wherein the total dry mass of all at least three amphipatic substances, which together form highly adaptable aggregates with an extended surface, is between 0.01 weight-% and 50 weight-%.

**20.** The combination according to any of preceding claims,
wherein total dry mass of all at least three substances, which together form highly adaptable aggregates with an extended surface, is between 0.5 weight-% and 30 weight-%.

**21.** The combination according to any of preceding claims,
wherein total dry mass of all at least three substances, which together form highly adaptable aggregates with an extended surface, is between 1 weight-% and 15 weight-%.

**22.** A combination according to any of preceding claims,
wherein the extended surfaces with a high adaptability, which contain said at least three substances, have an average curvature corresponding to an average radius between 15 nm and 5000 nm.

**23.** A combination according to any of preceding claims,
wherein the extended surfaces with a high adaptability, which contain said at least three substances, have an average curvature corresponding to an average radius between 30 nm and 1000 nm.

**24.** A combination according to any of preceding claims,
wherein the extended surfaces with a high adaptability, which contain said at least three substances, have an average curvature corresponding to an average radius between 40 nm and 300 nm.

**25.** A combination according to any of preceding claims,
wherein the extended surfaces with a high adaptability, which contain said at least three substances, have an average curvature corresponding to an average radius between 50 nm and 150 nm.

**26.** The combination of substances according to any of preceding claims,
wherein the concentration and the composition of the electrolyte in which the extended surfaces with at least one first, at least one second, and at least one third substance are suspended, and which comprises mono and/or oligovalent ions, is chosen to have ionic strength between I = 0.001 and I = 1.

**27.** The combination of substances according to any of preceding claims,
wherein the concentration and the composition of the electrolyte, in which the extended surfaces with at least one first, at least one second, and at least one third substance are suspended, and which comprise mono and/or oligovalent ions, is chosen to have pH value

a) in the vicinity of the logarithm of the apparent ionisation constant (*p*Ka) of said at least one second substance, if the latter is mono-ionizable, or in the vicinity of such pKa value that maximises the solubility of said at least one second substance, if the latter has several ionizable groups, or else
b) in the vicinity of pH optimum for the most rapidly decaying or the otherwise most sensitive amongst the said at least three substances, if the said at least one second substance is not ionizable.

**28.** The combination of substances according to any of preceding claims,
wherein the *p*H value of the polar medium in which the ESAs comprising at least one first, at least one second, and at least one third substance are suspended is between *p*H = *p*Ka - 3 and *p*H = *p*Ka + 3.

**29.** The combination according to any of preceding claims,
wherein the at least one first substance being less soluble in the liquid medium, and/or being the surface-building substance in the system, is a lipid, whereas the at least one second substance being more soluble in the liquid medium and/or increasing the tolerable surface curvature or adaptability of said extended surface, is a membrane destabilising amphipat, which is typically a surfactant, and said at least one third substance is either a biologically active amphipatic ingredient, which has a capability of its own to increase the tolerable surface curvature, or adaptability of said extended surface, or else is a different surfactant different from the said at least second substance.

**30.** The combination according to any of preceding claims,
wherein the molecules are arranged in the form of minute fluid droplets suspended or dispersed in a liquid medium and surrounded by a coating of one or several layers of the at least one first substance, which is capable of self-aggregation, and of at least one second substance and of at least one third substance, which are both amphipatic, such that

a) the former substance and the latter two substances differ in solubility in a suitable liquid medium at least 10-fold, or such that
b) the average radius of homo-aggregates of the more soluble amongst the at least one second and third substance or of hetero-aggregates of the at least one first, the at least one second and the at least one third substance is smaller than the average radius of homo-aggregates of said at least one first substance, which is the least soluble amongst the three.

**31.** Combination according to any of preceding claims,
wherein the at least one first substance is a polar or a non-polar, surface - forming lipid.

**32.** Combination according to any of preceding claims, wherein the at least one first substance in extended surfaces is capable of forming bilayer membranes and preferably forms bilayers on its own.

**33.** Combination according to any of preceding claims,
wherein the solubility of the at least one first substance in a polar liquid medium is between $10^{-12}$ M and $10^{-7}$ M.

**34.** Combination according to any of preceding claims,
wherein the at least one first substance forming extended surfaces is selected from the group comprising lipids, lipoids from a biological source, corresponding synthetic lipids, or modifications thereof.

**35.** Combination according to any of preceding claims,
wherein said at least one first substance forming extended surfaces is selected from the group comprising glycerides, glycolipids, glycerophospholipids, isoprenoidlipids, sphingolipids, steroids, sterines or sterols, sulphur-containing lipids, lipids containing at least one carbohydrate residue, or other polar fatty derivatives.

**36.** Combination according to any of preceding claims,
wherein said at least one first substance forming extended surfaces is selected from the group comprising phosphatidylcholines, phosphatidylethanolamines, phosphatidylglycerols, phosphatidylinositols, phosphatidic acids, phosphatidylserines, sphingomyelins, sphingophospholipids, glycosphingolipids, cerebrosides, ceramidpolyhexosides, sulphatides, sphingoplasmalogenes, or gangliosides.

**37.** Combination according to any of preceding claims,
wherein said extended surface-forming substance is selected from the group comprising lipids with one or two, not necessarily identical, fatty chains, especially with acyl-, alkanoyl-, alkyl-, alkylene-, alkenoyl-, alkoxy, or chains with omega-cyclohexyl-, cyclo-propane-, iso- or anteiso-branched segments, or the corresponding chains mixtures.

**38.** Combination according to any of preceding claims,
wherein said substance that forms extended surfaces is selected from the group comprising lipids with *n*-decyl, *n*-dodecyl (lauryl), *n*-tetradecyl (myristyl), *n*-hexadecyl (cetyl), *n*-octadecyl (stearyl), *n*-eicosyl (arachinyl), *n*-docosyl (behenyl) or *n*-tetracosyl (lignoceryl), 9-*cis*-dodecenyl (lauroleyl), 9-*cis*-tetradecenyl (myristoleyl), 9-*cis*-hexadecenyl (palmitoleinyl), 9-*cis*-octadecenyl (petroselinyl), 6-*trans*-octadecenyl (petroselaidinylj, 9-*cis*-octadecenyl (oleyl), 9-*trans*-octadecenyl (elaidinyl), 9-*cis*-eicosenyl (gadoleinyl), 9-*cis*-docosenyl (cetoleinyl) or -9-*cis*-tetracosoyl (nervonyl), *n*-decyloxy, *n*-dodecyloxy (lauryloxy), *n*-tetradecyloxy (myristyloxy), *n*-hexadecyloxy (cetyloxy), *n*-octadecyloxy (stearyloxy), *n*-eicosyloxy (arachinyloxy), *n*-docosoyloxy (behenyloxy) or *n*-tetracosoyloxy (lignoceryloxy), 9-*cis*-dodecenyloxy (lauroleyloxy), 9-*cis*-tetradecenyloxy (myristoleyloxy), 9-*cis*-hexadecenyloxy (palmitoleinyloxy), 6-*cis*-octadecenyloxy (petroselinyloxy), 6-*trans*-octadecenyloxy (petroselaidinyloxy), 9-*cis*-octadecenyloxy (oleyloxy), 9-*trans*-octadecenyloxy (elaidinyloxy), and 9-*cis*-eicosenyl (gadoleinyloxy), 9-*cis*-docosenyl (cetoleinyloxy) or 9-*cis*-tetracosoyl (nervonyloxy), *n*-decanoyloxy, *n*-dodecanoyloxy (lauroyloxy), *n*-tetradecanoyloxy (myristoyloxy), *n*-hexadecanoyloxy (palmitoyloxy) *n*-octadecanoyloxy (stearoyloxy), *n*-eicosanoyloxy (arachinoyloxy), *n*-*n*-docosoanyloxy (behenoyloxy) and *n*-tetracosanoyloxy (lignoceroyloxy), 9-*cis*-dodecenyloxy (lauroleoyloxy), 9-*cis*-tetradecenoyloxy (myristoleoyloxy), 9-*cis*-hexadecenoyloxy (palmitoleinoyloxy), 6-*cis*-octadecenoyloxy (petroselinoyloxy), 6-*trans*-octadecenoyloxy (petroselaidinoyloxy), 9-*cis*-octadecenoyloxy (oleoyloxy) , 9-*trans*-octadecenoyloxyelaidinoyloxy), and 9-*cis*-eicosenoyloxy (gadoleinoyloxy), 9-*cis*-docosenoyloxy (cetoleinoyloxy) and 9-*cis*-tetracosenoyloxy (nervonoyloxy) or the corresponding sphingosine derivative chains.

**39.** Combination according to any of preceding claims,
wherein said at least one second substance is a surfactant.

**40.** Combination according to any of preceding claims,
wherein said surfactant is selected from the group comprising nonionic, zwitterionic, anionic and cationic surfactants.

**41.** Combination according to any of preceding claims,
wherein said surfactant has the solubility in a polar liquid in which the extended surfaces are prepared between $10^{-6}$ M and $10^{-2}$ M.

**42.** Combination according to any of preceding claims,
wherein said surfactant is selected from the group comprising long-chain fatty acids or long chain fatty alcohols, long chain fatty ammonium salts, such as alkyl- or alkenoyl-trimethyl-, -dimethyl- and -methyl-ammonium salts, alkyl- or alkenoyl-sulphate salts, or monovalent salts of cholate, deoxycholate, glycocholate, glycodeoxycholate, taurodeoxycholate, taurocholate, acyl- or alkenoyl-dimethyl-aminoxides, long fatty chain, for example alkanoyl, dimethyl-aminoxides and especially dodecyl dimethyl-aminoxide, long fatty chain, for example alkyl-N-, methylglucamides and alkanoyl-N-methylglucamides, long fatty chain-N,N-dimethylglycines, for example N-alkyl-N,N-dimethylglycines, 3-(long fatty chain-dimethylammonio)-alkanesulphonates, for example 3-(acyldimethylammonio)-alkanesulphonates, long fatty chain derivatives of sulphosuccinate salts, long fatty chain-sulphobetaines, for example N-acyl-sulphobetaines, long fatty chain betaines, polyethylen-glycol-acylphenyl ethers, polyethylene-long fatty chain-ethers such as polyethylene-acyl ethers, polyethyleneglycol-iso long fatty chain ethers, such as polyethyleneglycol-isoacyl ethers, polyethyleneglycol-sorbitane-long fatty chain esters, for example polyethyleneglycol-sorbitane-acyl esters and especially polyethylenglykol-monolaurate (e.g. Tween 20), polyethylenglykol-sorbitan-monooleate (e.g. Tween 80), polyhydroxyethylene-long fatty chain ethers, for example polyhydroxyethylene-acyl ethers (Brij series), or the corresponding polyhydroxyethylene-acyl esters (Myrj series) and polyethoxylated castor oil 40 (Cremophor EL), sorbitane-mono long fatty chain, for example alkylate (Arlacel or Span series), long fatty chain -N-methylglucamides, such as acyl-N-methylglucamides or alkanoyl-N-methylglucamides, long fatty chain sulphates, for example alkyl-sulphates and their salts; long fatty chain thioglucosides, such as alkylthioglucosides, long fatty

chain derivatives of various carbohydrates, such as pentoses, hexoses and disaccharides, especially alkyl-glucosides and maltosides; further lysolipids, such as long fatty chains derivatives of common phospholipids, especially lysoglycerophosphatidylcholine (= lysolecithin), lysoglycerophosphatidylethanolamine (lysokephalin), lyso-glycerophosphatidic acid, lyso-glycerophosphorylglycerol, lyso-glycerophosphorylserine, corresponding short-chain phospholipids, and membrane destabilising oligo or polypeptides.

**43.** Combination according to any of preceding claims,
wherein the at least one second substance is charged if the at least one third substance is uncharged, and the at least one second substance is uncharged if the at least one third substance is charged, similar preferred combinations also being possible for the said at least one first and one second or for the said at least one first and one third substance.

**44.** Combination according to any of preceding claims,
wherein the surface, formed by the at least one first, one second and one third substance, at least one of which is charged, contains between 1% and 75% of the charged component.

**45.** Combination according to any of preceding claims,
wherein the surface, formed by the at least one first, one second and one third substance, at least one of which is charged, contains between 5% and 50% of the charged component.

**46.** Combination according to any of preceding claims,
wherein the surface, formed by the at least one first, one second and one third substance, at least one of which is charged, contains between 10% and 30% of the charged component.

**47.** Combination according to any of previous claims,
wherein the surface-supporting at least one first substance is a phosphatidylcholine, a phosphatidylethanolamine-N-mono- or N-di-methyl, phosphatidic acid or its methyl ester, phosphatidylserine and/or phosphatidylglycerol and the at least one second substance which on its own forms small aggregates is a lysophospholipid, especially a lysophosphatidic acid, lysomethylphosphatidic acid, lysophosphatidylglycerol, lysophosphatidyleholine, a partially N-methylated lysophosphatidylethanolamine, a monovalent salt of cholate, deoxycholate, glycocholate, glycodeoxycholate, taurocholate, or a sufficiently polar sterol derivative, a laurate, myristate, palmitate, oleate, palmitoleate, elaidate or other long-chain fatty acid salt and/or a Tween-, a Myrj-, or a Brij-surfactant, or a Triton, a long-chain fatty sulphonate, -sulphobetaine, -N-glucamide or - sorbitane (Arlacel or Span) surfactant.

**48.** Combination according to any of preceding claims,
wherein the at least one third substance, if not a surfactant different from the at least one second substance, but otherwise selected from similar surfactant classes, is a biologically active amphipat which can destabilise lipid membranes.

**49.** Combination according to any of preceding claims,
wherein the solubility of at least one third or of one second substance in a polar liquid is between 5 $x10^{-6}$ M and 1 M.

**50.** Combination according to any of preceding claims,
wherein the at least one third amphipat or the at least one second amphipat adsorbs to the surface of lipid bilayer membrane but is well miscible with or soluble in the polar liquid in which the said extended surfaces are formed.

**51.** Combination according to any of previous claims,
wherein the at least one third or one second substance is a drug.

**52.** Combination according to any of preceding claims,
wherein the amphipatic compound with biological activity, which can act as a drug, is a substituted ammonium compound of the formula

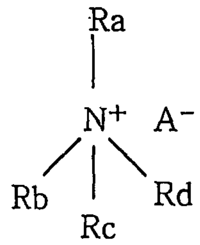

(1)

in which

a) Ra represents a hydrophobic group, and Rb, Rc, and Rd, independently of one another, each represents hydrogen, Cl-C4-alkyl, 2-hydroxyethyl, allyl or cycle-C3-C6-alkyl-Cl-C3-alkyl, or two of the radicals Rb, Rc and Rd together represent C4- or C5- alkylene interrupted by -HN-, -N(C1-C4-alkyl)-, -N(2-hydroxyethyl)- or by oxygen, or;
b) Ra and Rb are two hydrophobic groups or together represent a hydrophobic group, and Rc and Rd, independently of one another, each represents hydrogen, C1-C4-alkyl, allyl or cyclo-C3-C6-alkyl-C1-C3-alkyl, or
c) Ra, Rb and Rc together represent a hydrophobic group, and Rd represents hydrogen or C1-C4-alkyl, and $A^-$ represents the anion of a pharmaceutically acceptable acid, as a carboxylic acid salt of the formula

$$Ra\text{-}COO^- \, Y^+ \qquad (2)$$

in which Ra represents a hydrophobic group, and $Y^+$ represents the cation of a pharmaceutically acceptable base, as an alpha-amino acid compound of the formula

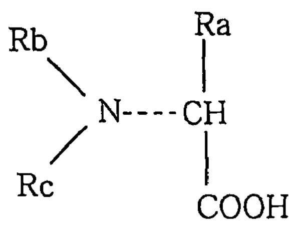

(3)

in which Ra represents a hydrophobic group, and Rb and Rc, independently of one another, each represents hydrogen or C1-C4-alkyl, as a phosphoric acid monoester of the formula

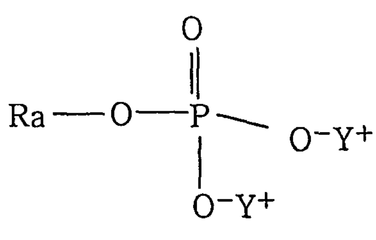

(4)

in which Ra represents a hydrophobic group and $Y^+$ represents the cation of a pharmaceutically acceptable base, or as an acid addition salt of a compound having a hydrophobic group Ra and an imidazoline, imidazolidine or hydrazino group as hydrophilic group.

**53.** Combination according to any of preceding claims, wherein the said at least one third or one second amphipatic substance with biological activity, which can act as a drug, is a substituted ammonium compound of the formula 1 in which

a) the hydrophobic group can be an aliphatic hydrocarbon radical that can be interrupted by an oxygen or sulphur atom, may contain the groups -CO(=O)-, -O-C(=O)-, -C(=O)-NH-, -O-C(=O)-NH- or hydroxy, and can be substituted by from 1 to 3 monocyclic, aliphatic or aromatic hydrocarbon radicals, by a bi- or tri-cyclic, aromatic or partially saturated hydrocarbon radical, by a monocyclic, aromatic, partially saturated or saturated heterocycle or by a bi- or tri-cyclic, aromatic, partially saturated or benzo-fused heterocycle, or can be a monocyclic, aliphatic or aromatic hydrocarbon radical or a bicyclic, aliphatic or benzo-fused hydrocarbon radical, and the hydrophilic group is a group of the formula

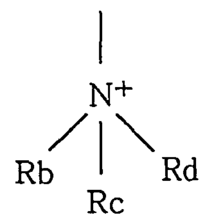

in which Rb, Rc, and Rd, independently of one another, each represents hydrogen, C1-C4-hydrogen, C1-C4-alkyl or 2-hydroxyethyl, or in which two of the radicals Rb, Rc and Rd together represent piperidino, piperazinyl, 1-methylpiperazinyl, 1-(2-hydroxyethylpiperazinyl or morpholino, and the other radical represents hydrogen, or,
b) the hydrophobic groups Ra and Rb can be two aliphatic hydrocarbon radicals which can be substituted by one or two monocyclic, aliphatic or aromatic hydrocarbon radicals or by substituted, monocyclic, aromatic, partially saturated or saturated heterocycle, or Ra and Rb together represent a monocyclic, aromatic, saturated, partially saturated or benzo-fused heterocycle, and the hydrophilic group is a group of the formula

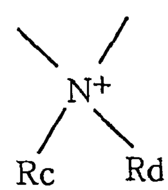

in which Rc and Rd, independently of one another each represents hydrogen or C1-C4-alkyl, or
c) the hydrophobic group is formed by Ra, Rb and Rc together and represents an aromatic, partially saturated or benzo-fused heterocycle and the hydrophilic group is a group of the formula

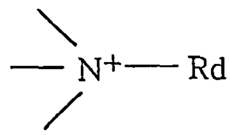

in which Rd represents hydrogen or C1-C4-alkyl, preferably methyl, and $A^-$ is the anion of a pharmaceutically acceptable acid, or a carboxylic acid salt of the formula 2 in which the hydrophobic group Ra can be an aliphatic hydrocarbon radical, which can be substituted by a monocyclic, aromatic hydrocarbon radical, or by a bi- or tri-cyclic, aromatic or partially saturated hydrocarbon radical, by a monocyclic, aromatic or partially saturated heterocycle or by a bi- or tri-cyclic, aromatic, partially saturated or benzo-fused heterocycle or by a steroid radical, or Ra can be a monocyclic, aromatic hydrocarbon radical, a bi- or tri-cyclic, aromatic or partially saturated hydrocarbon radical, a monocyclic, aromatic or partially saturated heterocycle or a bi- or tri-cyclic, aromatic, partially saturated or benzo-fused heterocycle, and $Y^+$ is the cation of a pharmaceutically acceptable base.

**54.** Combination according to any of preceding claims,
wherein the said at least one third or one second amphipatic substance, which acts as a drug, is a substituted ammonium compound or the corresponding amino compound that can be converted into the ammonium compound by salt formation, such as acetylcholine chloride, methacholine chloride, carbachol, muscarine, pilocarpine, arecoline, phyostigmine, neostigmine, pyridostigmine bromide, serotonin, histamine, tryptamine, bufotenine, psilocybin, morphine, hydromorphone, oxymorphone, levorphanol, codeine, hydrocodone, oxycodone, nalorphine, naloxone, naltrexon, buprenophine, butorphanol, nalbiphine, pholcodine, pentazocine, ketamine, metazocine, pentazocine, cyclazocine, pethidine, cetobemidon, alphaphrodine, ethoheptazine, prodilidine, profadol, methadone, normethadone, isomethadone, dipipanone, phenadoxone, dimephethanol, dextromoramide, D-propoxyphene, 1-benzyl-2-dimethylaminomethyl-1-propanoyloxytetralin, tramadol, dimethylthiambutene, diampromide, phenampromide, propiram, tilidine, metopholine, etonitazene, ergotamine, dihydroergotamine, dihydroergocryptine, methysergide, lisuride, dimetotiazin, dizotifen, oxetoron, cyproheptadine, procaine, chloroprocaine, hydroxyprocaine, propoxycaine I oxy-buprocaine, propoxymetacaine, piridocaine, leucinocaine, butacaine p tetracaine, hydroxytetracaine, cornecaine, edan, piperocaine, cyclomethycaine, parethoxysaine, stadacain, cinchocaine, lidocaine, pyrrocaine, granocaine, butanilicaine, tolycaine, mepivacaine, bupivacaine, prilocaine, carticaine, dipiperidon, propicocaine, dyclonine, pramocaine, fomocaine, quinisocaine, profenamine, promethazine, periciazine, perimethazine, chlorpromazine, perphenazine, prochlorperazine, triflumpromazine, trifluoperazine, fluphenazine, thioridazine, mesoridazine, piperacetazine, acetophenazine, ethymemazine, dimethacrine, opipramol, clomipramine, imipramine, desimipramine, trimipramine, chloroprothixene, thiothixene, amitriptyline, nortriptyline, doxepin, thiepin, protriptyline, prothipendyl, femoxetin, citalopram, zimelidine, trebenzomin, viloxazine, nomifensine, femoxetin, tranylcygromine, pargyline, etryptamine, flurazepam, mescaline, Nalpha,Nalpha-dimethyl-tryptamine, bufotenine, psilocin, psilocylein, scopolamine, atropine, benzatropine, trihexyphenidyl, cycrimine, pridinol, biperidine, procyclidine, caramiphene, phenglutarimide, orphenadrine, chlor-phenoxamine, metixen, doxapram, amphetamine, methamphetamine, propylhexedrine, prolintane, fencamfamine, methylphenidol, pipradrol, phenmetrazine, diethylpropion, meclofenoxat, naftidrofuryl, dexamphetamine, phentermin, chlorphentermine, fenfluramine, amfepramone, phenmetrazine, phendimetrazine, tubocumarin, alcuronium chloride, gallamin triethiodide, hexacarbacholine bromide, pancuronium bromide, suxamethonium chloride, decamethonium bromide, scopolamine butyl bromide, bevonium methyl sulphate, valethamate bromide, methanteline bromide, camylofine, hexahydroadiphenine, adiphenine, fencarbamide, benzyclamine, ditaxol, chloroquine, tamoxifen, ethamoxytriphetol, phenbenzamine, tripelenamin, chlorpyramine, mepyramine, metaphenilene, metapyrilene, chloropyrilene, histpyrroclin, bamipin, thenalidine, clemizole, meth-dilazine, isothipendyl, oxomenazine, diphenhydramine, medrylamine, chlorophenoxamine, silachlorophenoxamin, carbinoxamine, diphenpyraline, clemastine, ametho-benzepine, pheniramine, chlorophenamine, bromo-pheniramine, triprolidine, cycliramine, phenindamine, dimetindene, cyproheptadine, ketotifen, epinephrine (adrenaline), norepinephrine (noradrenaline), dopamine, nordefrin, ethylnorepinephrine, isoprenaline, iso--ethorine, metaproterenol, orciprenaline, metaraminol, phenylephrine, hydroxyamphetamine, methoxyphenamine, methoxamine, albuterol, ephedrine, norephedrine, fenfluramine, phenylpropanolamine, pholedrine, tyramine, dichloroisoprenaline, norfenefrine, octopamine, etilefrin, acebutolol, atenolol, meto-prolol, toliprolol, alprenolol, oxprenolol, bunitrolol, bupranolol, talinolol, phenbutolol, bufetolol, varbian (R,S- or S-form), propanolol, indenolol, pindolol, mepindolol, nadolol, bunolol, sofalol, nifenalol, cabetalol, bufenalol, reserpine, rescinnamine, syringopine, chlorotetracycline, oxytetracycline, tetracycline, demethylchlorotetracycline, metacycline, doxycycline, minocycline, rolitetracycline, quinine, conquinidine, quinidine, cinchonine, pamaquine, prlmaquine, pentaquine, chloroquine, santoquine, hydroxychloroquine, amodiaquine, mepacrin, biguanid-1,3,5-triazin, proguanil, bromoguanil, chloroproguanil, nitroguanil, cycloguanilembonate, pyrimethamine, tri-methoprim, lucanthone, hycanthone, miracil A or B, amantadine, cyclooctylamine, rimantadin, prednisolone diethylaminoacetate.

**55.** Combination according to any of preceding claims,
wherein the said at least one third or one second amphipatic substance takes the role of a drug as the substituted ammonium compound or as the corresponding amino compound that can be converted into the ammonium compound by salt formation, and is a compound selected from the group of the acid addition salts of antidepressants of the formula in which R1 represents lower alkyl, for example methyl, A represents the group N-υ R1, oxygen or sulphur, and R2 represents hydrogen or cyano; acid addition salts of antidepressants of the formula

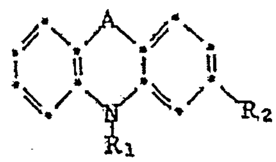

(1.10)

in which R1 represents lower alkylamino-lower alkyl, for example 3-methylamino-n-propyl, di-lower alkyl-amino-lower alkyl, for example 3-dimethylamino-n-propyl or 3-(4-(2-hydroxyethyl)-piperazin-1-yl)-n-propyl and A represents ethylene or vinylene, or acid addition salts of amphetamine, methamphetamine, benzphetamine, propyl-hexedrine, prolintan, fencamfin, methylphenidate, pipradrol, phenmetrazine, adiphenine, epinephrine, norepinephrine, dopamine, nordefrin, ethyl-norepinephrine, isoprenaline, isoethorine, meta-proterenol, orciprenaline, metaraminol, phenylephrine, hydroxyamphetamine, methoxyphenamine, ephedrine, norephedrine, pholedrine, tyramine, norfenefrin, octopamine, acebutolol, atenolol, toliprolol, alprenolol, oxprenolol, bunitrolol, bupranolol, talinolol, phenbutolol, bufetolol, varbian (R,S-form and S-form), reserpine, rescinnamine, syringopine or prednisolone diethylaminoacetate.

**56.** Combination according to any of preceding claims,
wherein the said at least one third or second amphipatic substance takes the role of a drug as the substituted ammonium compound of the formula 1 or as the corresponding amino compound that can be converted into the ammonium compound by salt formation, 1-(2R-2-hydroxy-3-methylaminopropyl)dibenzo[b,e]bicyclo[2.2.2]octadiene, and the 2R,S-isomeric mixture, maprotiline, benzoctamine, 3-methyldibenzo[2,3:6,7]-oxepino[4,5-diazepine hydrochloride, 7-cyano-3-methyl-2,3,4,5- tetrahydro-1H-dibenzo[2,3:6,7]-thiepino[4,5-d]azepine methanesulphonate, 3,10-dimethyl-1,2,3,4,5,10-hexahydrodibenzo[b,f]azepino[4,5]azepine maleate, clomipramine, opipramol, desipramine, imipramine or imipramine N-oxide, ephedrine, norephedrine, 1-iso-propylamino-3-[4-(2-methylthioethoxy)-phenoxy]-propan-2-ol, 1-isopropylamino-3-(2-pyrrol-1-ylphenoxy)-propan-2-ol, oxprenolol, prenalterol, adiphenine, prednisolone diethylaminoacetate, or reserpine.

**57.** Combination according to any of preceding claims,
wherein the said at least one third or second amphipatic substance takes the role of a drug as the as the carboxylic acid salt or the carboxylic acid compound that can be converted into the carboxylic acid salt by salt formation, methylprednisolone sodium succinate, prednisolone sodium succinate, 3,20-dioxo-5β-pregnane, hydroxydione succinate sodium, 11,20-dioxo-3alpha-hydroxy- 5alpha-pregnane, alphadolon, a cholic acid or deoxycholic acid salt, alclofenac, ibufenac, ibuprofen, clindanac, fenclorac, ketoprofen, fenoprofen, indoprofen, fenclofenac, diclofenac, flurbiprofen, pirprofen, naproxan, benoxaprofen, carprofen, cicloprofen, mefenamic acid, flufenamic acid, tolfenamic acid, meclofenamic acid, milflumic acid, clonixin, flunixin, indometacin, oxmetacin, intrazol, acemetazin, cinmetacin, zomepirac, tolmetin, colpirac, tiaprofenic acid, benzadac, PGE2 (dinoprostone), PGF2alpha (dinoprost), 15 (S)-15-methyl-PGE2, 15 (S)-15-methyl-PGF2alpha (carboprost), (±)15 (Xi)-15-methyl-13,14-dihydro-11-deoxy-PGE1 (deprostil), 15 (S)-15-methyl-11-deoxy-PGE, (doxaprost), 16,16-dimethyl-PGE2, 17-phenyl-18,19,20-trinor-PGF2alpha, 16-phenoxy-17,18,19,20-tetranor-PGF2, or N-methylsulphonyl-16-phenoxy-17,18,19,20-tetranor-PGF2 alpha (sulproston), nalixidic acid, cinoxacin, oxolinic acid, pironidic acid, pipenidic acid, penicillin G or V, phenethicillin, propicillin, nafcillin, oxacillin, cloxacillin, dicloxacillin, flucloxacillin, cyclacillin, epicillin, mecillinam, methicillin, azlocillin, sulbenicillin, ticarcillin, mezlocillin, piperacillin, carindacillin, azidocillin, ciclazillin, cefaclor, cefuroxime, cefazlur, cephacetrile, cefazolin, cephalexin, cefadroxil, cephaloglycin, cefoxitin, cephaloridine, cephsulodin, cefotiam, ceftazidine, cefonicid, cefotaxime, cefmenoxime, ceftizoxime, cephalothin, cephradine, cefamandol, cephanone, cephapirin, cefroxadin, cefatrizine, cefazedonep ceftrixon, ceforanid, moxalactam, clavulanic acid, nocardicine A, sulbactam, aztreonam, thienamycin, chlorambucil or methotrexate.

**58.** Combination according to any of preceding claims,
wherein the said at least one third or second amphipatic substance, which takes to role of a drug, acts as an adrenocorticostatic , a β-adrenolytic, an androgen an antiandrogen, an antiparasitic, an anabolic, an anaesthetic, an analgesic, an analeptic, an antiallergic, an antiarrhythmic, an antiarterosclerotic, an antiasthmatic, a bronchospasmolytic, an antibiotic, an antidrepressive, an antipsychotic, an antidiabetic, an antidot, an antiemetic, an antiepileptic, an antifibrinolytic, an anticonvulsive, an anticholinergic, an enzyme, a coenzyme or corresponding inhibitor, an antihistaminic, an antihypertonic, a biological inhibitor of drug activity, an antihypotonic, an anticoagulant, an

antimycotic, an antimyasthenic, an agent against Morbus Parkinson or Morbus Alzheimer, an antiphlogistic, an antipyretic, an antirheumatic, an antiseptic, a respiratory analeptic or a respiratory stimulant, a broncholytic, a cardiotonic, a chemotherapeutic, a coronary dilatator, a cytostatic, a diuretic, a ganglium-blocker, a glucocorticoid, an antiflew agent, a haemostatic, a hypnotic, an immunoglobuline or its fragment, an immunologically active substance, a bioactive carbohydrate, a bioactive carbohydrate derivative, a contraceptive, an anti-migraine agent, a mineralo-corticoid, a morphine-antagonist, a muscle relaxant, a narcotic, a neurotherapeutic, a neuroleptic, a neurotransmitter or its antagonist, a small peptide, a small peptide derivative, an ophthalmic, a sympaticomimetic or a sympathicolytic, a para-sympaticomimetic or a para-sympathicolytic, a psoriasis drug, a neurodermitis drug, a mydriatic, a psychostimulant, a rhinologic, a sleep-inducing agent or its antagonist, a sedating agent, a spasmolytic, tuberculostatic, an urologic agent, a vasoconstrictor or vasodilatator, a virustatic, a wound-healing substance, or a combination of aforesaid agents.

**59.** Combination according to any of preceding claims,
wherein the drug content is between 0.1 rel.% and 60 rel.% compared to the total mass of all three said substances that form said extended surfaces.

**60.** Combination according to any of preceding claims,
wherein said at least one third or second substance is a low molecular weight immunomodulator.

**61.** Combination according to any of preceding claims,
wherein said at least one third or second substance is a bio-catalyst.

**62.** Combination according to any of preceding claims,
wherein said at least one third or second substance is a low molecular weight agonist or antagonist of some biological substance action.

**63.** Combination according to any of preceding claims,
wherein said at least one third or second substance is a co-enzyme.

**64.** Combination according to any of preceding claims,
wherein said at least one third or second substance is a hormone.

**65.** Combination according to any of preceding claims,
wherein said at least one third or second substance is a low to intermediate weight polypeptide with membrane destabilising properties.

**66.** Combination according to any of preceding claims,
wherein said at last one second substance is a cyclooxygenase or lipoxygenase inhibitor and at least one third substance is a non-ionic surfactant with solubility in 1-10 $\mu$M range that preferably belongs to the class of sorbitane-polyoxyethylene-alkyl or -alkylene esters or else is a polyoxyethylene-alkyl or -alkylene ether.

**67.** The use of a combination of substances according to any of preceding claims, in drug carriers, drug depots, or for other kind of medicinal or biological application by providing the extended surfaces in the form of membranes formed by the at least one first substance, the at least one second and the at least one third substance, which together surround miniature droplets, wherein the substance with biological activity, being a drug, is mainly associated with said droplet surface or else is mainly incorporated into the droplet and then carried by the droplet to the place where the drug is intended to act.

**68.** The use of a combination of substances according to any of preceding claims for the manufacture of a preparation for the transport of an active ingredient, which can be one of the three amphipatic components, especially for biological, medical, immunological, or cosmetic purposes, into and through the skin of warm blood creatures.

**69.** A method of preparing a combination according to any of preceding claims in the form of a formulation of a biologically, cosmetically and/or pharmaceutically active agent, comprising the steps of

- selecting the at least one first and the at least one second substance which together form extended surfaces, when in contact with said medium, such that said extended surfaces formed by the at least one first and the at least one second substance are more adaptable than the at least one first substance alone and the surfaces

formed by the at least one second substance alone form small aggregates; alternatively
- selecting the at least one first and the at least one third substance which together form extended surfaces, when in contact with said medium, such that said extended surfaces formed by the at least one first and the at least one third substance are more adaptable than the at least one first substance alone and the surfaces formed by the at least one third substance alone form small aggregates, if this substance self-aggregates; and
- generating said surface-forming combination from at least one first, at least one second, and at least one third substance, such that the surface of resulting at least three component combination is even more adaptable than the surface prepared from at least one first and one second substance alone or of the surfaces formed by the at least one first and one third substance alone, bringing the combination of at least two or all three said substances into suspension by means of controlled mechanical fragmentation, in the presence of or before being mixed with the at least one third substance, such that said third substance is incorporated at least partly in said extended surface formed by controlled mechanical fragmentation to obtain final preparation.

**70.** The method according to any of preceding claims,
wherein said means of controlled mechanical fragmentation includes on filtration, pressure change or mechanical homogenisation, shaking, stirring, or mixing.

**71.** The method according to any of preceding claims,
wherein the liquid medium suspension characteristics correspond to any one of claims 1 to 65.

**72.** The method according to any of preceding claims,
wherein said active agent is selected from the group comprising anti-diabetic agents, growth factors, immunomodulators, enzymes, recognition molecules, adrenocorticostatics, adrenolytics, androgens, antiandrogens, antiparasitics, anabolics, anaesthetics, analgesics, analeptics, antiallergics, antiarrhythmics, antiarterosclerotics, antiasthmatics, bronchospasmolytics, antibiotics, antidrepressiva, antipsychotics, antidots, antiemetics, antiepileptics, antifibrinolytics, anticonvulsiva, anticholinergics, enzyme, coenzymes or corresponding inhibitors, antihistaminics, antihypertonics, biological inhibitors of drug activity, antihypotonics, anticoagulants, antimycotics, antimyasthenics, agents against Morbus Parkinson or Morbus Alzheimer, antiphlogistics, antipyretics, antirheumatics, antiseptics, respiratory analeptics or respiratory stimulants, broncholytics, cardiotonics, chemotherapeutics, coronary dilatators, cytostatics, diuretics, ganglium-blockers, glucocorticoids, antiflew agents, haemostatics, hypnotics, immunologically active substances, contraceptives, anti-migraine agents, mineralo-corticoids, morphine-antagonists, muscle relaxants, narcotics, neurotherapeutics, neuroleptics, neurotransmitters or their antagonists, peptides, peptide derivatives, opthalmics, sympaticomimetics or sympathicolytics, para-sympaticomimetics or para-sympathicolytics, anti-psoriasis drugs, neurodermitis drugs, mydriatics, psychostimulants, rhinologics, sleep-inducing agents or their antagonists, sedating agents, spasmolytics, tuberculostatics, urologics, vasoconstrictors or vasodilatators, virustatics, wound-healing substances, or a combination of aforesaid agents.

**73.** The method according to any of preceding claims,
wherein said at least three amphiphilic substances are either used as such, or dissolved in a physiologically compatible polar fluid, comprising water or water-miscible fluids, or in a solvation-mediating agent, together with a polar solution.

**74.** The method according to any of preceding claims,
wherein the said polar solution contains at least one surfactant or surfactant-like amphipat, which destabilises bilayer membrane, and at least one more membrane destabilising, biologically active ingredient or an additional surfactant.

**75.** The method according to any of preceding claims,
wherein the formation of said surfaces is induced by substance addition into a fluid phase, evaporation from a reverse phase, by injection or dialysis, or with the aid of mechanical stress.

**76.** The method according to any of preceding claims,
wherein the formation of said surfaces is induced by filtration, the filtering material having pores diameters between 0.01 $\mu$m and 0.8 $\mu$m, the preferred choice of pore diameter being dependent on the desired final aggregate dimensions.

**77.** The method according to any of preceding claims,
wherein several filters are used sequentially or in parallel.

**78.** The method according to any of preceding claims, wherein said agents and carriers are made to associate, at least partly, after formation of said extended surfaces.

**79.** The method according to any of preceding claims, wherein said extended surfaces, with which the agent molecules are allowed to associate, are prepared just before the application of the formulation, if convenient from a suitable concentrate or a lyophylisate.

**80.** A container comprising the pharmaceutical composition based a combination of substances according to any preceding claim.

**81.** A package comprising at least one container comprising the pharmaceutical composition based on a combination of substances according to any preceding claims.

**82.** A method for generating a therapeutic effect on a warm blood creature by applying a pharmaceutical composition based on a combination of substances according to any of previous claims onto or into such living creature body.

**83.** The method according to any of preceding claims, wherein different administration volumes are selected to control the applied medicament dose and the outcome of therapeutic application.

**84.** The method according to any of preceding claims, wherein a suspension of drug-free aggregates is loaded with the drug to be associated therewith during the day prior to an administration, preferably 360 min, more preferably 60 min and even more preferably 30 min before administering the resulting formulation in or on the body.

**85.** The method of any of preceding claims, **characterised in that** at least one dose of the pharmaceutical composition with therapeutic activity is administered.

**86.** The method according to any of preceding claims, wherein the flux of penetrants that carry a drug through the various pores in a well-defined barrier is determined as a function of a suitable driving force or a pressure acting across the barrier and the data are then conveniently described by a characteristic curve which, in turn, is employed to optimise the formulation or application further.

**87.** The method according to any of preceding claims, wherein the characteristic, e.g. penetrability vs. pressure, curve is analysed in terms of eq. (*) or alike.

**88.** The combination of any of preceding claims, wherein the adaptability of extended surface aggregates comprising all three said amphipatic components exceeds by at least 20% or by at least twice the standard deviation of a typical measurement, whichever is smaller, the adaptability of the extended surface aggregates comprising the at least one first and the at least one second amphipatic component, used at the corresponding concentrations, or the adaptability of the extended surface comprising the at least one first and the at least one third amphipatic component, used at the corresponding concentrations, whichever is smaller.

**89.** The combination of any of preceding claims, wherein the adaptability of extended surface aggregates comprising all three said amphipatic components exceeds by at least 30% the adaptability of the extended surface aggregates comprising the at least one first and the at least one second amphipatic component, used at the corresponding concentrations, or the adaptability of the extended surface comprising the at least one first and the at least one third amphipatic component, used at the corresponding concentrations, whichever is smaller.

**90.** The combination of any of preceding claims, wherein the total concentration of said at least one second and said at least one third compound in the ESAs comprising all three said amphipatic components is equal to or less than the concentration of said at least one second compound in the ESAs comprising at least one first and at least one second compound and the corresponding concentration of the at least one first compound.

**91.** The combination of any of preceding claims, wherein the total concentration of said at least one second and said at least one third compound in the ESAs comprising all three said amphipatic components is equal to or less than the concentration of said at least one third compound in the ESAs comprising at least one first and at least one second compound at the corresponding concentration of the at least one first compound.

**92.** The combination of any of preceding claims, wherein the adaptability is expressed as the inverse value of the $p^*$ value corresponding to a predefined fraction of $P_{max}$-value, which is often selected around 60% and preferably is 57% of $P_{max}$-value.

**93.** Use of an additional at least third amphipatic component as at least second membrane destabilizing compound to increase the adaptability of ESAs that otherwise would comprise only two amphipatic compounds, one of which is membrane forming and one of which is the first membrane destabilising compound, to obtain the corresponding three-component ESAs, comprising one membrane forming and two membrane destabilising compounds, such that the latter kind of ESAs have higher adaptability than the former ESAs.

**94.** Use of the third amphipatic component according to claim 93 in a combination according to any of preceeding claims 1 to 93.

**95.** A suspension of extended surface aggregates in a liquid medium comprising:

at least one first amphipatic component;
at least one second amphipatic component;
at least one third amphipatic component;
the first amphipatic component being a membrane forming lipid component;
the second and third component being membrane destabilising components;

wherein the third component is a non-steroidal anti-inflammatory drug (NSAID); and
whereby the aggregates are capable of penetrating semi-permeable barriers with pores at least 50% smaller than the average aggregate diameter before the penetration without changing the aggregate diameter by more than 25%.

**96.** A suspension of extended surface aggregates in a liquid medium comprising:

at least one first amphipatic component;
at least one second amphipatic component;
at least one third amphipatic component;
the first amphipatic component being a membrane forming lipid component;
the second and third component being membrane destabilising components;

wherein the third component is a NSAID; and
whereby extended surface aggregate comprising the first and second (but not the third) component, or the first and third (but not the second) component, the second or third component being present at a relative concentration X with respect to the concentration of the first component, have a lower propensity to overcome barriers with pores at least 50% smaller than the average aggregate diameter before the pore crossing than the extended surface aggregates comprising the first, second and third component together, whereby the concentration of the combined second and third components is at or below the relative concentration X.

**97.** The suspension according to claims 95 or 96, said extended surface aggregates being membrane-enclosed, liquid-filled vesicles, said first component being a membrane-forming lipid, and said second and third components being membrane-destabilising components.

**98.** A suspension of extended surface aggregates in a liquid medium comprising:

at least one first amphipatic component;
at least one second amphipatic component;
at least one third amphipatic component;
the first amphipatic component being a membrane forming lipid component;
the second and third component being membrane destabilising components;

wherein the third component is a non-steroidal anti-inflammatory drug (NSAID); and
whereby the extended surface aggregates are capable of penetrating intact mammalian skin, thus increasing NSAID concentration in the skin and/or increasing the reach of NSAID distribution beyond the skin, in comparison with the result of the same NSAID application in a solution on the skin.

**99.** The suspension of claim 98, said extended surface aggregates being membrane-enclosed, liquid-filled vesicles, said first component being a membrane-forming lipid, and said second and third components being membrane-destabilising components.

**100.** The suspension of any preceeding claim, wherein the third (NSAID) component is ketoprofen, ibuprofen, diclofenac, indomethacin, naproxen or piroxicam.

**101.** The suspension of any preceeding claim, wherein the first component is selected from the group consisting of phosphatidylcholines, phosphatidylethanolamines, phosphatidylglycerols, phosphatidylinositols, phosphatidic acids, phosphatidylserines, sphingomyelins, sphingophospholipids, glycosphingolipids, cerebrosides, ceramidpolyhexosides, sulphatides, sphingoplasmalogenes, or gangliosides.

**102.** The suspension of claim 101, wherein the first component is a phosphatidylcholine of biological, preferably plant, origin, especially soy (bean), coconut, olive, safflower or sunflower, linseed, evening primrose, primrose, or castor oil, etc..

**103.** The suspension of any preceding claim, wherein the second component is a surfactant.

**104.** The suspension of claim 103, wherein the surfactant has a solubility in the liquid medium ranging from about 5 $x10^{-7}$ M to about $10^{-2}$ M.

**105.** The suspension of claim 103, wherein the surfactant has hydrophilicity-lipophilicity ratio (HLB) between 10 and 20, even better between 12 and 18 and most preferred between 13 and 17.

**106.** The suspension of claim 102, wherein the surfactant is selected from the group of nonionic surfactants, and preferably is a polyethyleneglycol-sorbitan-long fatty chain ester, from polyethyleneglycol-long fatty chain ester or -ether, a polyhydroxyethylen-long fatty chain ester or -ether, or a surfactant-like nonionic phospholipid.

**107.** The suspension of any preceding claim, wherein the first component is a phosphatidylcholine and the third (NSAID), component is ketoprofen, diclofenac ibuprofen, indomethacin, naproxen, or piroxicam.

**108.** The suspension of claim 13, wherein the second component is a non-ionic surfactant, preferably is a polyethyleneglycol-sorbitan-long fatty chain ester, polyethyleneglycol-long fatty chain ester or polyethyleneglycol-long fatty chain ether type, the polyethyleneglycol chain being potentially replaced by a polyhydroxyethylene polar group, or else is a surfactant-like nonionic phospholipid.

**109.** The suspension of any preceding claim, wherein the average aggregate diameter before the aggregates penetrate the pores is at least 40% larger than the average pore diameter.

**110.** The suspension of any preceding claim, wherein the first component and the second component differ in solubility in the liquid medium at least 10-fold, on the average.

**111.** The suspension any preceding claim, wherein the second component and the third component differ in solubility on the average at least 2-fold.

**112.** The suspension of any preceding claim, wherein the total dry mass of the at least three amphipatic components is between 0.01 weight-% and 50 weight-%.

**113.** The suspension according to any preceding claim, wherein the extended surfaces formed by the at least three components have an average curvature corresponding to an average diameter between 15 nm and 5000 nm.

**114.** The suspension according to any previous claim, wherein the at least one further membrane destabilising component is a lower aliphatic alcohol.

**115.** A pharmaceutical preparation comprising the suspension of claims 95 to 114.

**116.** A pharmaceutical preparation comprising a suspension of liquid-filled vesicles in an aqueous medium, the vesicles being enclosed by membranes formed from at least one lipid component and comprising at least two membrane

destabilising components,
whereby extended surface aggregate comprising the first and second (but not the third) component, or the first and third (but not the second) component, the second or third component being present at a relative concentration X with respect to the concentration of the first component, have a lower propensity to overcome barriers with pores at least 50% smaller than the average aggregate diameter before the pore crossing than the extended surface aggregates comprising the first, second and third component together, whereby the concentration of the combined second and third components is at or below the relative concentration X.

**117.** A pharmaceutical preparation comprising a suspension of liquid-filled vesicles in an aqueous medium, the vesicles being enclosed by membranes formed from at least one lipid component and comprising at least three membrane destabilising components, whereby the membrane destabilising components comprise a surfactant, a non-steroidal anti-inflammatory drug, and / or a lower aliphatic alcohol, whereby the membrane destabilising components increase the vesicle ability to penetrate mammalian skin and thus increase the reach of NSAID distribution in the skin, and beyond, in comparison with the result of an NSAID application in a solution on the skin.

**118.** The pharmaceutical preparation of claim 117, wherein the first component is phosphatidylcholine and the third component is an NSAID, such as ketoprofen, diclofenac, ibuprofen indomethacin, naproxen, or piroxicam.

**119.** The pharmaceutical preparation of claim 117, wherein the surfactant is selected from the group of nonionic surfactants, and preferably is a polyethyleneglycol-sorbitan-long fatty chain ester, a polyethyleneglycol-long fatty chain ester or a polyethyleneglycol-long fatty chain ether, the polyethyleneglycol chain being potentially replaced by a polyhydroxyethylene polar group, or else is a nonionic, surfactant like phospholipid.

**120.** The pharmaceutical preparation of claim 117, wherein the alcohol is n-propanol, iso-propanol, 2-propanol, n-butanol, 2-butanol, 1,2-propanediol, 1,2-butanediol, or ethanol.

**121.** The pharmaceutical preparation of claim 117, wherein the bulk pH value is above the logarithm of the apparent dissociation constant (pKa) of NSAID drug in a solution and in extended surface aggregates, and the latter pKa is higher than the former.

**122.** The pharmaceutical preparation of claim 117, wherein the bulk pH value is between 6.4 and 8.3, more preferably is between 6.7 and 8 and most preferably is between 7 and 7.7.

**123.** The pharmaceutical preparation of claim 117, wherein the bulk ionic strength is between 0.005 and 0.3, even better is between 0.01 and 0.2 and best is between 0.05 and 0.15.

**124.** The pharmaceutical preparation of claim 117, wherein the formulation viscosity is between 50 mPa s and 30.000 mPa s, preferably is between 100 mPa s and 10.000 mPa s, more preferably is between 200 mPa s and 5000 mPa s, and most preferred is between 400 mPa s and 2000 mPa s.

**125.** The pharmaceutical preparation of claim 117, wherein the first, i.e. phospholipid, component and the third, i. e. NSAID, component are present in the suspension in a relative molar ratio between 10/1 and 1/1.

**126.** The pharmaceutical preparation of claims 117, wherein the first, i.e. a phospholipid, component, and the second, i.e. a surfactant, component, are present in the suspension in a relative molar ratio between 40/1 and 4/1.

**127.** A kit comprising, in a tube or otherwise packaged form, at least one dose of the pharmaceutical preparation according to any one of claims 115 to 126.

**128.** A method for treating peripheral pain and/or inflammation by applying a pharmaceutical preparation according to any one of claims 114 to 127 on the skin of a warm blooded mammal.

**129.** The method according to claim 128 wherein different formulation doses per area are selected to control the depth of drug delivery.

**130.** The method according to 127 and 128. wherein the pharmaceutical formulation is applied in a non-occlusive patch.

**131.** Use of transdermal carriers according to any preceding claim to deliver NSAID molecules below the skin and into

the subcutaneous muscle, and/or subcutaneous joint.

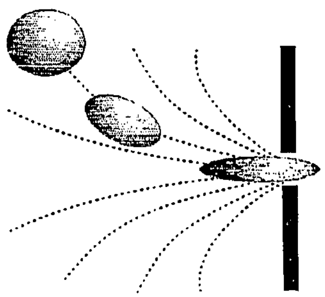

Fig. 1

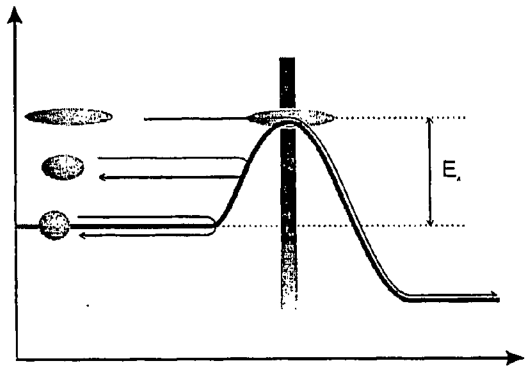


Fig. 2

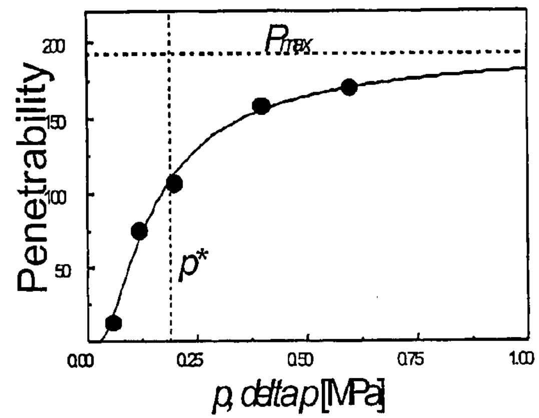


Fig. 3

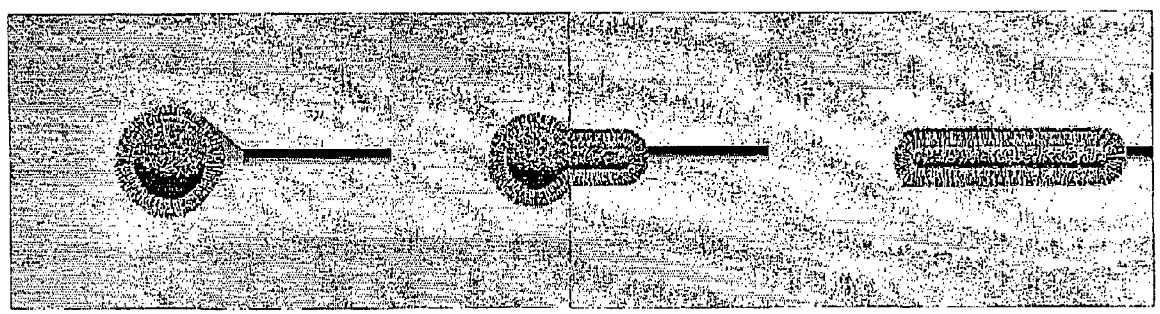

Fig. 4

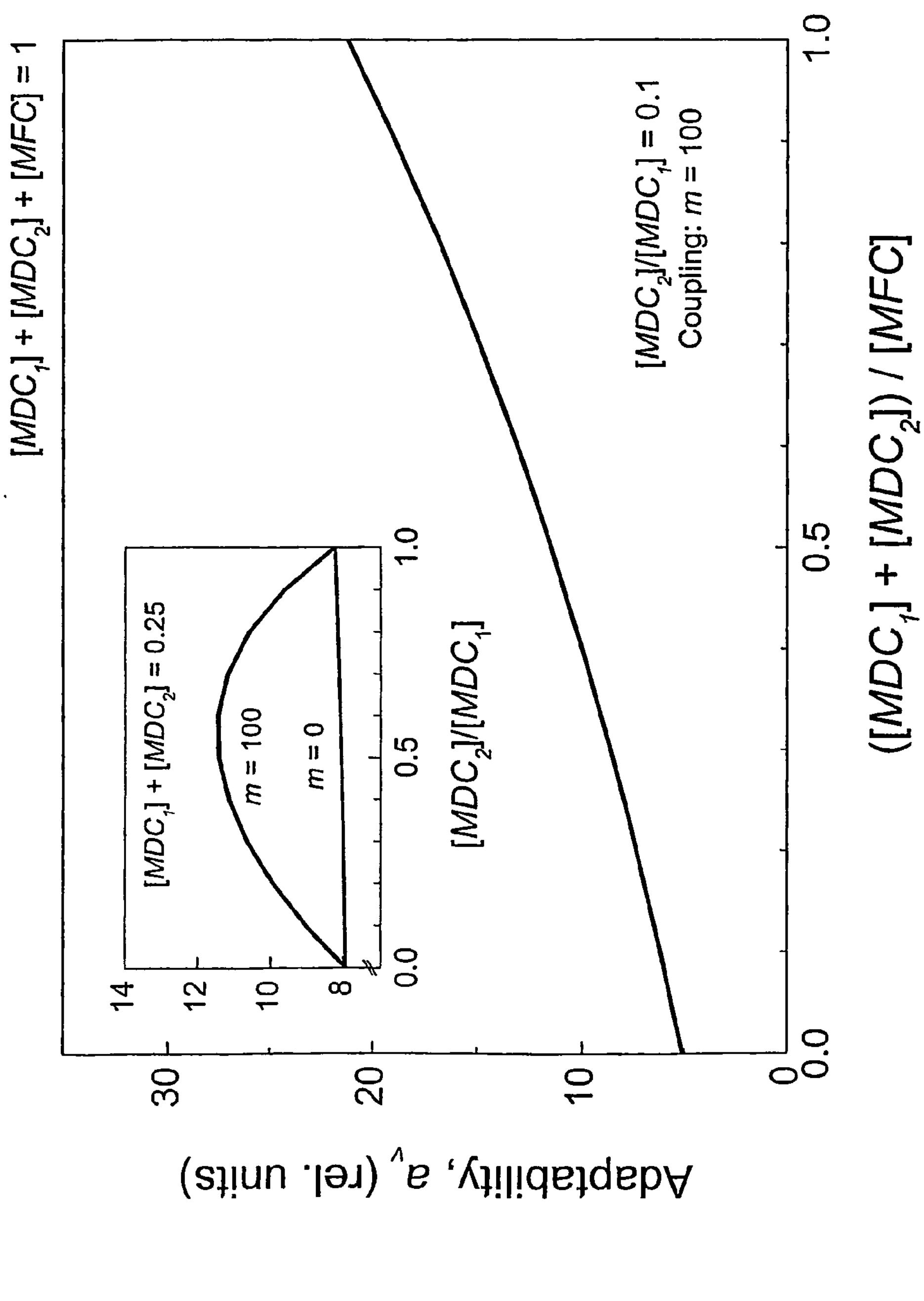

[MDC1] + [MDC2] + [MFC] = 1
Adaptability, av (rel. units)
0
10
20
30
0.0
0.5
1.0
([MDC1] + [MDC2]) / [MFC]
[MDC2]/[MDC1] = 0.1
Coupling: m = 100
[MDC1] + [MDC2] = 0.25
m = 100
m = 0
8
10
12
14
[MDC2]/[MDC1]


Fig. 5

Fig. 6

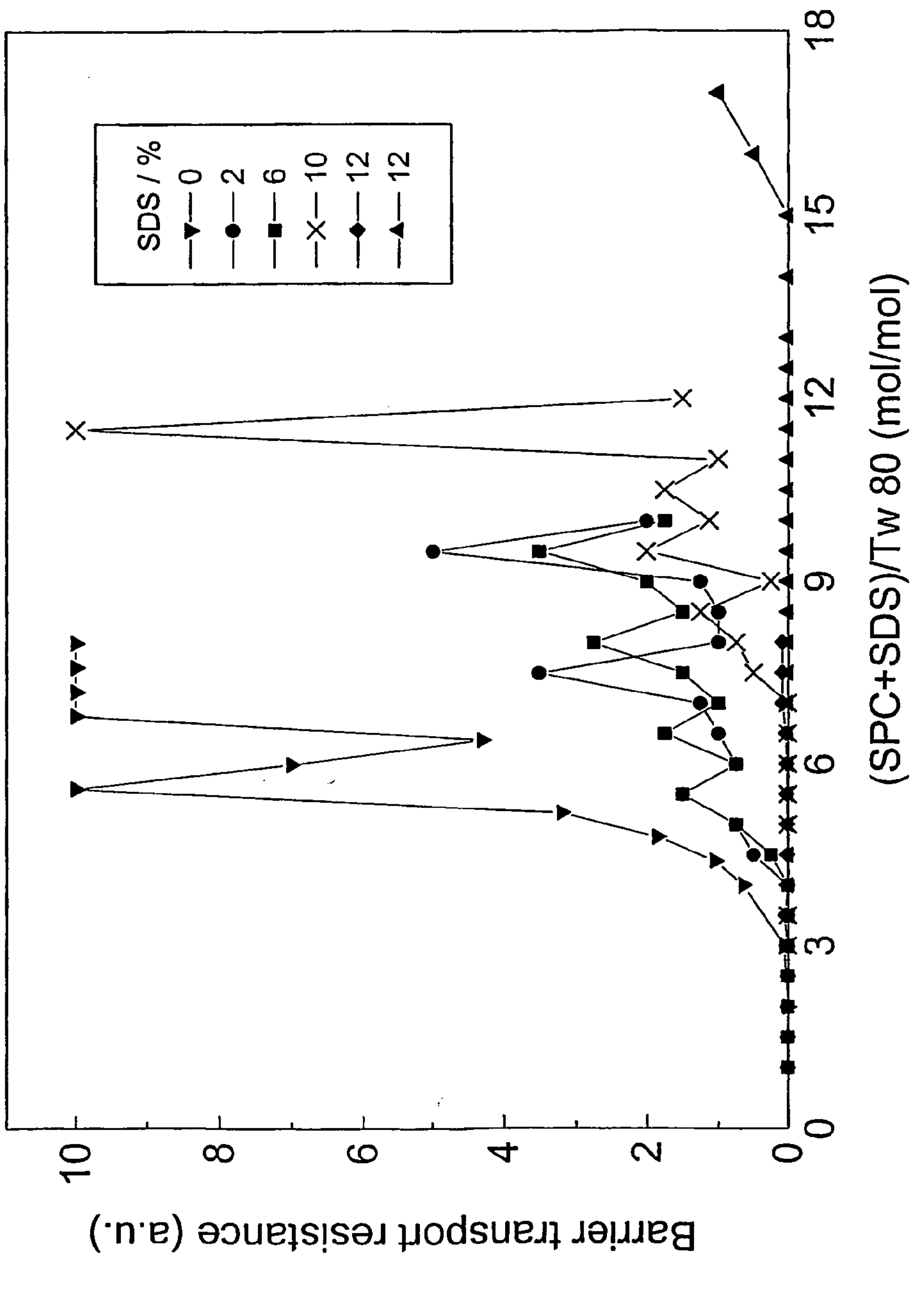

Barrier transport resistance (a.u.)
0
2
4
6
8
10
0
3
6
9
12
15
18
(SPC+SDS)/Tw 80 (mol/mol)
SDS / %
0
2
6
10
12
12

Fig. 7

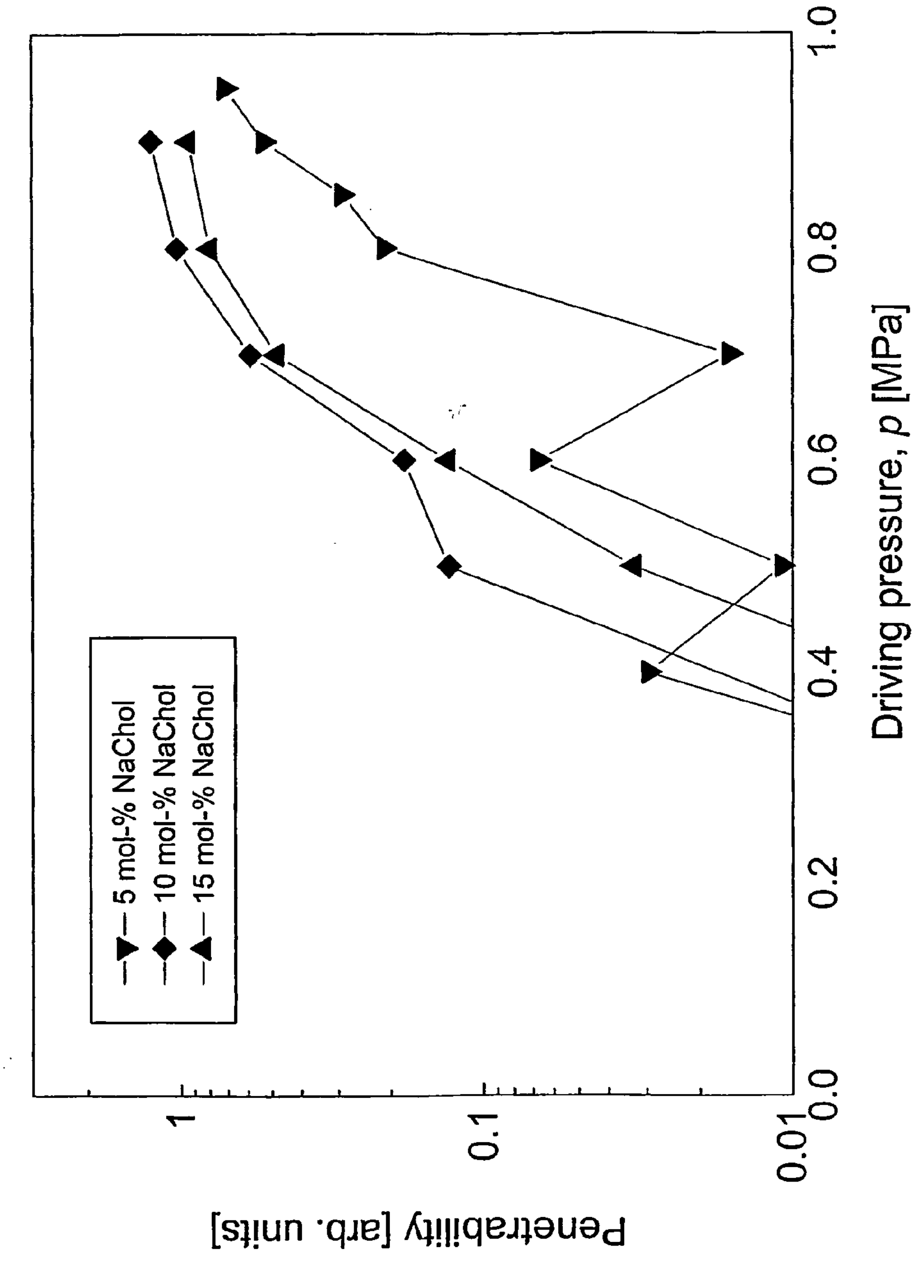

5 mol-% NaChol
10 mol-% NaChol
15 mol-% NaChol
Penetrability [arb. units]
1
0.1
0.01
0.0
0.2
0.4
0.6
0.8
1.0
Driving pressure, p [MPa]

Fig. 8

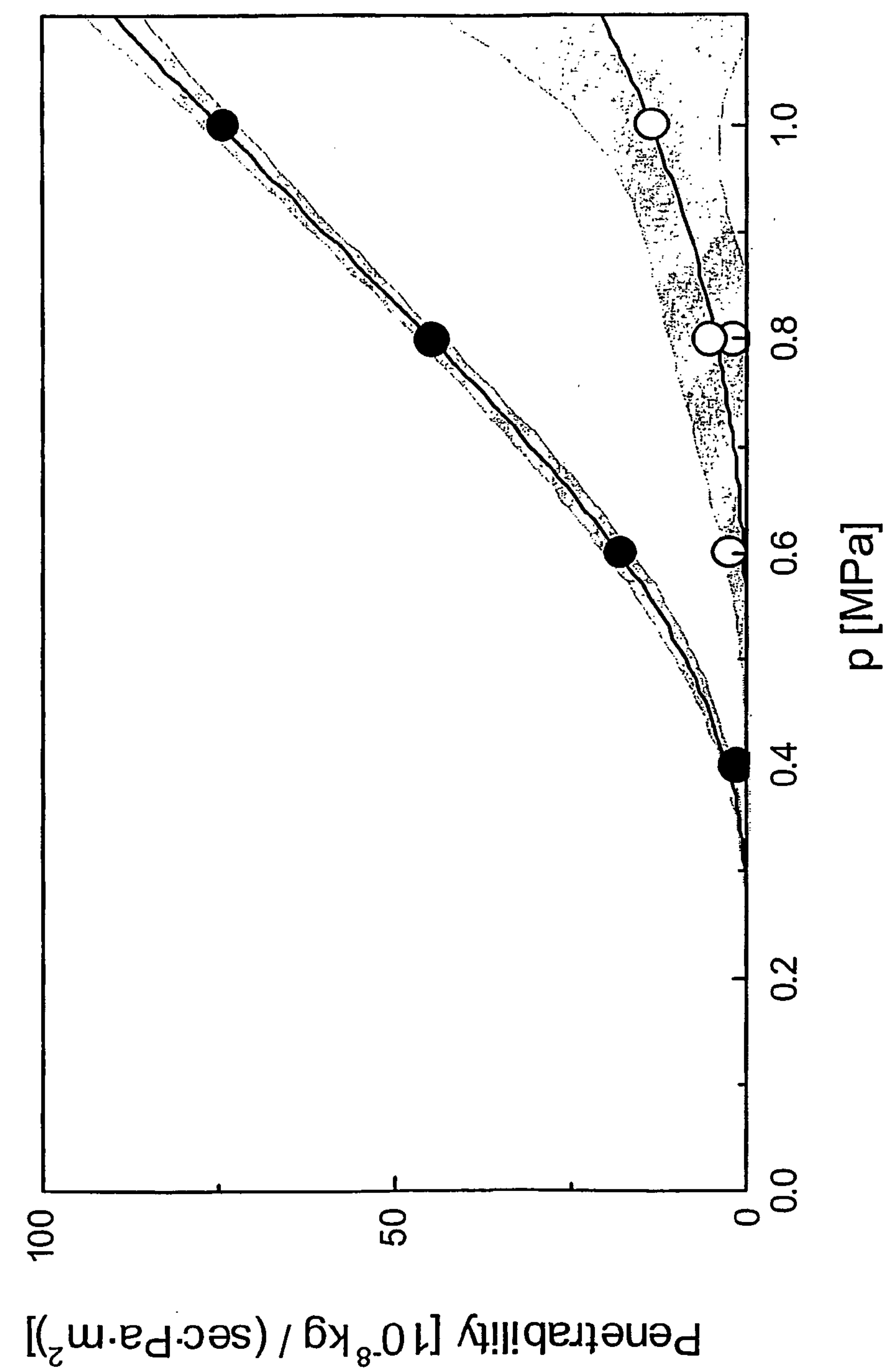

Fig. 9

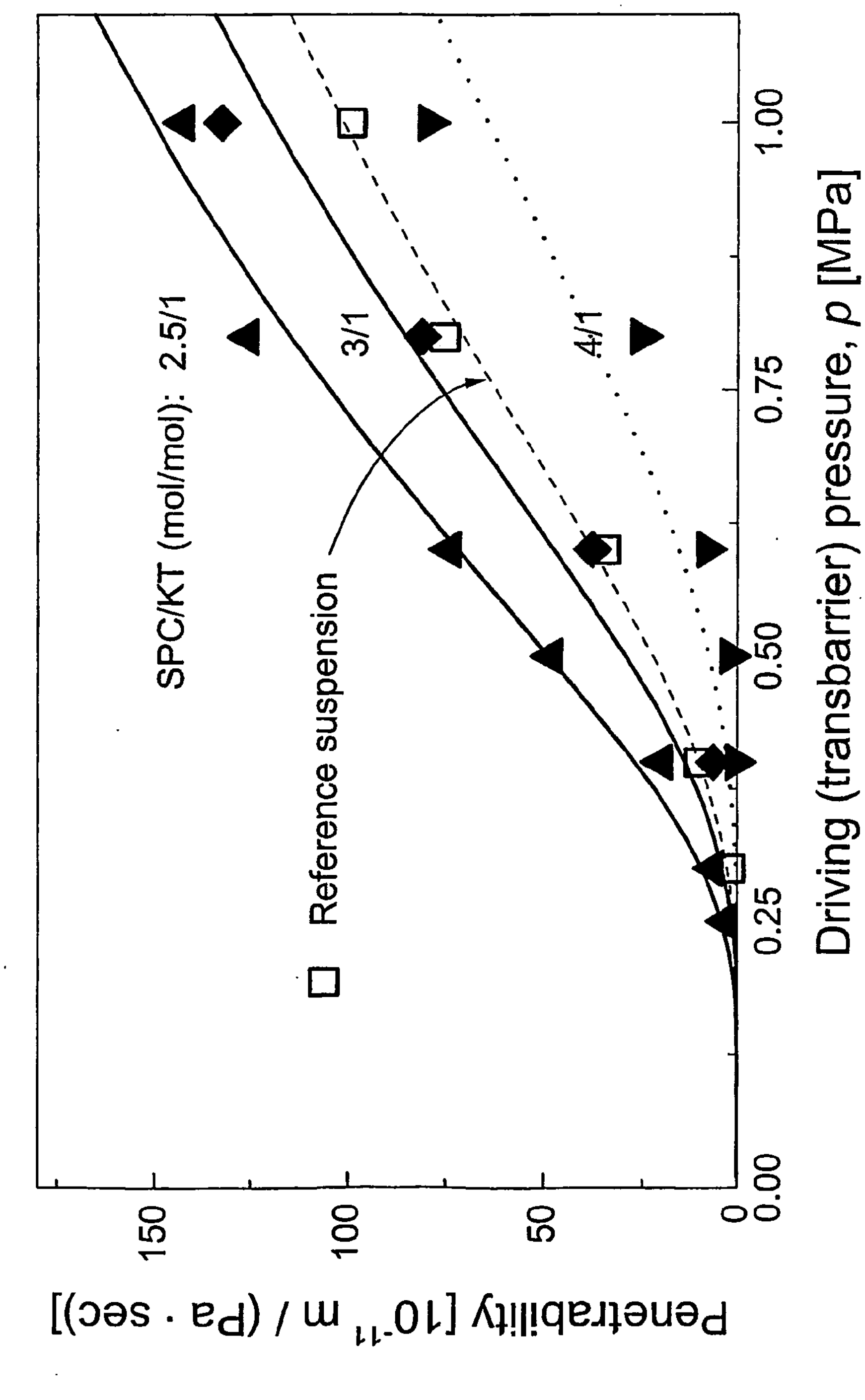
SPC/KT (mol/mol): 2.5/1
3/1
4/1
Reference suspension
Driving (transbarrier) pressure, p [MPa]
Penetrability [10-11 m / (Pa · sec)]
0.00
0.25
0.50
0.75
1.00
0
50
100
150

Fig. 10

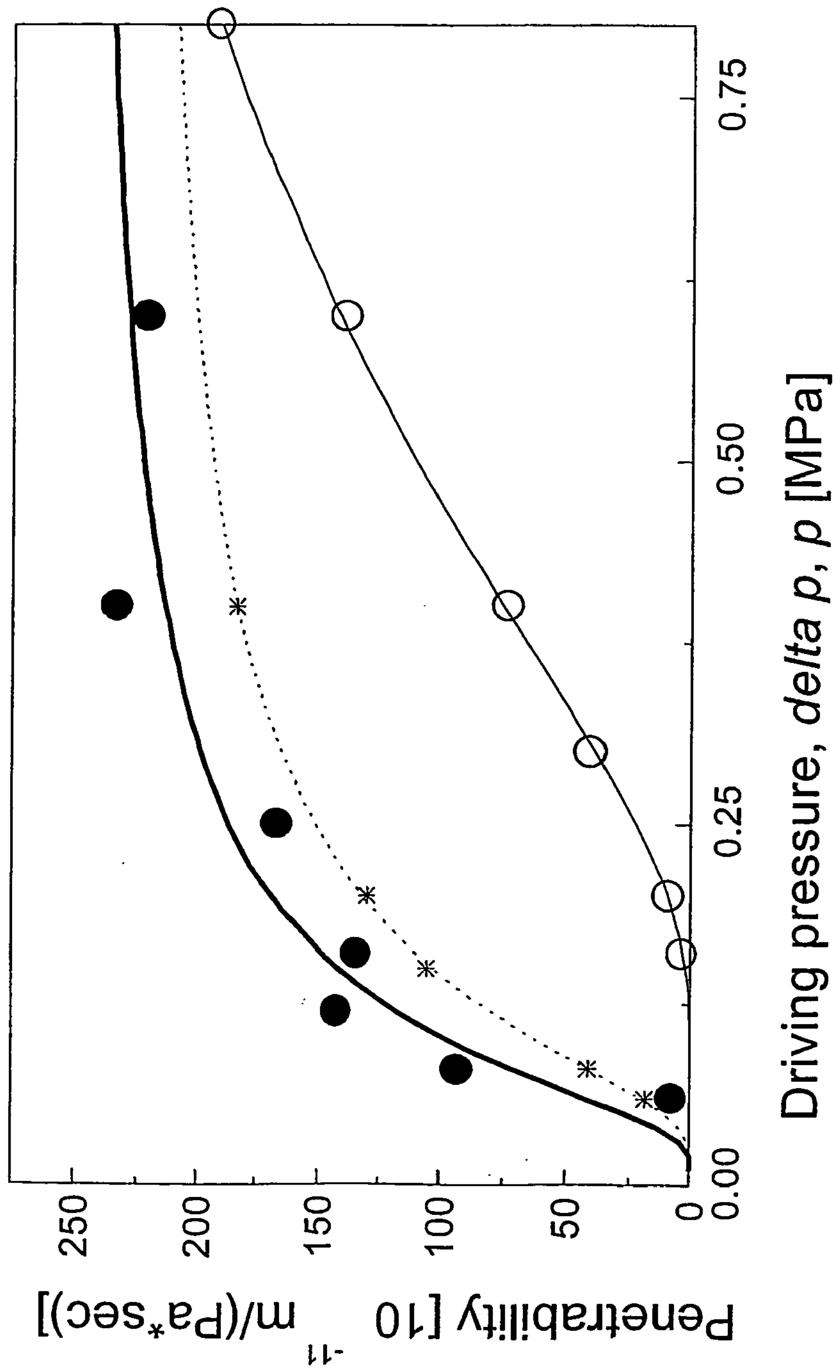
Penetrability [10 -11 m/(Pa*sec)]
0
50
100
150
200
250
0.00
0.25
0.50
0.75
Driving pressure, delta p, p [MPa]

Fig. 11

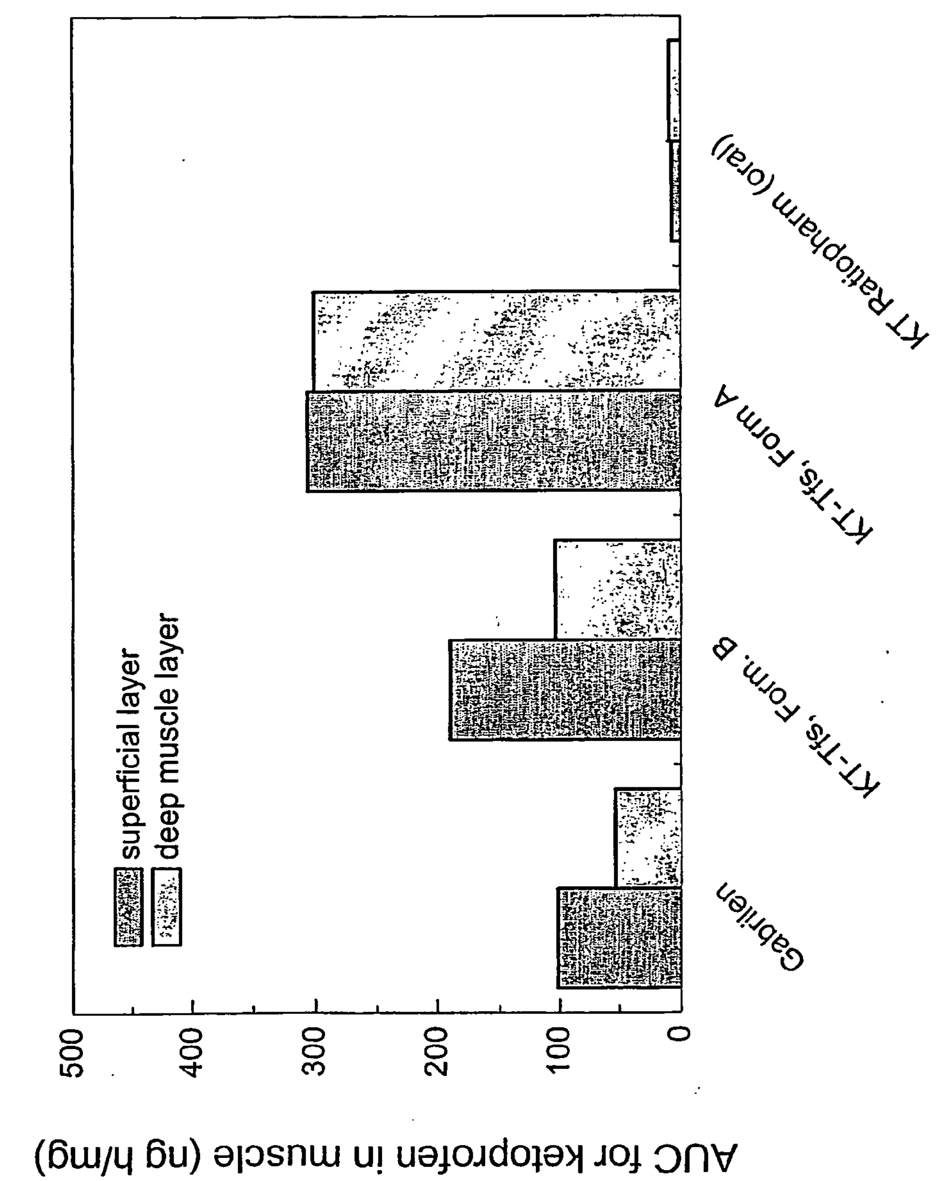
superficial layer
deep muscle layer
AUC for ketoprofen in muscle (ng h/mg)
0
100
200
300
400
500
Gabrilen
KT-Tfs, Form. B
KT-Tfs, Form A
KT Ratiopharm (oral)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 61271204 A
- WO 871938 A1
- US 6193996 B
- EP 102324 A
- EP 0088046 A
- US 4619794 A
- EP 0152379 A, Muntwyler and Hauser
- EP 0475160 A
- US 6165500 A
- CA 2067754
- WO 9817255 A
- AU 724218
- WO 9203122 A
- WO 98172550 A
- EP 9808421 W
- EP 0475160 A1

### Non-patent literature cited in the description


- *G. Cevc. Exp. Opin. Invest. Drugs,* 1997, vol. 6, 1887-1937
- **PRICE, C.E.** The Plant Cuticle. Price, Publisher, 1981, 237252
- **SIDDIQUI ; CHIEN.** *Crit. Rev. Ther. Drug. Carrier Syst.,* 1987, vol. 3, 195-208
- **BUMETTE ; ONGPIPATTANAKUL.** *J. Pharm. Sci.,* 1987, vol. 76, 765-773
- **VYAS et al.** *J Microencapsul,* 1995, vol. 12, 149-54
- **GESZTES ; MEZEI.** *Anesth. Analg.,* 1988, vol. 67, 1079-1081
- **GESZTES A ; MEZEI M.** Topical anesthesia of the skin by liposome-encapsulated tetracaine. *Anesth. Analg.,* 1988, vol. 67, 10791081
- **FOLDVARI M ; GESZTES A ; MEZEI M.** Dermal drug delivery by liposome encapsulation: clinical and electron microscopic studies. *J Microencapsul,* 1990, vol. 7, 479-489
- **FOLDVARI M.** In vitro cutaneous and percutaneous delivery and in vive efficacy of tetracaine from liposomal and conventional vehicles. *Pharm Res,* 1994, vol. 11, 1593-1598
- **FOLDVARI M.** Effect of vehicle on topical liposomal drug delivery: petrolatum bases. *J Microencapsul,* 1996, vol. 13, 589-600
- **P. GONZALEZ ; M. E. PLANAS ; L. RODRIGUEZ ; S. SANCHEZ ; G. CEVC.** Noninvasive, percutaneous induction of topical analgesia by a new type of drug carriers and prolongation of the local pain-insensitivity by analgesic liposomes. *Anesth. Analg.,* 1992, vol. 95, 615-621
- **PETERS ; MOLL.** Pharmacodynamics of a liposomal preparation for local anaesthesia. *Arzneimittelforschung,* 1995, vol. 45, 1253-6
- **CARAFA et al.** Lidocaine-loaded non-ionic surfactant vesicles: characterisation and in vitro permeation studies. *Int J Pharm,* 2002, vol. 231, 21-32
- **HENMI et al.** *Chem Pharm Bull,* 1994, vol. 42, 651-655
- **BURNHAM et al.** *Clin J Sport Med,* 1998, vol. 8, 78-81
- **SCHRAMLOVA et al.** *Folia Biol (Praha,* 1997, vol. 43, 195-199
- **SAUNDERS et al.** *J Pharm Pharm Sci,* 1999, vol. 2, 99-107
- **CALPENA et al.** *Arzneimittelforschung,* 1999, vol. 49, 1012-1017
- **STOYE et al.** *Eur J Pharm Biopharm,* 1998, vol. 46, 191-200
- **SCHATZLEIN A. ; RICHARDSEN H.** Ultradeformable Lipid Vesicles Can Penetrate the Skin and other SemiPermeable Barriers Intact. Evidence from Double Label CLSM Experiments and Direct Size Measurements. *Biochim. Biophys. Acta,* 2002, vol. 1564, 21-30
- Phospholipids Handbook. Marcel Dekker Publishers, 1993
- **B. FRISKEN.** *Biophys. J.,* 1998, vol. 74, 2996-3002
- *Langmuir,* 2000, vol. 16, 928-933
- *Critical Reviews in Therapeutic Drug Carrier Systems,* 1996, vol. 13, 257-388
- *Adv. Drug Delivery Rev.,* 1996, vol. 18, 349-378
- *Biochim. Biophys. Acta,* 1998, vol. 1368, 201-215